# EUROPEAN PATENT APPLICATION

(11) **EP 4 089 413 A1**
(43) Date of publication of application: **16.11.2022**
(21) Application number: 21305617.9
(22) Date of filing: 12.05.2021
(51) Int. Cl.: G01N 33/542, G01N 33/68

(54) **METHOD FOR DETERMINING THE BINDING OF AN ANTIBODY TO THE COMPLEMENT COMPONENT 1Q (C1Q)**

(71) Applicant: Cisbio Bioassays, 30200 Codolet (FR)
(72) Inventor: DOUAYRY, Najim, 84000 AVIGNON (FR); MENSAT, Patrick, 30320 MARGUERITTES (FR); ROUX, Thomas, 30900 NÎMES (FR); VALLAGHE, Julie, 30126 SAINT LAURENT DES ARBRES (FR); TRINQUET, Eric, 30126 SAINT LAURENT DES ARBRES (FR)
(74) Representative: Cabinet Beau de Loménie

(57) **Abstract**

The invention relates to a novel method for determining the binding of an antibody to the complement component 1q (C1q). The process according to the invention makes it possible to measure the binding of an antibody to the C1q in a Homogeneous Proximity Assay (HAS).

## Description

### TECHNICAL FIELD

The invention relates to a novel method for determining the binding of an antibody to the complement component 1q (C1q). The process according to the invention makes it possible to measure the binding of an antibody to the C1q in a Homogeneous Proximity Assay (HAS).

### PRIOR ART

Antibodies are immunological proteins that bind a specific antigen. In most mammals, including humans and mice, antibodies are constructed from paired heavy and light polypeptide chains. Each chain is made up of two distinct regions, referred to as the variable (Fv) and constant (Fc) regions. The light and heavy chain Fv regions contain the antigen binding determinants of the molecule and are responsible for binding the target antigen. The Fc region defines the class (or isotype) of antibody (IgG for example).

The Fc region interacts with a number of natural proteins, such as Fc gamma receptors or complement component 1q (C1q), to elicit important biochemical events, such as antibody-dependent cell-mediated cytotoxicity (ADCC) and/or complement-dependent cytotoxicity (CDC). Fc binding to C1q activates the complement cascade of the immune system, which reflects the ability of an antibody to mediate CDC.

The C1q binding site on IgG is CH2 domain of the Fc region. The three charged residues Glu-318, Lys-320 and Lys-322 on the CH2 domain are crucial for the binding of C1q to IgG. The C1q binding site on IgM is CH3 domain of the Fc region.

C1q has six globular heads that can each bind a single IgG molecule (via its Fc region). C1q is therefore capable of binding six antibodies, although binding to two antibodies (e.g. two IgGs) is sufficient to activate the complement pathway. C1q forms a complex with the C1r and C1s serine proteases to form the C1 complex of the complement pathway.

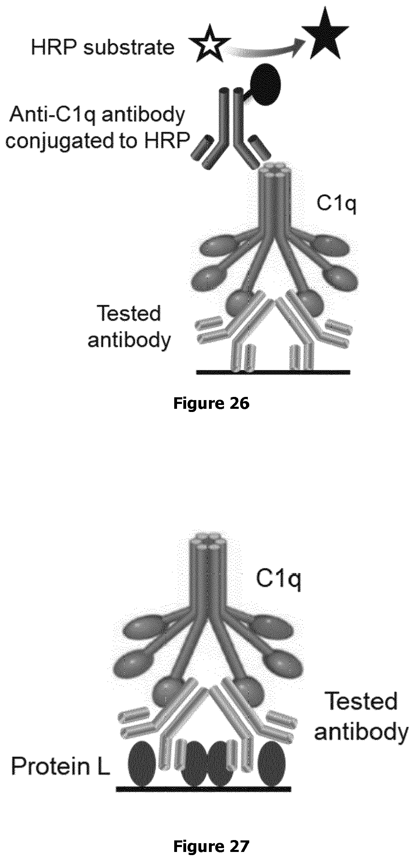

It is necessary C1q binds at least two antibodies (e.g. two IgGs) to activate the complement pathway. Therefore, a solid phase is always used in the prior art to assemble IgG into appropriate clusters able to bind C1q with high affinity and therefore measuring the ability of an antibody to bind C1q. The solid phase methods that are commonly used in the prior art to assess the binding of antibodies to C1q include enzyme-linked immunosorbent assay (ELISA) and surface plasmon resonance (SPR). There are three ways to cluster IgGs on a solid surface, either on an ELISA plate or a SPR surface: (i) Antibodies are randomly immobilized on the surface (passive coating) [Ref. 1], (ii) Antibodies are indirectly immobilized on the surface using Protein L [Ref. 2] or (iii) Antigens are coated on the surface and then antibodies are added [Ref. 3] (Figure 26, Figure 27 and Figure 28).

However, the existing solid-phase methods are not fully satisfying because the hexameric IgG/C1q binding model has geometrical constraints and it is difficult to predict the right IgG clustering to bind C1q with high affinity [Ref. 4]. The results are therefore not always reproducible and there is often a need to normalise each data.

There is therefore a need for developing an efficient, easy-to-implement and reproducible method for determining the binding of an antibody (tested antibody) to a complement component 1q (C1q).

### SUMMARY OF THE INVENTION

The present invention aims to propose a novel *in vitro* method for determining the binding of an antibody (tested antibody) to a complement component 1q (C1q), i.e. an *in vitro* method for assessing the ability of an antibody (tested antibody) to mediate CDC.

According to a first aspect, the invention relates to an *in vitro* method for determining the binding of an antibody (tested antibody) to a complement component 1q (C1q), comprising the following steps:
a) Contacting into a measurement medium:
   - a tested antibody,
   - a biotinylated anti-Fab ligand, said biotinylated anti-Fab ligand being able to bind the Fab region of the tested antibody,
   - a streptavidin directly or indirectly labelled with the first member of a pair of HPA (Homogeneous Proximity Assay) partners, and
   - a C1q directly or indirectly labelled with the second member of a pair of HPA partners;
b) Measuring the HPA signal in the measurement medium, the existence of a HPA signal being representative of the binding of the tested antibody to the C1q.

According to a second aspect, the invention relates to a kit of reagents for carrying out the method of the invention, comprising:
(i) a biotinylated anti-Fab ligand, and
(ii) a streptavidin or a streptavidin directly labelled with the first member of a pair of HPA partners, and
(iii) a C1q or a C1q directly labelled with the second member of a pair of HPA partners, and
(iv) if the streptavidin is not directly labelled with the first member of a pair of HPA partners, an anti-streptavidin ligand directly labelled with the first member of a pair of HPA partners, and
(v) if the C1q is not directly labelled with the second member of a pair of HPA partners, an anti-Clq ligand directly labelled with the second member of a pair of HPA partners.

### BRIEF DESCRIPTION OF THE FIGURES

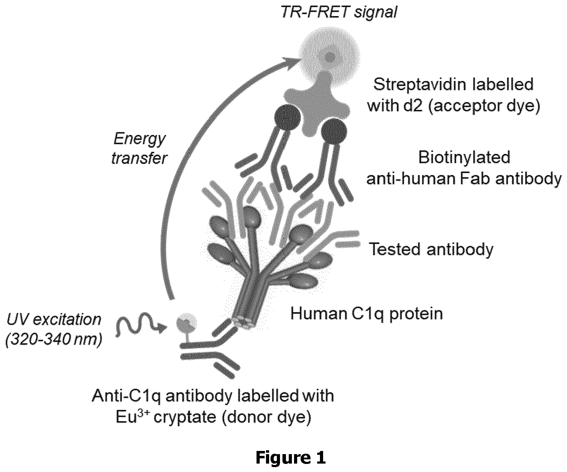

**Figure 1** illustrates the TR-FRET sandwich assay carried out to determine the binding of an IgG to a human C1q protein using a biotinylated anti-human Fab antibody and a C1q indirectly labelled with the donor. The assay is based on HTRF^{®} technology and uses an anti-Clq antibody conjugated to Eu³⁺ cryptate (donor) and a streptavidin labelled with d2 (acceptor). When the tested antibody binds to human C1q, it brings the donor in proximity to the acceptor. Excitation of the donor with a light source (UV range) triggers an energy transfer towards the acceptor which in turn emits specific fluorescence (TR-FRET signal).

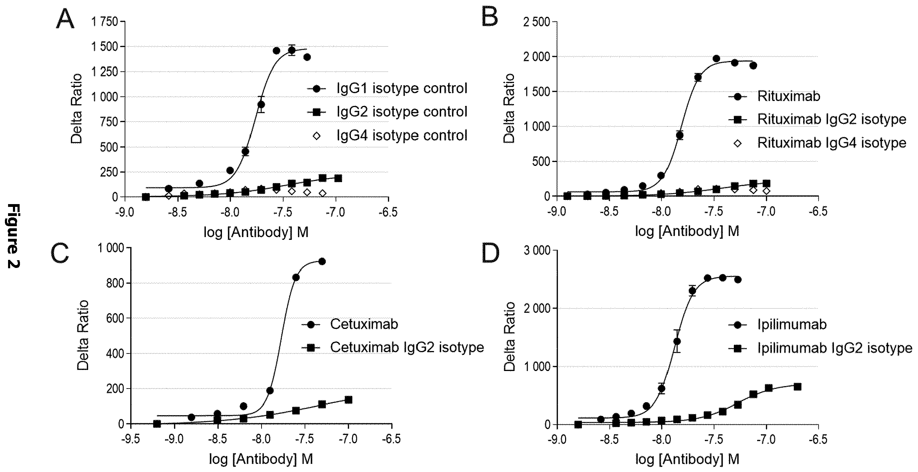

**Figure 2** presents the results of the binding of IgG antibodies of different isotypes to human C1q based on the TR-FRET method described in Figure 1. The sigmoidal dose response curves represent the log [antibody] (M) versus the specific HTRF signal (Delta Ratio). A) Results obtained with recombinant human IgG isotype controls (IgG1, IgG2 and IgG4). B) Results obtained with the therapeutic antibody Rituximab (IgG1) and its isotype variants IgG2 and IgG4. C) Results obtained with the therapeutic antibody Cetuximab (IgG1) and its isotype variant IgG2. D) Results obtained with the therapeutic antibody Ipilimumab (IgG1) and its isotype variant IgG2.

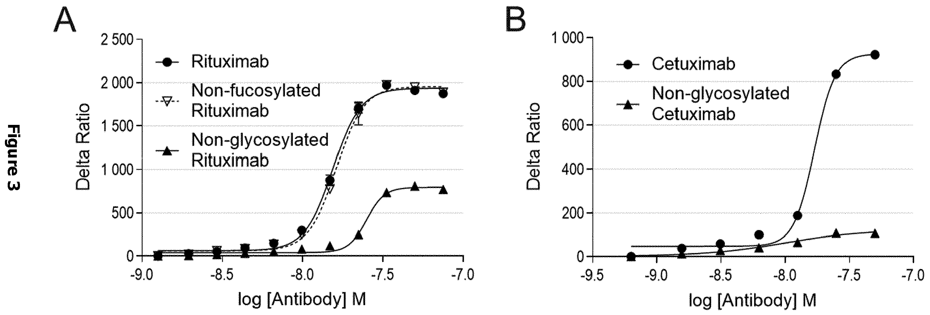

**Figure 3** presents the results of the binding of therapeutic antibodies and their non-glycosylated or non-fucosylated variants to human C1q, based on the TR-FRET method described in Figure 1. The sigmoidal dose response curves represent the log [antibody] (M) versus the specific HTRF signal (Delta Ratio). A) Results obtained with Rituximab (glycosylated antibody) and its non-fucosylated and non-glycosylated variants. B) Results obtained with Cetuximab (glycosylated antibody) and its non-glycosylated variant.

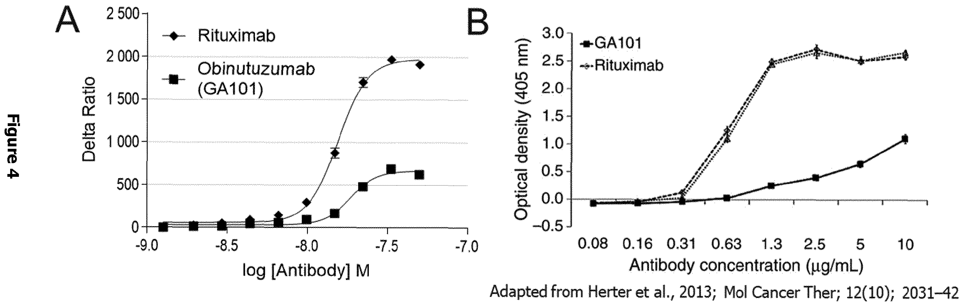

**Figure 4** presents the binding profiles of the therapeutic type I and type II anti-CD20 antibodies Rituximab and Obinutuzumab (GA101) to human C1q. A) Results obtained using the TR-FRET method described in Figure 1. The sigmoidal dose response curves represent the log [antibody] (M) versus the specific HTRF signal (Delta Ratio). B) Data from the literature obtained using an ELISA assay (adapted from [Ref. 5]). The binding curves represent the [antibody] (µg/mL) versus the ELISA signal (OD at 405 nm).

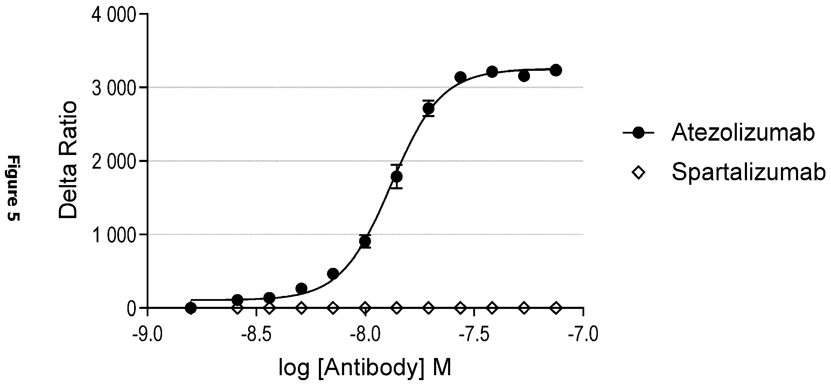

**Figure 5** presents the results of the binding of the therapeutic antibodies Atezolizumab (IgG1) and Spartalizumab (IgG4) to human C1q based on the TR-FRET method described in Figure 1. The sigmoidal dose response curves represent the log [antibody] (M) versus the specific HTRF signal (Delta Ratio).

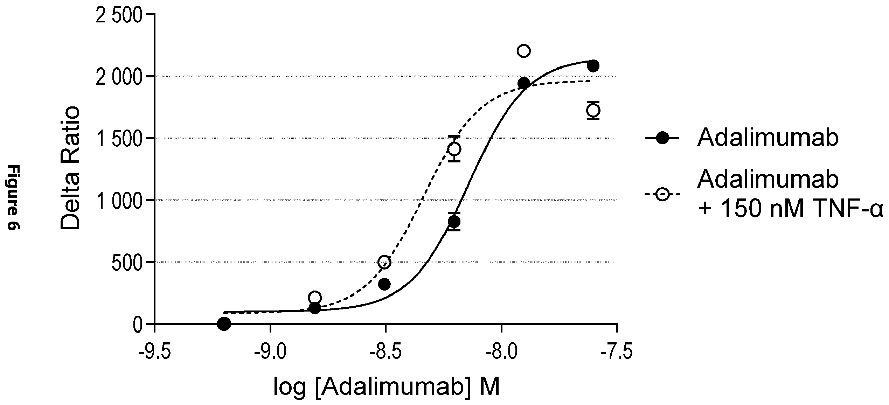

**Figure 6** presents the results of the binding of the therapeutic anti-TNF-α antibody Adalimumab (pre-complexed or not with 150 nM human TNF-α) to human C1q, based on the TR-FRET method described in Figure 1. The sigmoidal dose response curves represent the log [antibody] (M) versus the specific HTRF signal (Delta Ratio).

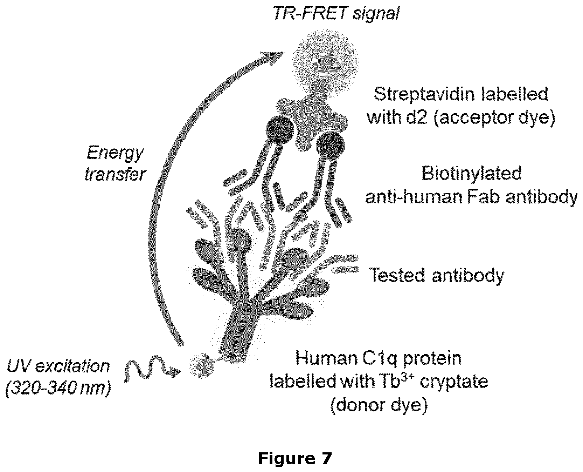

**Figure 7** illustrates the TR-FRET sandwich assay carried out to determine the binding of an IgG to a human C1q protein using a biotinylated anti-human Fab antibody and a C1q directly labelled with the donor. The assay is based on HTRF^{®} technology and uses a human C1q conjugated to Tb³⁺ cryptate (donor) and a streptavidin labelled with d2 (acceptor). When the tested antibody binds to human C1q, it brings the donor in proximity to the acceptor. Excitation of the donor with a light source (UV range) triggers an energy transfer towards the acceptor which in turn emits specific fluorescence (TR-FRET signal).

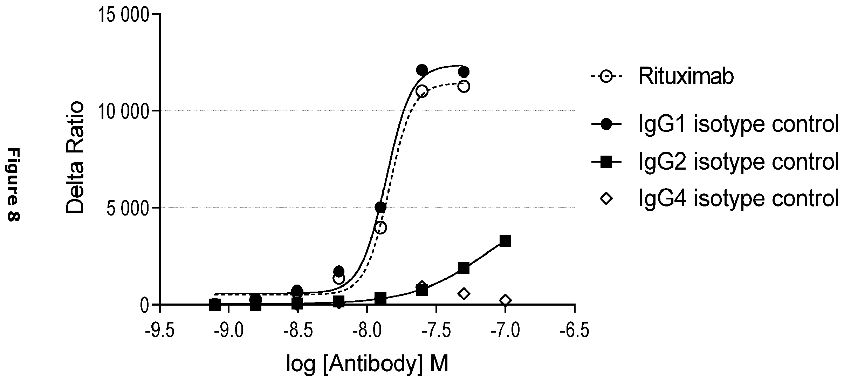

**Figure 8** presents the results of the binding of IgG antibodies of different isotypes to human C1q based on the TR-FRET method described in Figure 7. The sigmoidal dose response curves represent the log [antibody] (M) versus the specific HTRF signal (Delta Ratio). The tested antibodies are the therapeutic antibody Rituximab (IgG1), as well as the recombinant human IgG isotype controls IgG1, IgG2 and IgG4.

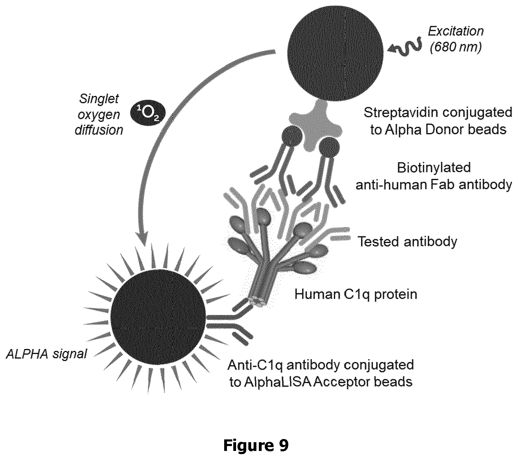

**Figure 9** illustrates the ALPHA sandwich assay carried out to determine the binding of an IgG to a human C1q protein using a biotinylated anti-human Fab antibody and a C1q indirectly labelled with the acceptor. The assay is based on AlphaLISA^{®} technology and uses Alpha streptavidin-coated donor beads (donor) and an anti-Clq antibody conjugated to AlphaLISA acceptor beads (acceptor). When the tested antibody binds to human C1q, it brings the donor in proximity to the acceptor. Excitation of the donor with a laser at 680 nm triggers the production and diffusion of singlet oxygen to the acceptor which in turn produces specific fluorescence (ALPHA signal).

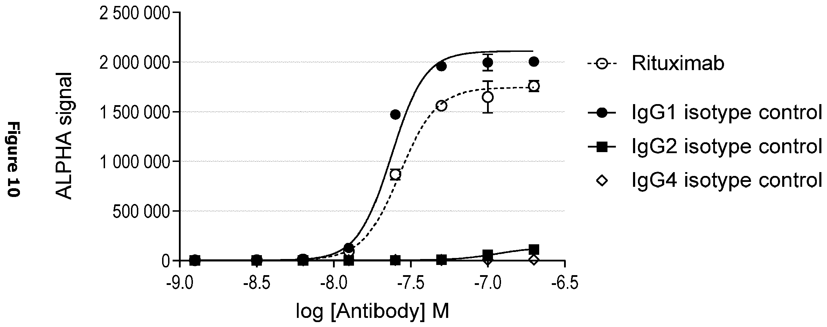

**Figure 10** presents the results of the binding of IgG antibodies of different isotypes to human C1q based on the ALPHA method described in Figure 9. The sigmoidal dose response curves represent the log [antibody] (M) versus the ALPHA signal. The tested antibodies are the therapeutic antibody Rituximab (IgG1), as well as the recombinant human IgG isotype controls IgG1, IgG2 and IgG4.

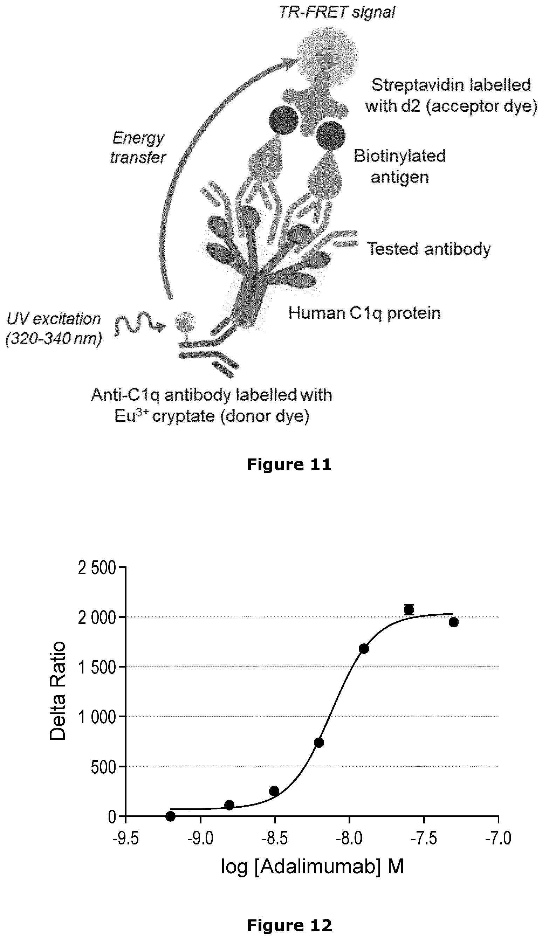

**Figure 11** illustrates the TR-FRET sandwich assay carried out to determine the binding of an IgG to a human C1q protein using a biotinylated antigen and a C1q indirectly labelled with the donor. The assay is based on HTRF^{®} technology and uses an anti-Clq antibody conjugated to Eu³⁺ cryptate (donor) and a streptavidin labelled with d2 (acceptor). When the tested antibody binds to human C1q, it brings the donor in proximity to the acceptor. Excitation of the donor with a light source (UV range) triggers an energy transfer towards the acceptor which in turn emits specific fluorescence (TR-FRET signal).
**Figure 12** presents the results of the binding of the therapeutic anti-TNF-α antibody Adalimumab to human C1q based on the TR-FRET method described in Figure 11 using biotinylated recombinant human TNF-α. The sigmoidal dose response curve represents the log [antibody] (M) versus the specific HTRF signal (Delta Ratio).

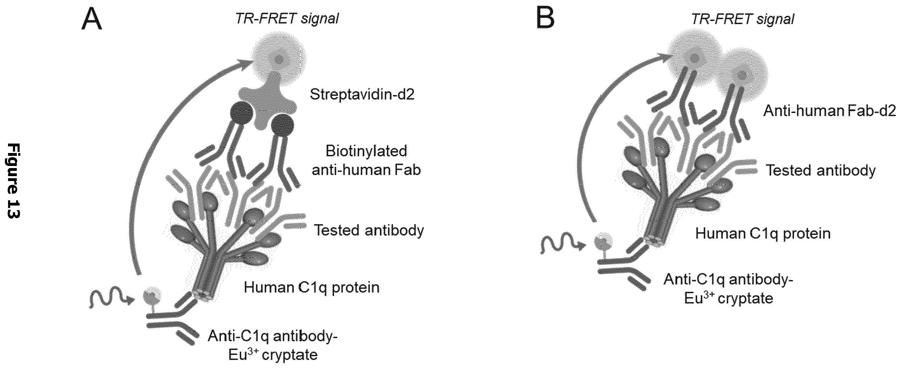

**Figure 13** illustrates the TR-FRET sandwich assays carried out to determine the binding of an IgG to a human C1q protein using a C1q indirectly labelled with the donor and an anti-human Fab antibody either biotinylated and complexed to acceptor-labelled streptavidin, or directly conjugated to the acceptor. The assays are based on HTRF^{®} technology and use an anti-Clq antibody conjugated to Eu³⁺ cryptate (donor) and A) a biotinylated anti-human Fab antibody complexed to streptavidin-d2 (acceptor) or B) the same anti-human Fab antibody directly labelled with d2. When the tested antibody binds to human C1q, it brings the donor in proximity to the acceptor. Excitation of the donor with a light source (UV range) triggers an energy transfer towards the acceptor which in turn emits specific fluorescence (TR-FRET signal).

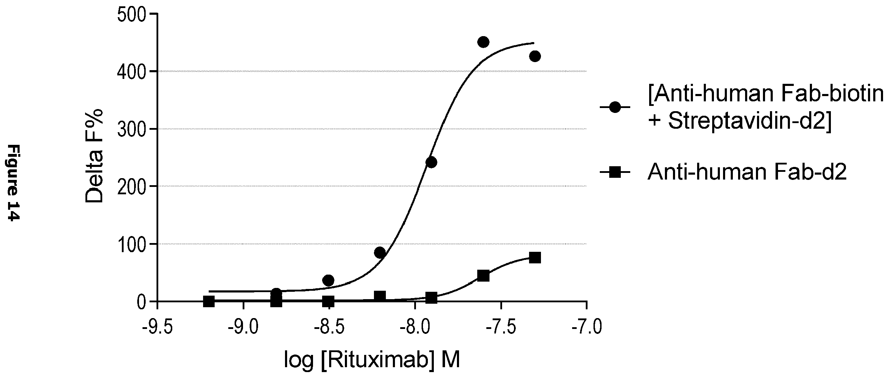

**Figure 14** presents the results of the binding of the therapeutic antibody Rituximab to human C1q using the two different TR-FRET assay formats described in Figure 13. The sigmoidal dose response curves represent the log [antibody] (M) versus the normalized HTRF signal (Delta F%).

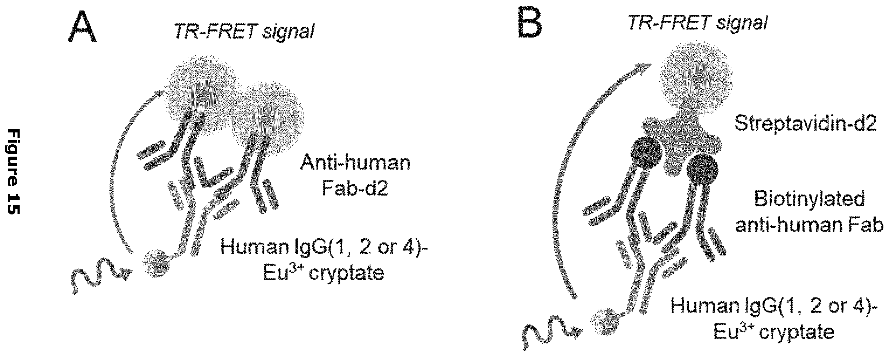

**Figure 15** illustrates the TR-FRET sandwich assays used to perform saturation binding experiments and determine the affinity (Kd) of anti-human Fab antibodies for human IgG of different isotypes (IgG1, IgG2 and IgG4) labelled with the donor. The assays are based on HTRF^{®} technology and use a human IgG (IgG1, IgG2 or IgG4) labelled with Eu³⁺ cryptate (donor) and A) an anti-human Fab antibody directly labelled with d2 (acceptor) or B) the same anti-human Fab conjugated to biotin and complexed to streptavidin-d2. The binding of the anti-human Fab antibody to the human IgG triggers the emission of a TR-FRET signal which increases proportionally with the concentration of the tested anti-human Fab antibody until reaching a plateau (saturation).

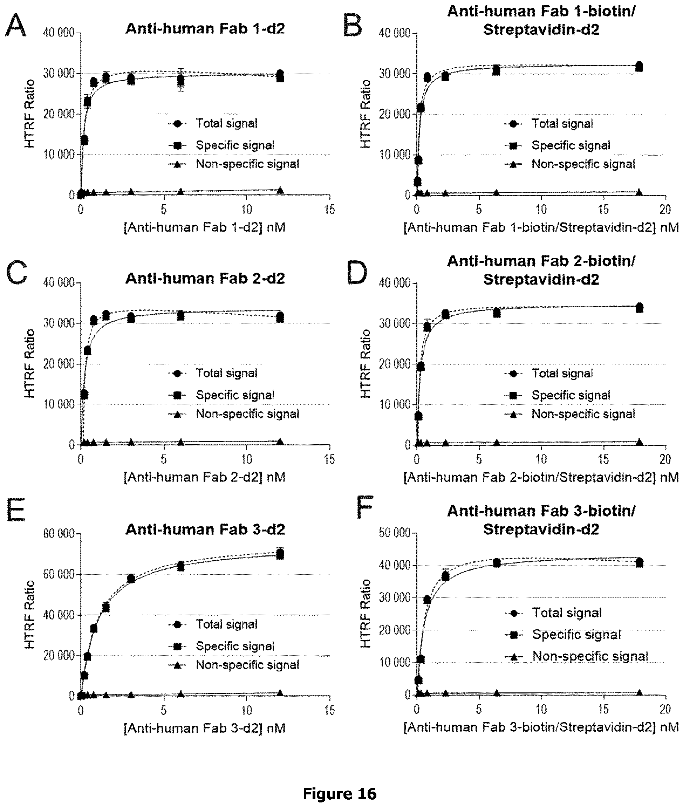

**Figure 16** presents the results of the binding of three different anti-human Fab antibodies (1, 2 and 3) to Eu³⁺ cryptate-human IgG1 using the two different TR-FRET sandwich assays described in Figure 15. The saturation binding curves represent the [anti-human Fab antibody] (nM) versus the HTRF signal (HTRF Ratio). The total signal corresponds to the signal obtained in absence of unlabeled human IgG1. The non-specific signal corresponds to the signal obtained in the presence of an excess of unlabeled IgG1. The specific signal was calculated by subtracting the non-specific signal to the total signal. A) Results obtained with anti-human Fab 1 directly labelled with d2. B) Results obtained with biotinylated anti-human Fab 1 complexed with streptavidin-d2. C) Results obtained with anti-human Fab 2 directly labelled with d2. D) Results obtained with biotinylated anti-human Fab 2 complexed with streptavidin-d2. E) Results obtained with anti-human Fab 3 directly labelled with d2. F) Results obtained with biotinylated anti-human Fab 3 complexed with streptavidin-d2.

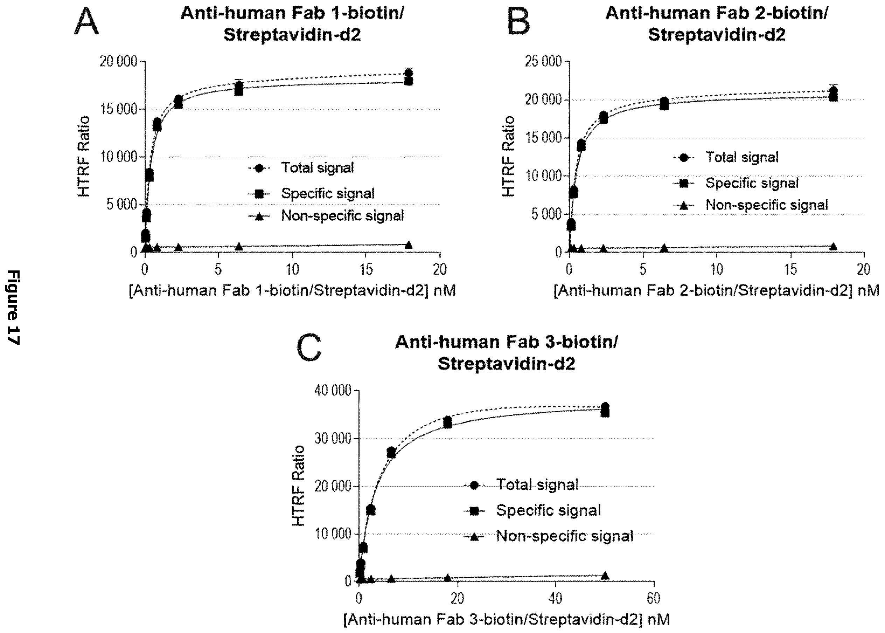

**Figure 17** presents the results of the binding of three different anti-human Fab antibodies (1, 2 and 3) to Eu³⁺ cryptate-human IgG2 using the two different TR-FRET sandwich assays described in Figure 15. The saturation binding curves represent the [anti-human Fab antibody] (nM) versus the HTRF signal (HTRF Ratio). The total signal corresponds to the signal obtained in absence of unlabeled human IgG2. The non-specific signal corresponds to the signal obtained in the presence of an excess of unlabeled IgG2. The specific signal was calculated by subtracting the non-specific signal to the total signal. A) Results obtained with biotinylated anti-human Fab 1 complexed with streptavidin-d2. B) Results obtained with biotinylated anti-human Fab 2 complexed with streptavidin-d2. C) Results obtained with biotinylated anti-human Fab 3 complexed with streptavidin-d2.

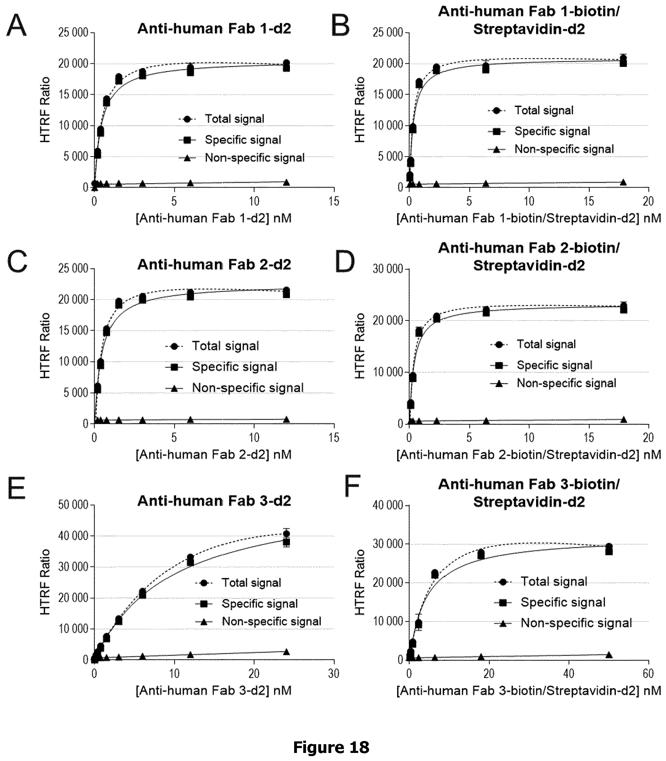

**Figure 18** presents the results of the binding of three different anti-human Fab antibodies (1, 2 and 3) to Eu³⁺ cryptate-human IgG4 using the two different TR-FRET sandwich assays described in Figure 15. The saturation binding curves represent the [anti-human Fab antibody] (nM) versus the HTRF signal (HTRF Ratio). The total signal corresponds to the signal obtained in absence of unlabeled human IgG4. The non-specific signal corresponds to the signal obtained in the presence of an excess of unlabeled IgG4. The specific signal was calculated by subtracting the non-specific signal to the total signal. A) Results obtained with anti-human Fab 1 directly labelled with d2. B) Results obtained with biotinylated anti-human Fab 1 complexed with streptavidin-d2. C) Results obtained with anti-human Fab 2 directly labelled with d2. D) Results obtained with biotinylated anti-human Fab 2 complexed with streptavidin-d2. E) Results obtained with anti-human Fab 3 directly labelled with d2. F) Results obtained with biotinylated anti-human Fab 3 complexed with streptavidin-d2.

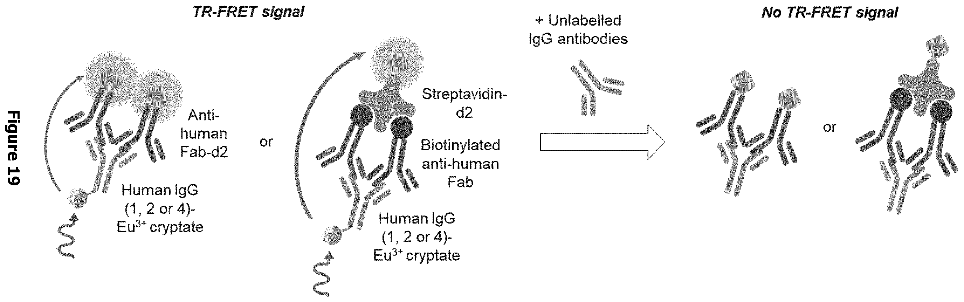

**Figure 19** illustrates the TR-FRET competition assays used to perform competitive binding experiments and determine the affinity (Ki) of anti-human Fab antibodies for fully human, humanized, or chimeric IgG of different isotypes (IgG1, IgG2 and IgG4). The assays are based on the two different HTRF^{®} sandwich assay formats presented in Figure 15. In absence of competitor (unlabeled IgG), the Eu³⁺ cryptate-human IgG binds to the acceptor-anti-human Fab, which triggers the emission of a TR-FRET signal. In presence of unlabeled IgG able to interact with the anti-human Fab antibody and to compete with the Eu³⁺ cryptate-human IgG, the TR-FRET signal decreases proportionally with the concentration of the tested IgG.

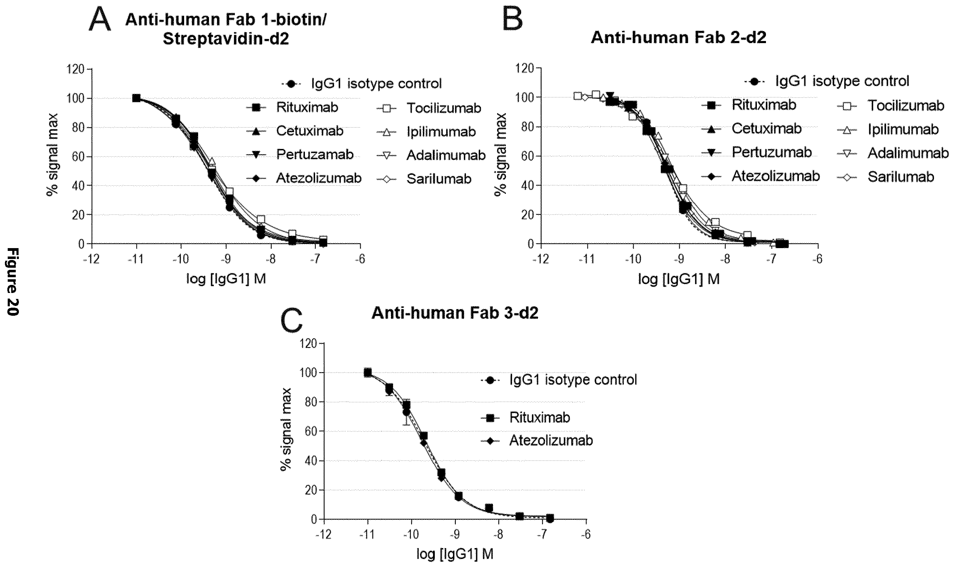

**Figure 20** presents the results of the competition between Eu³⁺ cryptate-human IgG1 and different unlabeled IgG1 antibodies, with three different anti-human Fab antibodies (1, 2 and 3), using one of the two TR-FRET competition assays described in Figure 19. The sigmoidal dose response inhibition curves represent the log [unlabeled IgG1] (M) versus the % of normalized HTRF signal max (= Delta F%_{unlabeled IgG1 antibody/}Delta F%ₘₐₓ x 100). A) Results obtained with biotinylated anti-human Fab 1 complexed with streptavidin-d2. B) Results obtained with anti-human Fab 2 directly labelled with d2. C) Results obtained with anti-human Fab 3 directly labelled with d2.

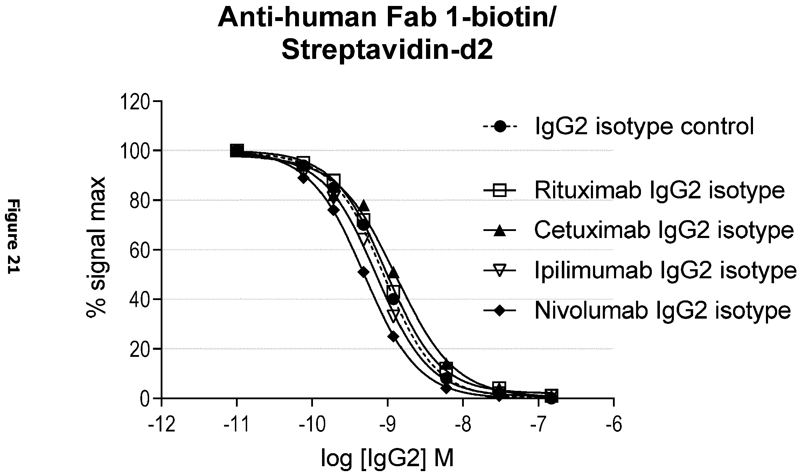

**Figure 21** presents the results of the competition between Eu³⁺ cryptate-human IgG2 and different unlabeled IgG2 antibodies, with the biotinylated anti-human Fab 1 complexed with streptavidin-d2, as described in Figure 19. The sigmoidal dose response inhibition curves represent the log [unlabeled IgG2] (M) versus the % of normalized HTRF signal max (= Delta F%_{unlabeled IgG1 antibody}/Delta F%ₘₐₓ x 100).

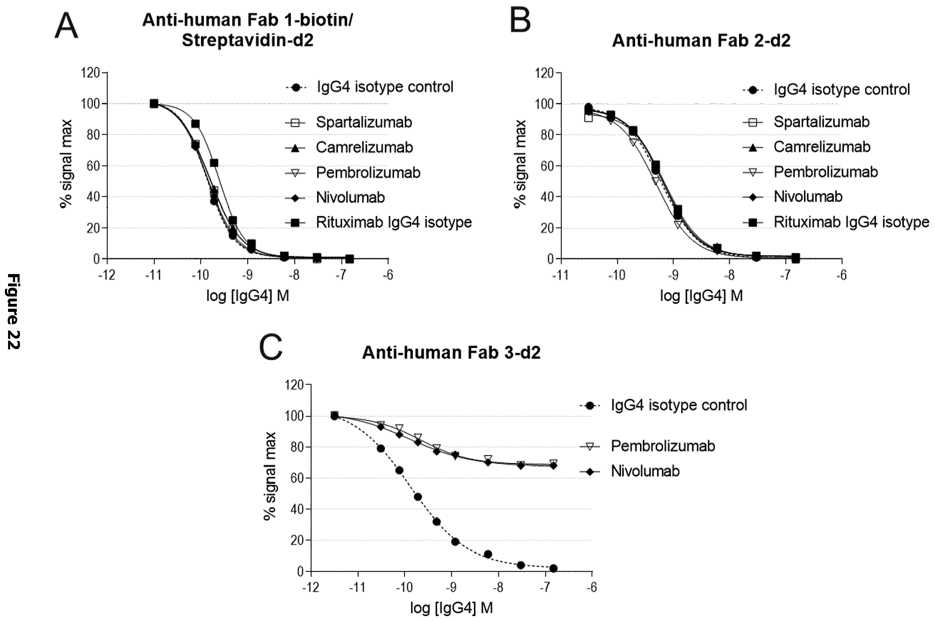

**Figure 22** presents the results of the competition between Eu³⁺ cryptate-human IgG4 and different unlabeled IgG4 antibodies, with three different anti-human Fab antibodies (1, 2 and 3), using one of the two TR-FRET competition assays described in Figure 19. The sigmoidal dose response inhibition curves represent the log [unlabeled IgG4] (M) versus the % of normalized HTRF signal max (= Delta F%_{unlabeled IgG1 antibody/}Delta F%ₘₐₓ x 100). A) Results obtained with biotinylated anti-human Fab 1 complexed with streptavidin-d2. B) Results obtained with anti-human Fab 2 directly labelled with d2. C) Results obtained with anti-human Fab 3 directly labelled with d2.

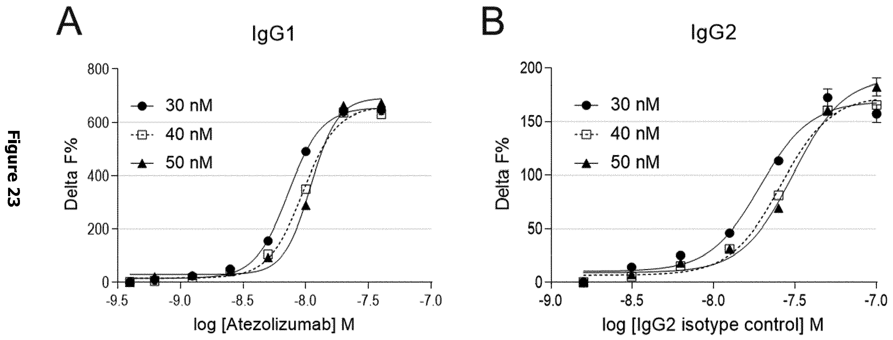

**Figure 23** presents the results of the binding of IgG1 and IgG2 antibodies to human C1q using different concentrations of biotinylated anti-human Fab antibody complexed to streptavidin-d2 (ratio 1/1), based on the TR-FRET method described in Figure 1. The sigmoidal dose response curves represent the log [antibody] (M) versus the normalized HTRF signal (Delta F%). A) Results obtained with the therapeutic antibody Atezolizumab (IgG1). B) Results obtained with the recombinant human IgG2 isotype control.

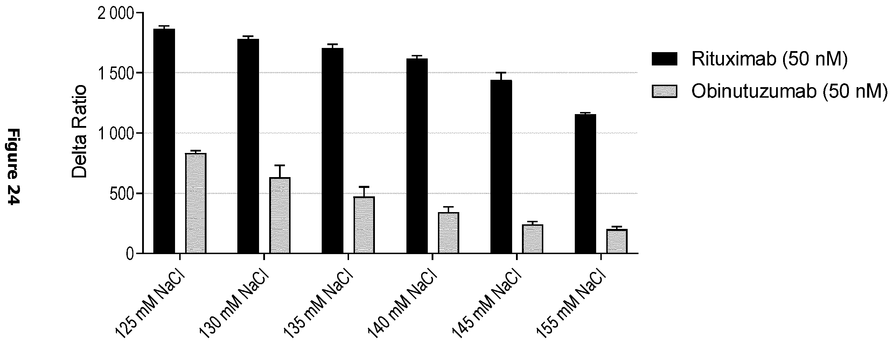

**Figure 24** presents the results of the binding of the therapeutic type I and type II anti-CD20 antibodies Rituximab and Obinutuzumab to human C1q in the presence of different concentrations of NaCl, based on the TR-FRET method described in Figure 1. The histogram represents the specific HTRF signal (Delta Ratio) obtained with 50 nM of each tested antibody in presence of the different concentrations of NaCl.

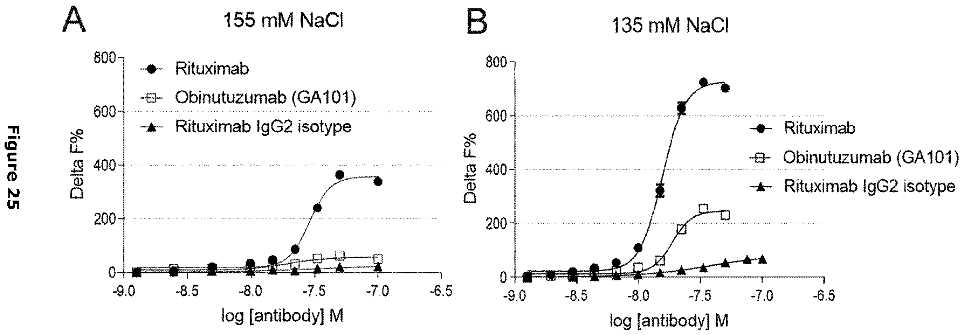

**Figure 25** presents the results of the binding of different IgG antibodies to human C1q in the presence of different concentrations of NaCl, based on the TR-FRET method described in Figure 1. The sigmoidal dose response curves represent the log [antibody] (M) versus the normalized HTRF signal (Delta F%). A) Results obtained in the presence of 155 mM NaCl. B) Results obtained in the presence of 135 mM NaCl.
**Figure 26** illustrates a method for determining the binding of an antibody to the C1q according to the prior art (ELISA), wherein antibodies are randomly immobilized on the surface (passive coating) [Ref. 1].
**Figure 27** illustrates a method for determining the binding of an antibody to the C1q according to the prior art (SPR), wherein antibodies are indirectly immobilized on the surface using Protein L [Ref. 2].

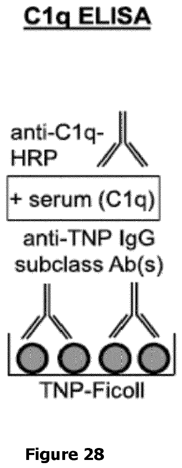

**Figure 28** illustrates a method for determining the binding of an antibody to the C1q according to the prior art (ELISA), wherein antigens are coated on the surface and then antibodies are added [Ref. 3].

### DETAILED DESCRIPTION OF THE INVENTION

### Definitions

The term "C1q" means complement component 1q. C1q is a protein involved in the complement system, which is part of the innate immune system. C1q together with C1r and C1s form the C1 complex. The Fc portion of antibodies can bind to C1q in order to activate the complement pathway of the complement system. According to the invention, the C1q is preferably from the species of the Fc portion of the tested antibody. For example, if the tested antibody is a chimeric (i.e. having a human Fc portion), a humanized or a human tested antibody, the C1q is preferably a human C1q.
In the sense of the invention, the term "ligand" refers to a molecule capable of binding specifically and reversibly to a target molecule. In the context of the present invention, the target molecule is a Fab, streptavidin or C1q. The present description therefore refers to "anti-Fab ligand", "anti-streptavidin ligand" or "anti-Clq ligand" respectively. The ligand can be of a protein nature (e.g. a protein or a peptide) or of a nucleotide nature (e.g. a DNA or a RNA). In the context of the invention, the ligand is advantageously chosen from an antibody, an antibody fragment, a protein, a peptide or an aptamer, preferably an antibody or an antibody fragment. The ligands that are used in the method of the invention are capable of binding their target molecule with sufficient affinity such that the ligand is useful as a diagnostic agent in targeting C1q.
By "antibody", also commonly called "immunoglobulin", includes a heterotetramer constituted by two heavy chains of approximately 50-70 kDa each (called the H chains, for Heavy) and two light chains of approximately 25 kDa each (called the L chains, for Light), joined together by intra- and interchain disulphide bridges. Each chain is constituted, in the N-terminal position, by a variable region or domain, called VL for the light chain, VH for the heavy chain and, in the C-terminal position, by a constant region, constituted by a single domain called CL for the light chain and of three or four domains called CH1, CH2, CH3, CH4, for the heavy chain. An antibody according to the invention may be of mammalian origin (e.g. human or mouse or camelid), humanized, chimeric, recombinant. It is preferably a monoclonal antibody produced recombinantly by genetically modified cells using techniques widely known to the skilled person. The antibody can be of any isotype, e.g. IgG, IgM, IgA, IgD or IgE, and of any subtypes such as IgG1, IgG2, IgG3 or IgG4.
The term "antibody fragment" means any part of an immunoglobulin obtained by enzymatic digestion or obtained by bio-engineering comprising at least one disulfide bridge and which is capable of binding to the antigen recognized by the whole antibody, such as Fv, Fab, Fab', Fab'-SH, F(ab')², diabodies, Single Domain Antibodies (also known as sdAb or nanobodies), single chain antibodies (e.g. scFv). Enzymatic digestion of immunoglobulins by pepsin generates a F(ab')² fragment and an Fc fragment split into several peptides. F(ab')² fragment is formed from two Fab' fragments linked by inter-chain disulfide bridges. The Fab fragment is formed by the variable domains (V_{L} and V_{H}), the CH₁ domain and the C_{L} domain. The Fab' fragment is formed by a Fab region and the hinge region. Fab'-SH refers to a Fab' fragment in which the cysteine residue of the hinge region carries a free thiol group.
The term "antigen" means the molecule or molecular structure, such as a protein, that is bound by the antigen-binding site of an antibody or antibody fragment. According to the invention the "biotinylated antigen" that may be used in the method of the invention is therefore a molecule or molecular structure, such as a protein, that is bound by the antigen-binding site of the tested antibody.
The term "biotinylated" according to the invention, means directly conjugated with a biotin molecule. According to the invention, the anti-Fab ligand may be conjugated with a biotin molecule that reacts with a lateral NH₂ group of a lysine or the NH₂ group of the N-terminus of an anti-Fab antibody, antibody fragment or the antigen. Biotinylation reagents and kits are commonly used in the art.
Streptavidin is a 52.8 kDa protein (tetramer) purified from the bacterium *Streptomyces avidinii.* Streptavidin homo-tetramers have an extraordinarily high affinity for biotin. With a dissociation constant (Kd) on the order of about 10⁻¹⁴ mol/L, the binding of biotin to streptavidin is one of the strongest non-covalent interactions known in nature. Streptavidin is used extensively in molecular biology due to the streptavidin-biotin complex.
The term "homogeneous assay", unlike the heterogeneous assay, refers to an assay format allowing to make an assay-measurement by a mix and measure procedure without the necessity to process samples by separation, such as without washing steps and/or without centrifugation steps.
The term "HPA" refers to "Homogeneous Proximity Assay". HPA is well known in the art and can be defined as a homogeneous assay that measures a signal resulting from the proximity between a donor (hereinafter also referred to as the "donor compound") and an acceptor (hereinafter also referred to as the "acceptor compound").

The term "HPA partner pair" refers to a pair consisting of a donor (hereinafter also referred to as the "donor compound") and an acceptor (hereinafter also referred to as the "acceptor compound"); when they are in proximity to each other and the donor is excited, these compounds emit a HPA signal.
The term "HPA signal" refers to any measurable signal representative of a HPA between a donor compound and an acceptor compound.
The term "RET" refers to "Resonance Energy Transfer". The RET may be a FRET or a BRET.
The term "FRET" refers to "Fluorescence Resonance Energy Transfer". FRET is defined as a non-radiative energy transfer resulting from a dipole-dipole interaction between a donor and an energy acceptor. This physical phenomenon requires an energetic compatibility between these molecules. This means that the donor's emission spectrum must cover, at least partially, the acceptor's absorption spectrum. In accordance with Förster's theory, FRET is a process that depends on the distance between the two molecules, donor and acceptor: when these molecules are in proximity to each other, a FRET signal will be emitted. The FRET may be a TR-FRET (Time Resolved FRET).
The term "BRET" refers to "Bioluminescence Resonance Energy Transfer".
In the sense of the invention, the streptavidin and the C1q are labeled with a member of a RET partner pair. The streptavidin and the C1q may be labeled directly or indirectly by methods well known to the skilled person, for example as described below.
In a specific embodiment, the streptavidin is labeled directly by covalent binding with a member of a RET partner pair.
In another specific embodiment, the C1q is labeled indirectly with an anti-Clq antibody labelled with a member of a RET partner pair. According to this embodiment of the invention, the skilled person in the art understands that the biotinylated anti-Fab ligand of step a) should not bind the Fab of the anti-Clq antibody. For example, the species of the Fab of the tested antibody is different from the species of the Fab of the anti-Clq antibody, such as the Fab of the tested antibody is human and the Fab of the anti-Clq antibody is murine.

The term "RET partner pair" refers to a pair consisting of an energy donor compound (hereinafter referred to as the "donor compound") and an energy acceptor compound (hereinafter referred to as the "acceptor compound"); when they are in proximity to each other and excited at the excitation wavelength of the donor compound, these compounds emit a RET signal. It is known that for two compounds to be RET partners, the emission spectrum of the donor compound must partially cover the excitation spectrum of the acceptor compound. For example, "FRET partner pair" is used when using a fluorescent donor compound and an acceptor compound or "BRET partner pair" is used when using a donor bioluminescent compound and an acceptor compound.
The term "RET signal" refers to any measurable signal representative of a RET between a donor compound and an acceptor compound. For example, a FRET signal may therefore be a variation in the intensity or lifetime of fluorescence of the fluorescent donor compound or the acceptor compound when the latter is fluorescent.
The term "LOCI" means "Luminescent Oxygen Channeling Assay". The LOCI is an induced luminescence immunoassay which is described in U.S. Pat. No. 5,340,716, the entire contents of which are expressly incorporated herein by reference. The LOCI technology involves a homogeneous assay (i.e., no separation steps involved) that has high sensitivity, and the assay uses several reagents and requires that two of these reagents (referred to as a "donor bead" and a "acceptor bead") held by other immunoassay reagents to be in close proximity to achieve a signal. Upon exposure to light at a certain wavelength, the donor bead releases singlet oxygen, and if the two beads are in close proximity, the singlet oxygen is transferred to the acceptor bead; this causes a chemical reaction that results in the acceptor bead giving off light that can be measured at a different wavelength.
The term "LOCI partner pair" refers to a pair consisting of a singlet oxygen donor compound (hereinafter referred to as the "donor bead") and a singlet oxygen acceptor compound (hereinafter referred to as the "acceptor bead"); when they are in proximity to each other and excited at the excitation wavelength of the donor bead, these compounds emit a LOCI signal. The term "LOCI signal" refers to any measurable signal representative of a singlet oxygen transfer between a donor bead and an acceptor bead. For example, a LOCI signal may therefore be a variation in the intensity of the light emitted by the acceptor bead.
The term "container" refers to a well of a plate, a test tube or other suitable container for mixing a membrane preparation with the reagents necessary for the implementation of the method according to the invention.

### Method for determining the binding of an antibody to C1q

According to a first aspect, the invention relates to an *in vitro* method for determining the binding of an antibody (tested antibody) to a complement component 1q (C1q), i.e. an *in vitro* method for assessing the ability of an antibody (tested antibody) to mediate CDC. According to a first aspect, the invention relates to an *in vitro* method for determining the binding of an antibody (tested antibody) to a complement component 1q (C1q), comprising the following steps:
a) Contacting into a measurement medium:
   - a tested antibody,
   - a biotinylated anti-Fab ligand, said biotinylated anti-Fab ligand being able to bind the Fab region of the tested antibody,
   - a streptavidin directly or indirectly labelled with the first member of a pair of HPA (Homogeneous Proximity Assay) partners, and
   - a C1q directly or indirectly labelled with the second member of a pair of HPA partners;
b) Measuring the HPA signal in the measurement medium, the existence of a HPA signal being representative of the binding of the tested antibody to the C1q.

### Step a)

According to the present invention, step a) consists in contacting into a measurement medium, the following four elements:
- a tested antibody, said tested antibody being preferably an IgG or an IgM,
- a biotinylated anti-Fab ligand, said biotinylated anti-Fab ligand being able to bind the Fab region of the tested antibody,
- a streptavidin directly or indirectly labelled with the first member of a pair of HPA (Homogeneous Proximity Assay) partners, and
- a C1q directly or indirectly labelled with the second member of a pair of HPA partners;

The measurement medium may be contained in a container. The different elements can be introduced into the measurement medium sequentially in any order, or simultaneously or almost simultaneously. For example, the different elements are introduced into the measurement medium in the following order: the biotinylated anti-Fab ligand, the streptavidin directly or indirectly labelled with the first member of a pair of HPA partners, the tested antibody and the C1q directly or indirectly labelled with the second member of a pair of HPA partners.

The mixing of the elements makes it possible to obtain a reaction solution adapted to the implementation of a HPA. Therefore, other elements can be added to the measurement medium to adapt the solution to the implementation of the HPA. For example, the measurement medium may also comprise a buffer, the antigen of the tested antibody (assuming that the antigen should not be biotinylated), and/or any compound that is suitable for implementing the method of the invention, such as albumin, a surfactant, a preservative.

In some embodiments, the measurement medium has an osmolarity adapted to detect the HPA signal. Preferably, the measurement medium has an osmolarity from 250 mOsm/L to 500 mOsm/L, preferably from 250 mOsm/L to 350 mOsm/L, such as from 275 mOsm/L to 325 mOsm/L, such as from 275 mOsm/L to 310 mOsm/L, for example from 280 mOsm/L to 300 mOsm/L, for example about 282 mOsm/L.

In some embodiments, the measurement medium comprises a sodium buffer, such as NaCl and/or a phosphate buffer, such as Na₂HPO₄ and/or KH₂PO₄, in an amount sufficient to detect the HPA signal. Advantageously, the measurement medium may comprise from 50 mM to 250 mM of sodium buffer (e.g. NaCl), such as from 100 mM to 200 mM, for example from 125 mM to 150 mM, for example 135 mM. A NaCl concentration that is suitable for implementing the method of the invention can be easily determined by the skilled person in the art, for example as explained in Example 9.

In a preferred embodiment, the pH of the measurement medium is suitable to detect the HPA signal. Advantageously, the pH is from pH 6.0 to pH 8.0, such as from pH 7.0 to pH 8.0, such as from pH 7.2 to pH 7.6, such as pH 7.4. A pH that is suitable for implementing the method of the invention can be easily determined by the skilled person in the art.

In a preferred embodiment, the measurement medium comprises (ii) a phosphate buffer, such as Na₂HPO₄ and/or KH₂PO₄, (ii) a sodium buffer, such as NaCl and, eventually, (iii) albumin.

The measurement medium may also comprise a surfactant, such as Tween-20 and/or a preservative, such as ProClin-300.

In a very specific embodiment, the measurement medium comprises Na₂HPO₄ 3 mM, KH₂PO₄ 1 mM, NaCl 135 mM, BSA (preferably protease free and IgG free) 0.1%, Tween-20 0.05%, ProClin-300 0.01%, pH 7.4.

According to the invention, the tested antibody may be an IgG or an IgM. IgG and IgM are known to bind C1q. Preferably, the tested antibody is an IgG, such as an IgG1, an IgG2 or an IgG4. In some embodiments, the tested antibody is an IgG1 or an IgG2.

The tested antibody may be a chimeric antibody, a humanized antibody or a human antibody.

According to the invention, the biotinylated anti-Fab ligand is able to bind the Fab region of the tested antibody. As explained above, the anti-Fab ligand is advantageously selected from an anti-Fab antibody, an anti-Fab antibody fragment, an anti-Fab peptide, an anti-Fab aptamer or an antigen. Even if the anti-Fab antibody can be an anti-Fab polyclonal antibody, the anti-Fab antibody is preferably an anti-Fab monoclonal antibody.

In one embodiment, the biotinylated anti-Fab ligand binds the C_{L} region and/or the CH₁ region of the tested antibody. In this embodiment, the anti-Fab ligand is preferably an anti-Fab antibody or an anti-Fab antibody fragment.

In another embodiment, the biotinylated anti-Fab ligand binds the variable region of the tested antibody, such as V_{H} and/or V_{L}. In this embodiment, the anti-Fab ligand is preferably an antigen.

The skilled person will have no difficulty to select a suitable biotinylated anti-Fab ligand depending on the tested antibody. For example, if the tested antibody is a human antibody, a humanized antibody or a chimeric antibody, the biotinylated anti-Fab ligand may bind the C_{L} region and/or the CH₁ region of the tested antibody. Thus, the biotinylated anti-Fab ligand may be a biotinylated anti-human Fab ligand, such as a biotinylated anti-human Fab antibody, a biotinylated anti-human Fab antibody fragment, a biotinylated anti-human Fab peptide, a biotinylated anti-human Fab aptamer or a biotinylated antigen.

Anti-Fab ligands are commercially available, either biotinylated or non-biotinylated, such as biotinylated or non-biotinylated anti-Fab antibodies. Examples of biotinylated and non-biotinylated anti-Fab ligands are : the biotinylated recombinant human TNF-α protein from Abcam (#ab167747), the recombinant human TNF-α protein from R&D Systems (#10291-TA), the anti-human IgG Fab (mouse IgG2b, monoclonal antibody, clone 4A11) from ThermoFisher Scientific (#SA1-19255), the anti-human IgG Fab (mouse IgG2b, monoclonal antibody, clone 2A11) from GeneTex (#GTX27497), the anti-human IgG Fab (goat IgG, polyclonal antibody) from Sigma-Aldrich (#15260), the biotinylated anti-human Ig (IgG, IgM, and IgA) Fab (goat IgG, polyclonal antibody) from SouthernBiotech (#2085-08), the biotinylated anti-human IgG Fab (goat IgG, polyclonal antibody) from Sigma-Aldrich (#SAB3701251), the biotinylated anti-human IgG Fab (goat IgG, polyclonal antibody) from Jackson ImmunoResearch Inc. (#109-065-006), the biotinylated anti-human IgG Fab (chicken IgY, polyclonal antibody) from ThermoFisher Scientific (#SA1-72044). If the anti-Fab ligand is non-biotinylated, the skilled person will have no difficulty to biotinylated it with standard procedures known in the art, such as biotinylation of the anti-human IgG Fab (mouse IgG2b, monoclonal antibody, clone 4A11) from ThermoFisher Scientific (#SA1-19255), the anti-human IgG Fab (mouse IgG2b, monoclonal antibody, clone 2A11) from GeneTex (#GTX27497), or the anti-human IgG Fab (goat IgG, polyclonal antibody) from Sigma-Aldrich (#15260). Thus, the skilled person will have no difficulty to obtain a biotinylated anti-Fab ligand for any and all tested antibodies.

In a specific embodiment, the biotinylated anti-Fab ligand is a murine biotinylated anti-Fab antibody or antibody fragment, such as a mouse biotinylated anti-Fab antibody or antibody fragment. It has been shown by the Applicant that the method of the invention can be implemented with murine biotinylated anti-Fab antibodies or antibody fragments. Indeed, it has been shown by the Applicant that a murine biotinylated anti-Fab antibody or antibody fragment does not interfere with the binding of the tested antibody with the C1q, and therefore does not alter the RET signal in step b).

Preferably, the biotinylated anti-Fab ligand is in excess in comparison with the tested antibody. The skilled person will have no difficulty to identify a concentration of the biotinylated anti-Fab ligand that is suitable for the implementation of the method of the invention, for example as explained in Example 8. In some embodiments, the concentration of the biotinylated anti-Fab ligand may be from 10 to 200 times, such as from 50 to 150 times, such as 100 times, the dissociation constant (Kd), in nM, of said biotinylated anti-Fab ligand to the tested antibody. For example, if the Kd is 0.5 nM, the concentration of the biotinylated anti-Fab ligand is 50 nM. The skilled person will have no difficulty to determine the Kd of the biotinylated anti-Fab ligand to the tested antibody, for example as explained in Example 7.

The streptavidin and the C1q can be labeled directly or indirectly.

Direct labeling of the streptavidin and the C1q with a member of a pair of HPA partners, for example a fluorescent compound when HPA is FRET, can be carried out by conventional methods known to the skilled person, based on the presence of reactive groups on the streptavidin and the C1q. For example, the following reactive groups may be used: the terminal amino group, carboxylate groups of aspartic and glutamic acids, amino groups of lysines, guanidine groups of arginines, thiol groups of cysteines, phenol groups of tyrosines, indole rings of tryptophans, thioether groups of methionines, imidazole groups of histidines. The streptavidin and the C1q may also be labeled indirectly, for example by introducing into the measurement medium an antibody or antibody fragment, which is itself covalently bound to an acceptor/donor compound, this second antibody or antibody fragment specifically recognizing the streptavidin or the C1q. Obviously, it is important that the indirect labelling of the streptavidin or of the C1q with a member of a pair of HPA partners does not involve any biotinylation of the streptavidin, of the C1q or of the member of a pair of HPA partners.

Advantageously, the streptavidin is directly labelled with the first member of a pair of HPA partners and the C1q is indirectly labelled with an anti-Clq ligand labelled, such as an anti-Clq antibody or antibody fragment, with the second member of a pair of HPA partners.

In some embodiments, (i) the first member of a pair of HPA partners is an acceptor and the second member of a pair of HPA partners is a donor; or (ii) the first member of a pair of HPA partners is a donor and the second member of a pair of HPA partners is an acceptor.

The skilled person will have no difficulty to identify the concentration ratio [Streptavidin] : [biotinylated anti-Fab ligand] that is suitable for the implementation of the method of the invention. For example, the concentration ratio [Streptavidin] : [biotinylated anti-Fab ligand] may be about 1 : 1.

### Step b)

Step b) consists in measuring the HPA signal in the measurement medium, the existence of a HPA signal being representative of the binding of the tested antibody to the C1q.

In the present description, the term "the existence" in step b) can be replaced by the term "the intensity". The skilled person in the art understands that the intensity of the HPA signal increases when the binding of the tested antibody to the C1q increases.

The measured signal corresponds to the signal obtained in the measurement medium in the presence of the tested antibody. The measurement can be made by conventional methods widely known to the skilled person and does not pose any particular problem. A device is usually used to detect and measure the HPA signal, such as the PHERAstar FS microplate reader (BMG Labtech) with TR-FRET or the VICTOR Nivo (PerkinElmer) with ALPHA.

In a specific embodiment, the HPA signal of a tested antibody is compared to the HPA signal obtained with another antibody, such as a standard antibody or a reference antibody. In this specific embodiment, the HPA signal of the other antibody is obtained by implementing the method of the invention using the other antibody, i.e. by replacing the tested antibody by the other antibody. The other antibody may be a standard antibody for which the level of the HPA signal is already known or a reference antibody to which the tested antibody is compared. The comparison is particularly interesting when the tested antibody is a biosimilar antibody and the reference antibody is a princeps antibody. In this specific embodiment, the HPA signal in the measurement medium comprising the tested antibody can be performed in a first container, and the HPA signal in the measurement medium comprising the other antibody can be performed in a second container; and then the HPA signals obtained in each of the two containers are compared.

In some embodiments, steps (a) and (b) are repeated with different concentrations of the tested antibody and, preferably, said method comprises an additional step (c) of determining:
- the dissociation constant (Kd) of the binding of the tested antibody (or of the other antibody) to the C1q, and/or
- the EC₅₀ of the binding of the tested antibody (or of the other antibody) to the C1q.

According to the invention, the HPA may be selected from (i) Amplified Luminescent Oxygen Channeling Immunoassay (LOCI), such as Amplified Luminescence Proximity Homogeneous Assay (ALPHA), (ii) Resonance Energy Transfer (RET), such as Fluorescence Resonance Energy Transfer (FRET), and (iii) Spatial Proximity Analyte Reagent Capture Luminescence (SPARCL). Therefore, in the present description, the term "HAS" can be replaced by "RET", "LOCI" or "SPARCL". Specific embodiments of "RET", "LOCI" and "SPARCL" are detailed below.

### Method SPARCL

SPARCL technology is a proximity dependent, non-separation, chemiluminescent detection method. In a SPARCL assay, a chemiluminescent substrate (acridan) is brought into the proximity of an oxidative enzyme (horseradish peroxidase, HRP) through the specific antigen/antibody interaction. A flash of light proportional to the quantity of analyte present in the sample is generated upon addition of a trigger solution containing H₂O₂ and para-hydroxycinnamic acid (pHCA).

### Method RET

If the HPA is a RET, the invention therefore relates to an *in vitro* method for or determining the binding of an antibody (tested antibody) to a complement component 1q (C1q), comprising the following steps:
a) Contacting into a measurement medium:
   - a tested antibody,
   - a biotinylated anti-Fab ligand, said biotinylated anti-Fab ligand being able to bind the Fab region of the tested antibody,
   - a streptavidin directly or indirectly labelled with the first member of a pair of RET (Resonance Energy Transfer) partners, and
   - a C1q directly or indirectly labelled with the second member of a pair of RET partners;
b) Measuring the RET signal in the measurement medium, the existence of a RET signal being representative of the binding of the tested antibody to the C1q.

Other elements can be added to the measurement medium to adapt the solution to the implementation of the RET. For example, coelenterazine h (benzyl-coelenterazine) or bisdeoxycoelenterazine (DeepBlueC^{™}) or didhydrocoelenterazine (coelenterazine-400a) or D-luciferin can be added.

### • Labeling of the streptavidin and the C1q with a member of a pair of RET partners

Reactive groups can form a covalent bond with a reactive group carried by a member of a RET partners pair. The appropriate reactive groups, carried by the member of a pair of RET partners, are well known to the skilled person, e.g. a donor compound or an acceptor compound functionalized with a maleimide group will for example be capable of covalently binding with thiol groups carried by cysteines carried by a protein or peptide, e.g. the streptavidin or the C1q. Similarly, a donor/acceptor compound carrying an N-hydroxysuccinimide ester will be able to covalently bind to an amine containing a protein or peptide.

In the context of the invention, the streptavidin and the C1q may be each labeled with a member of a pair of RET partners, one of the members of the pair being a fluorescent donor or luminescent donor compound and the other member of the pair being a fluorescent acceptor compound or a non-fluorescent acceptor compound (quencher).

### • Labeling for the implementation of FRET

In a specific embodiment, the RET is a FRET. Therefore, the streptavidin and the C1q are each labeled with a member of a FRET partners pair, i.e. a fluorescent donor compound or a fluorescent energy-accepting compound.

The selection of the FRET partners pair to obtain a FRET signal is within the reach of the skilled person. For example, donor-acceptor pairs that can be used to study FRET phenomena are described in the work by Joseph R. Lakowicz (Principles of fluorescence spectroscopy, 2nd edition 338), to which the skilled person may refer.

### Fluorescent donor compounds

Long-lived energy-donating fluorescent compounds (> 0.1 ms, preferably in the range 0.5 to 6 ms), in particular rare earth chelates or cryptates, are advantageous since they allow time-resolved FRET without having to deal with a large part of the background noise emitted by the measurement medium. For this reason, they are generally preferred for the implementation of the process according to the invention. Advantageously, these compounds are lanthanide complexes. These complexes (such as chelates or cryptates) are particularly suitable as a member of the energy-donating FRET pair.

Complexes of europium (Eu³⁺), terbium (Tb³⁺), or samarium (Sm³⁺) are rare earth complexes also suitable for the invention, with europium (Eu³⁺) and terbium (Tb³⁺) complexes being particularly preferred.

In one embodiment, the lanthanide complex Ln³⁺ is chosen from one of the following complexes:

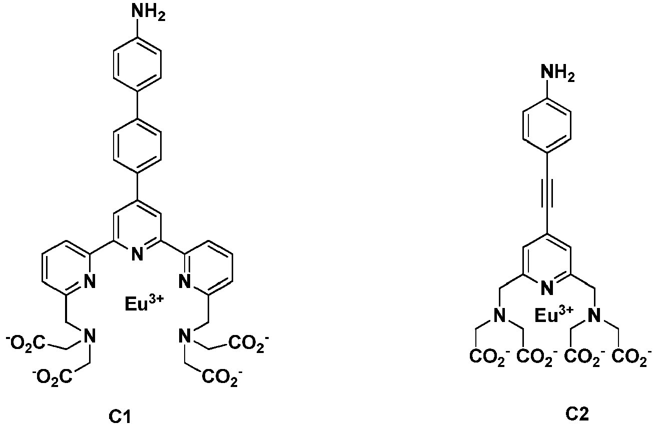

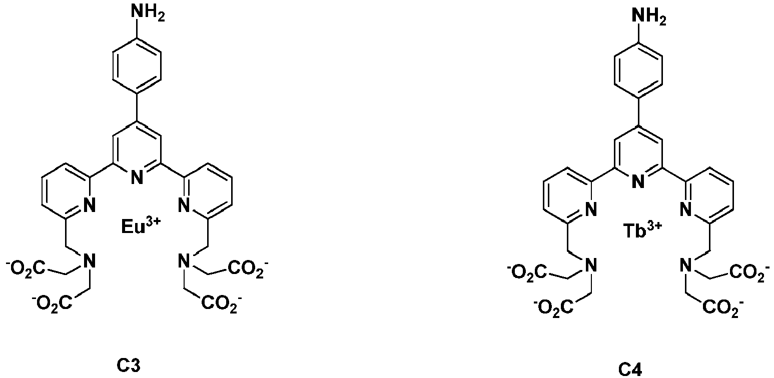

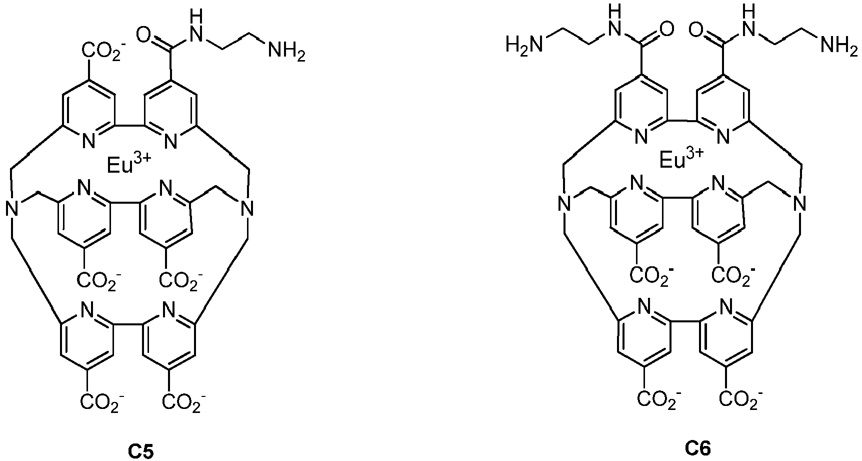

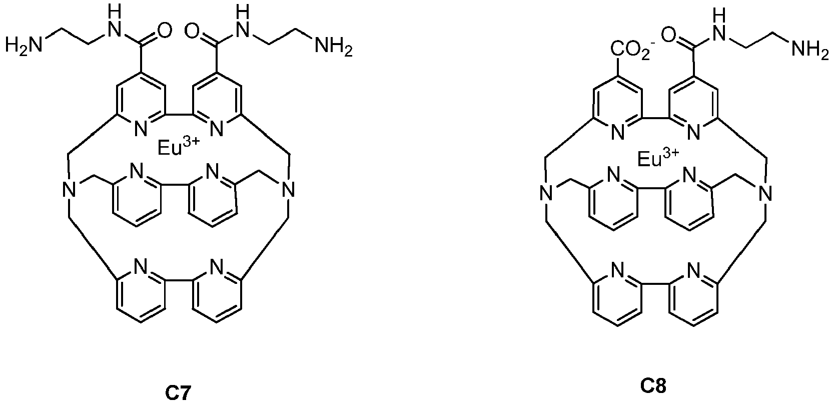

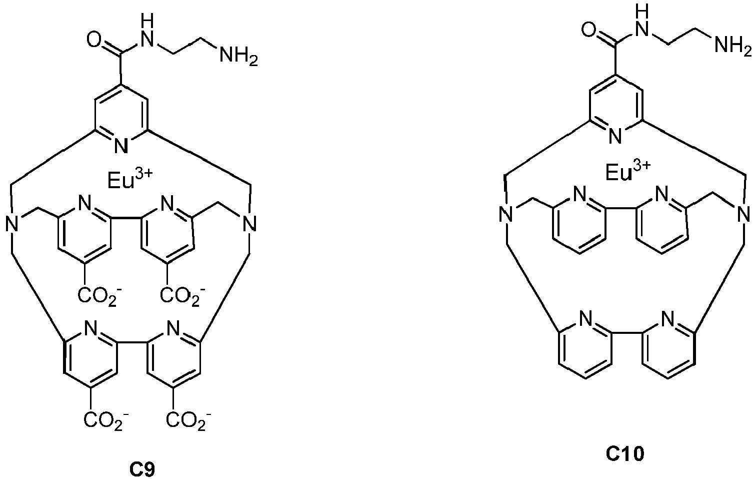

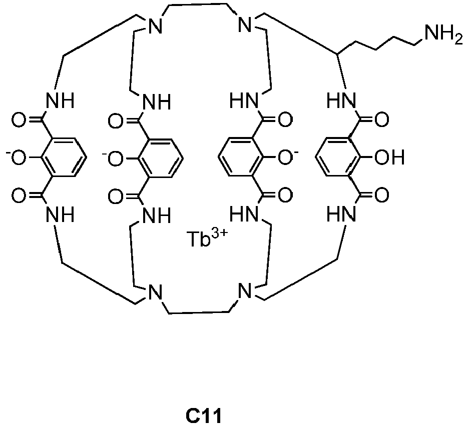

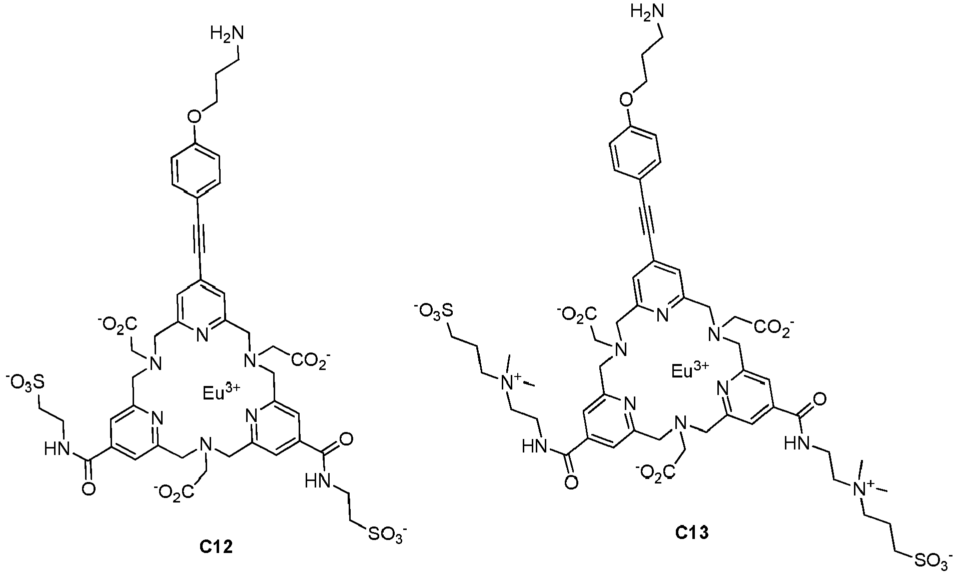

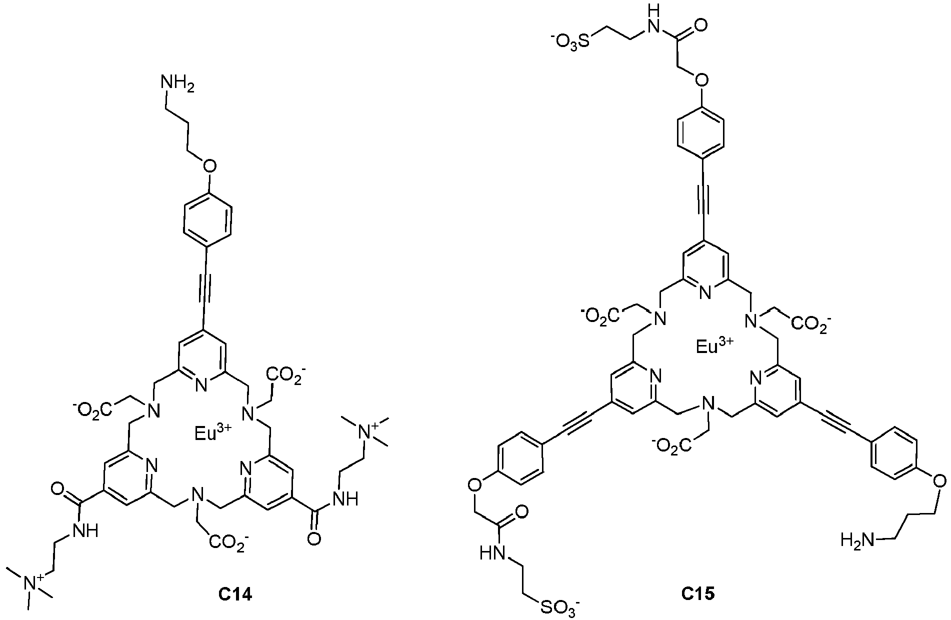

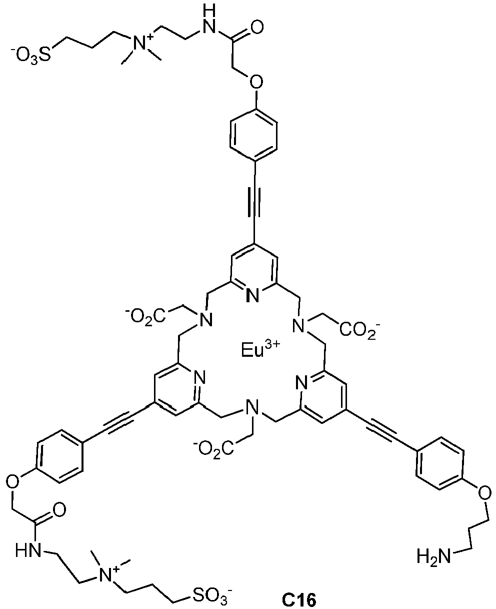

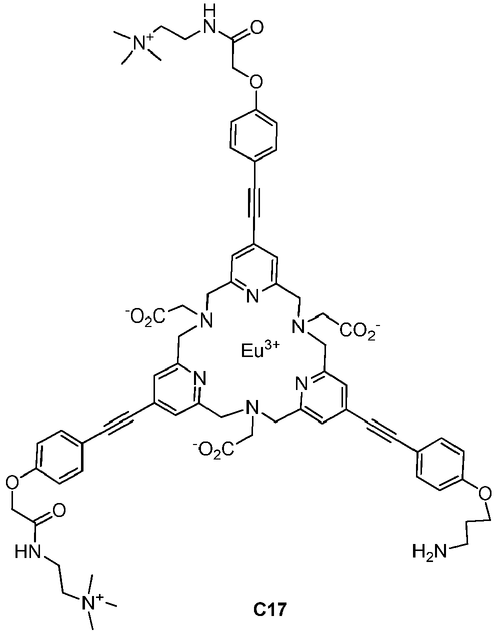

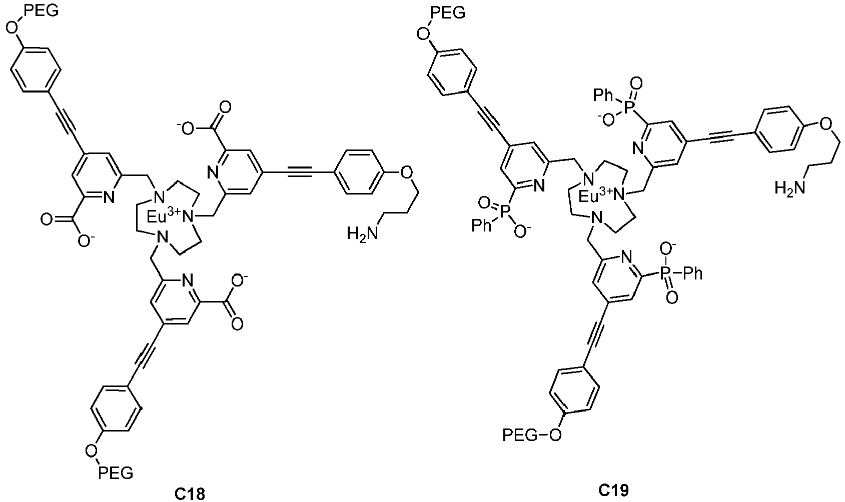

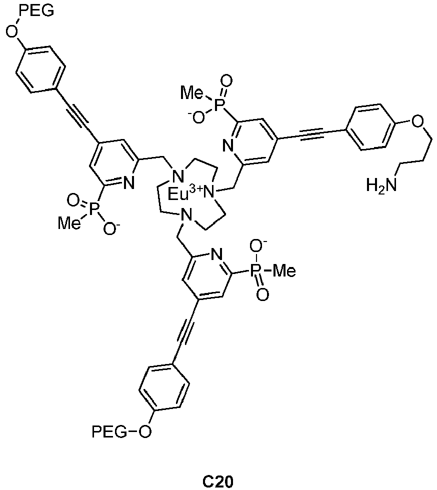

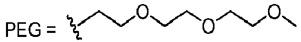

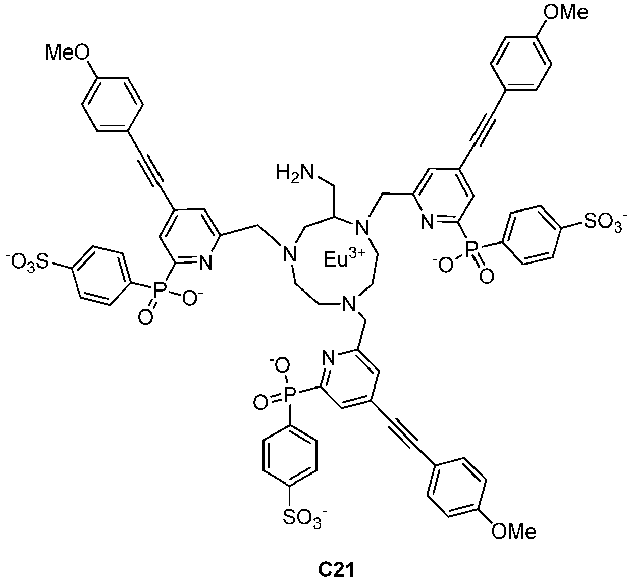

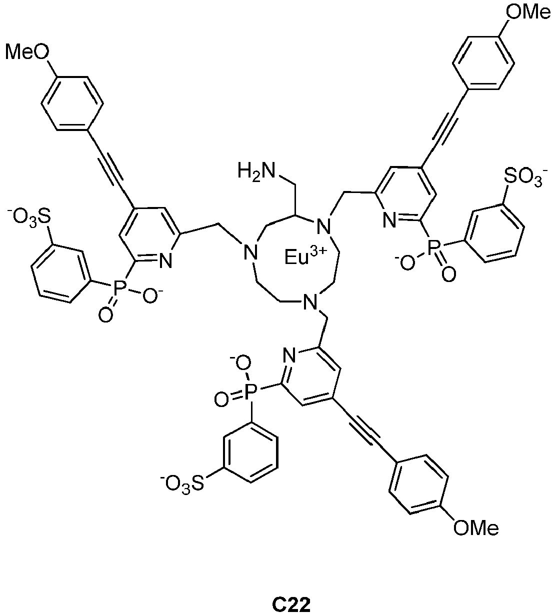

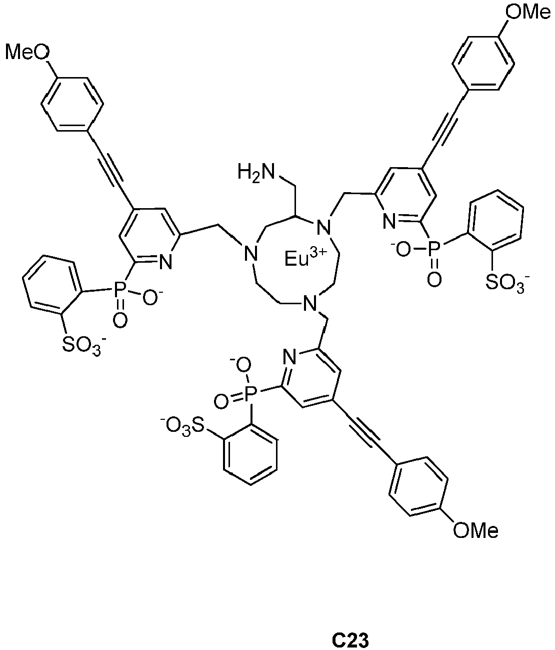

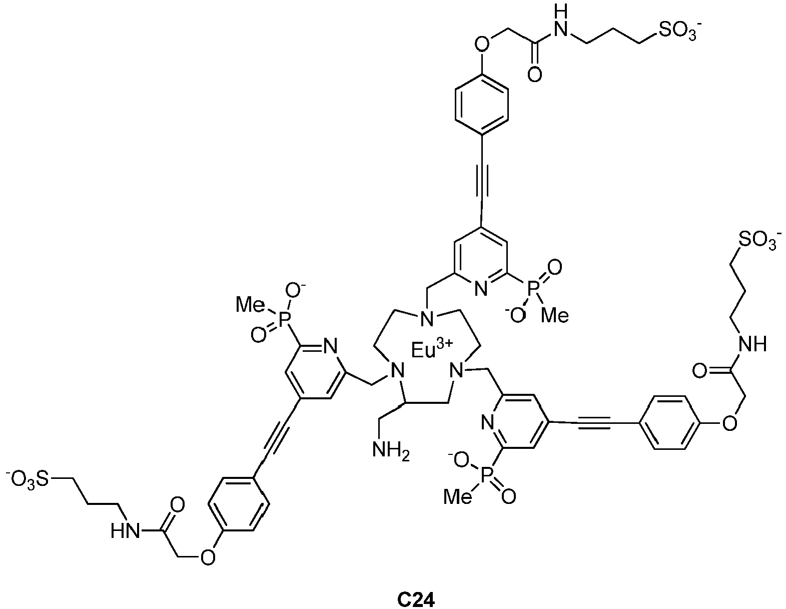

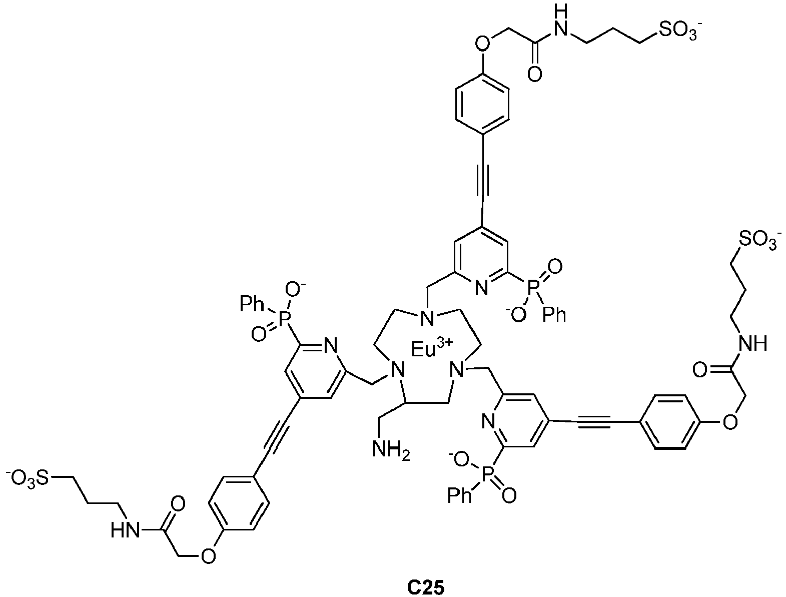

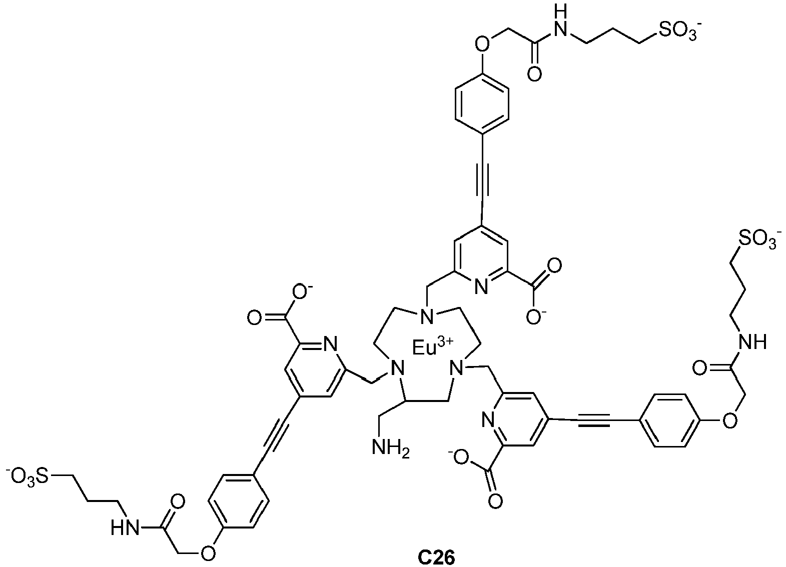

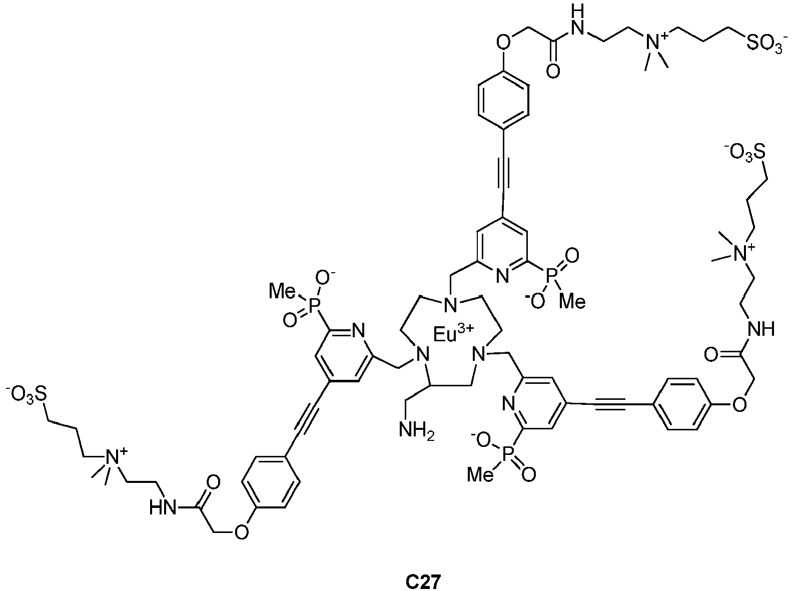

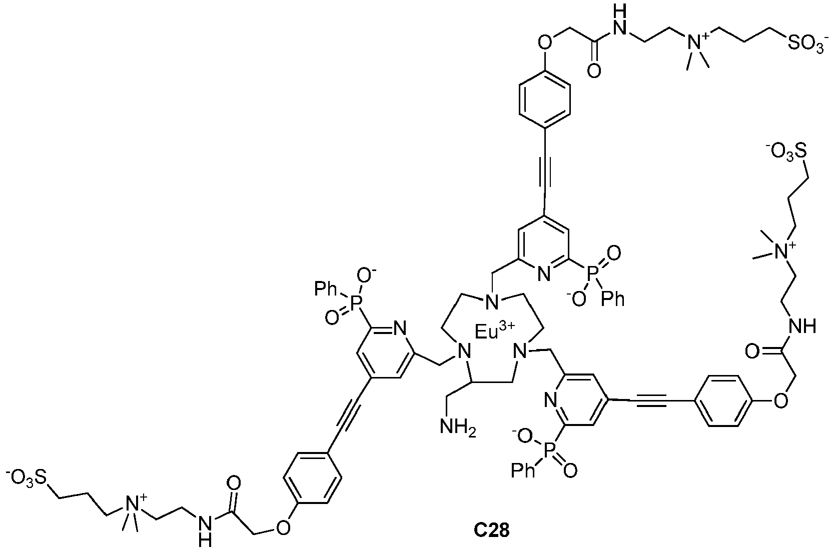

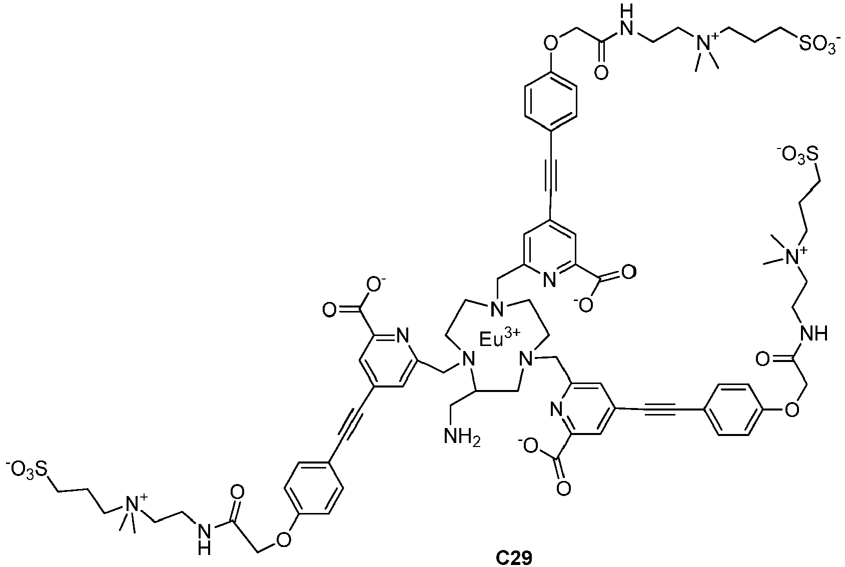

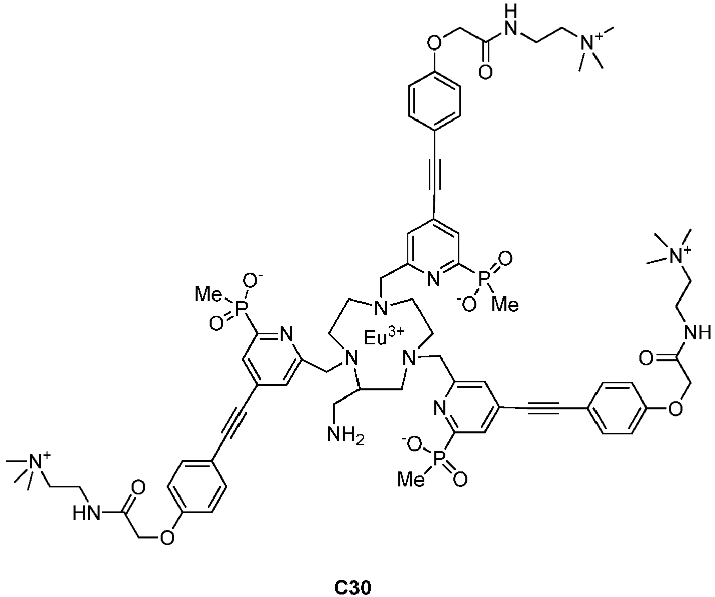

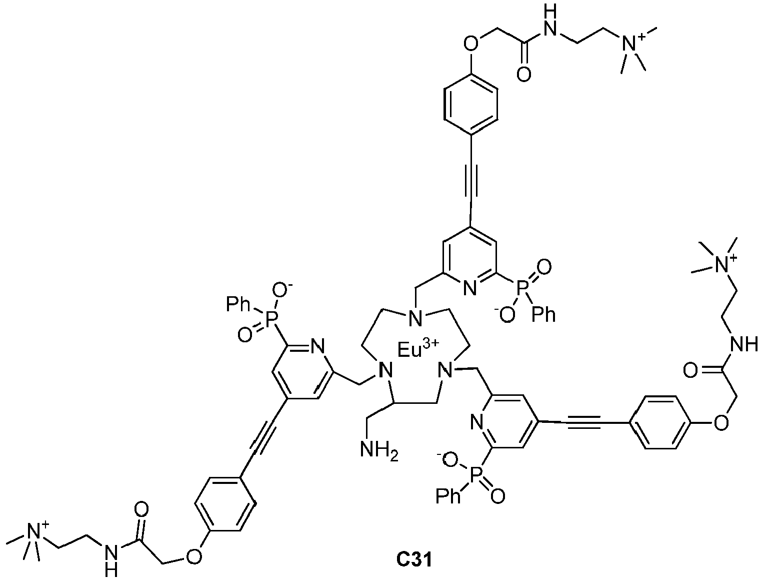

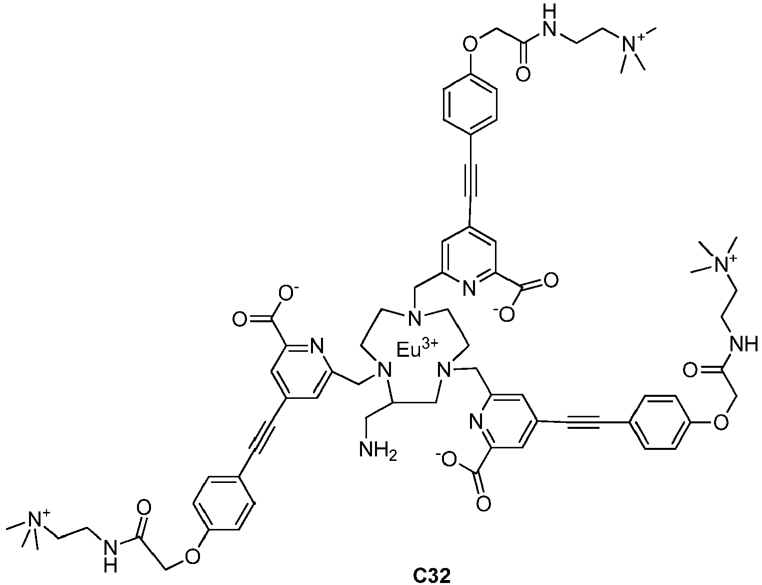

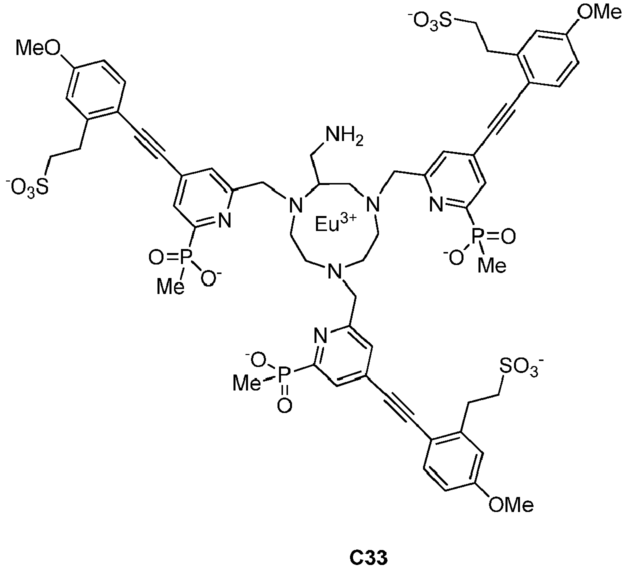

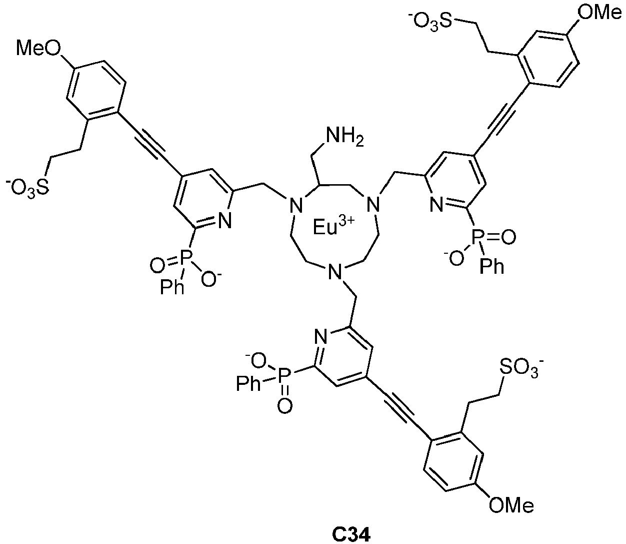

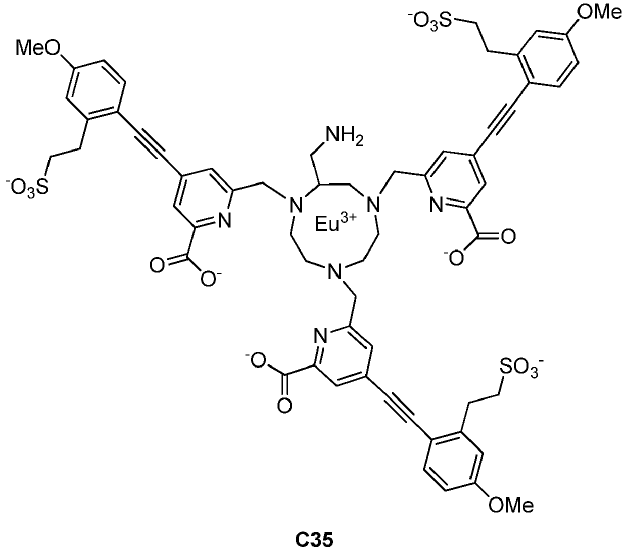

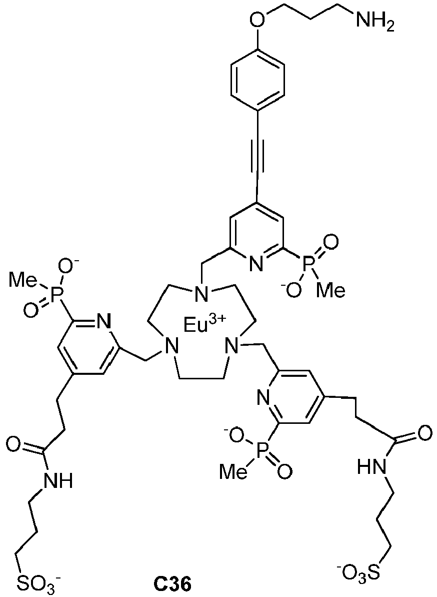

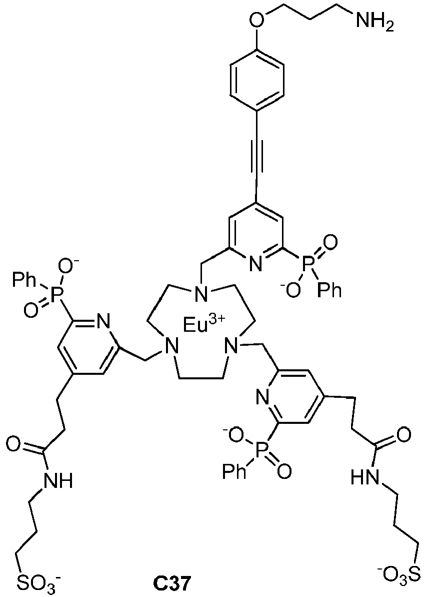

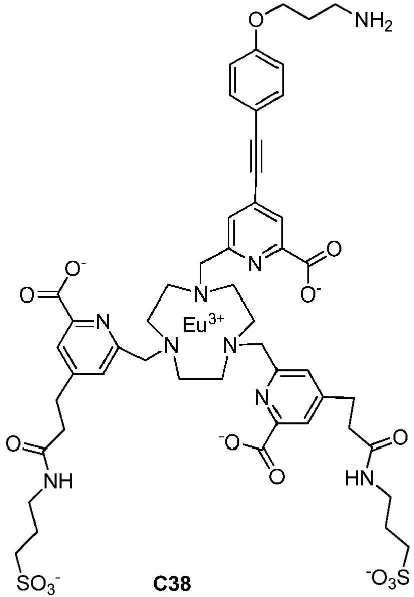

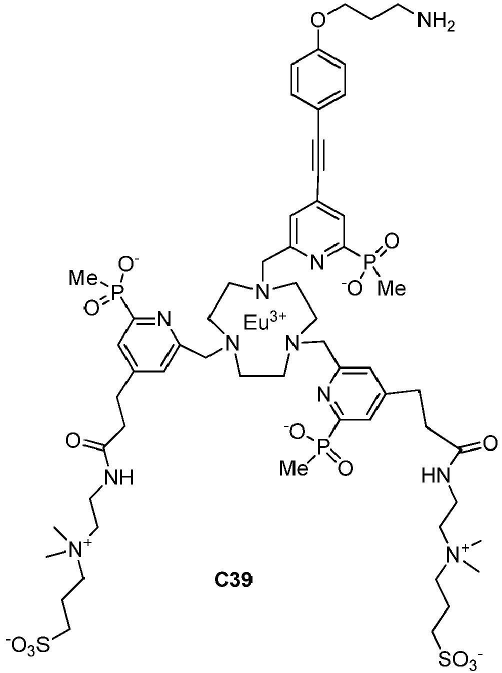

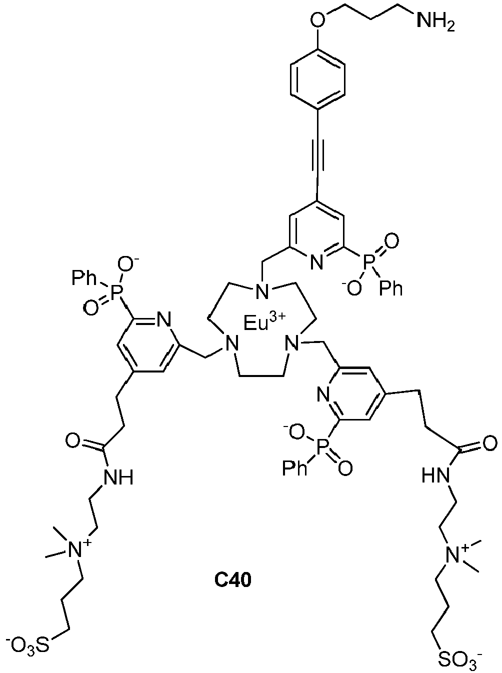

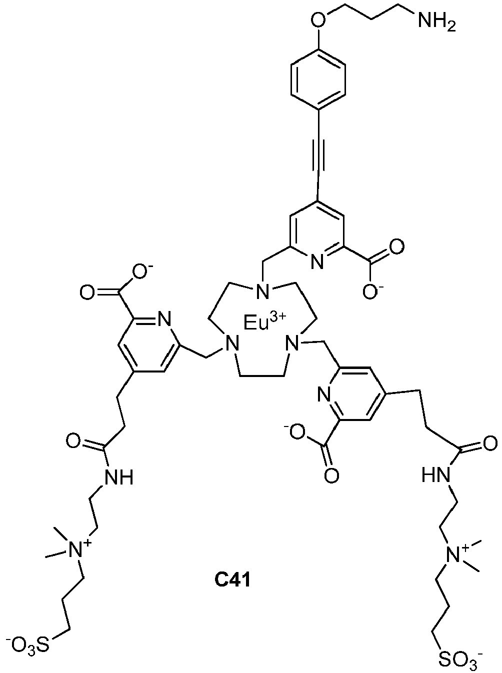

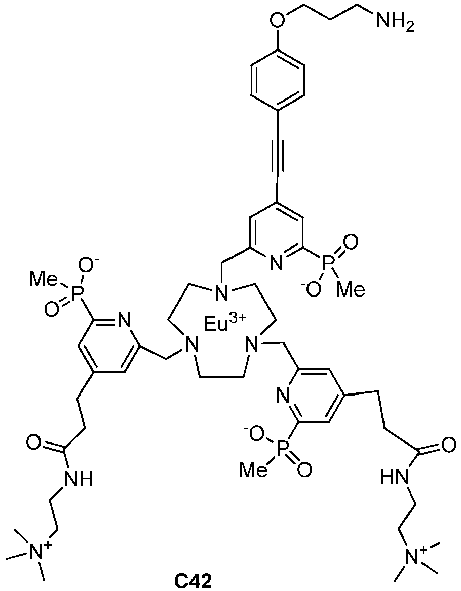

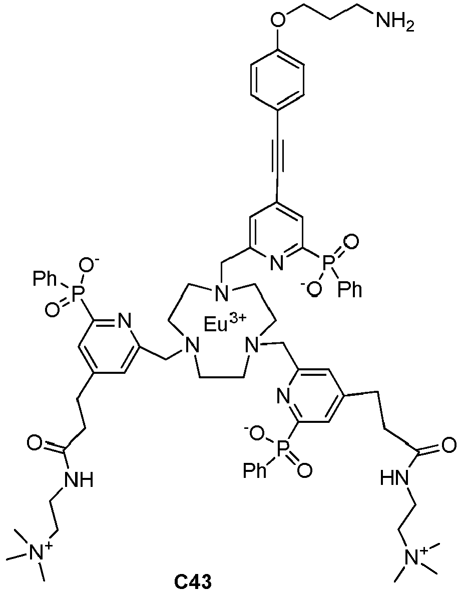

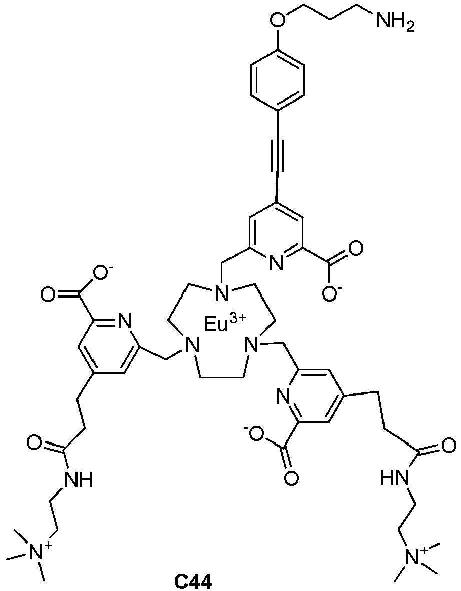

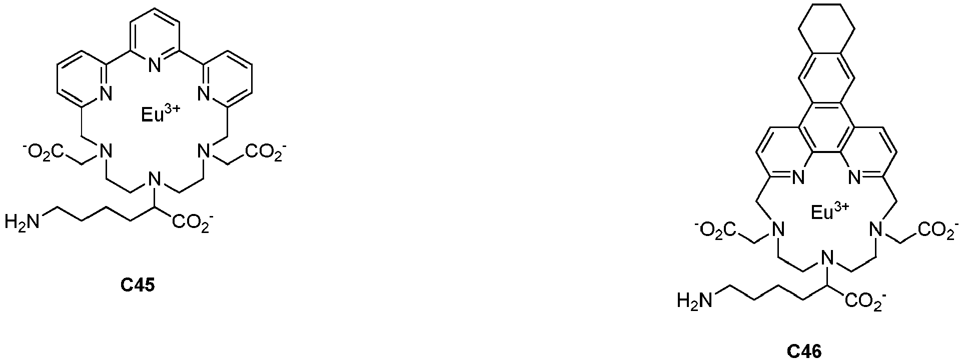

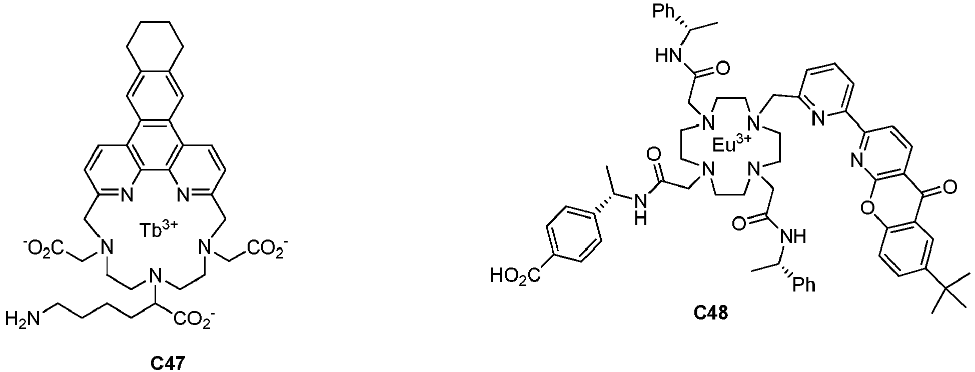

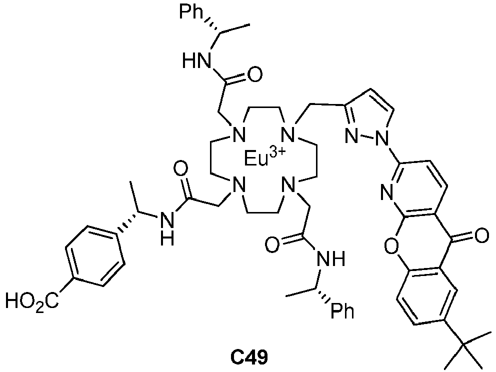

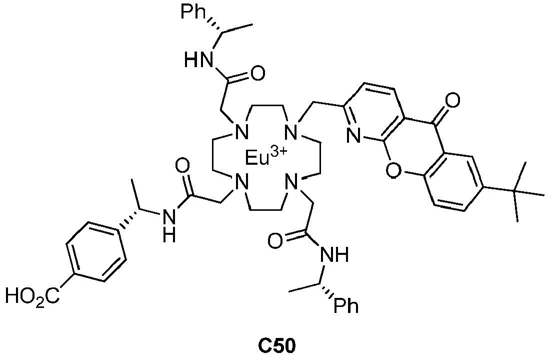

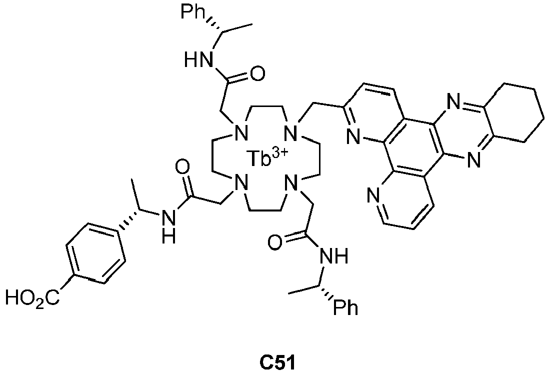

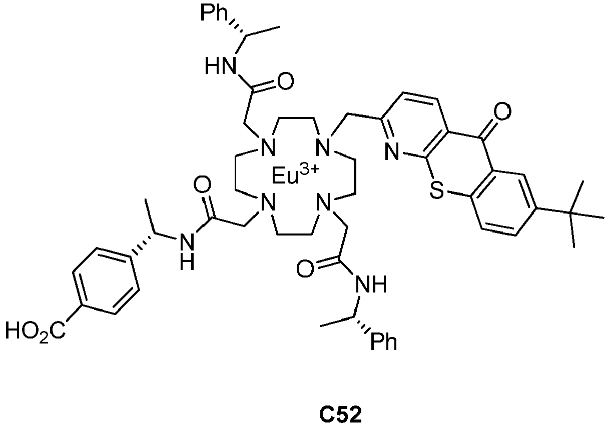

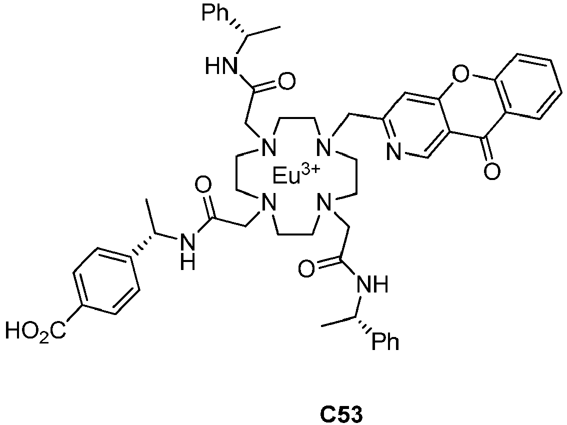

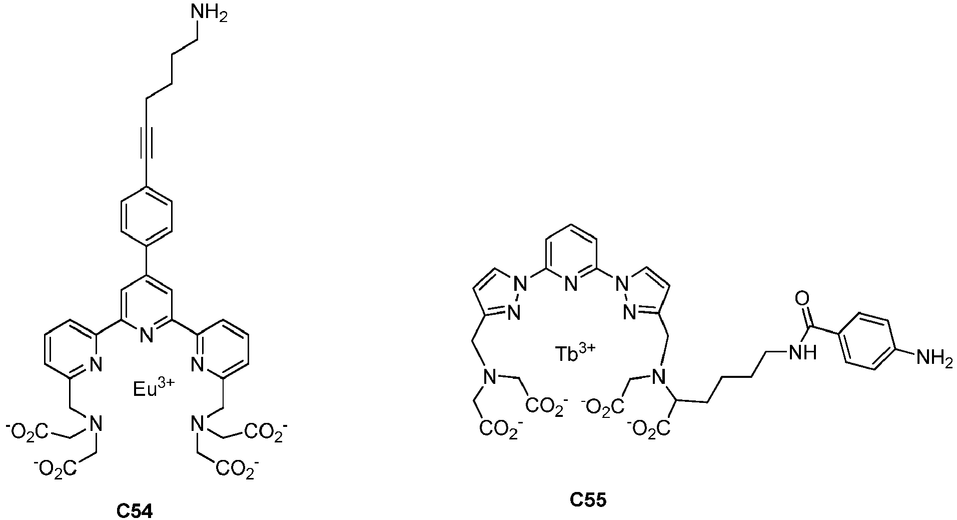

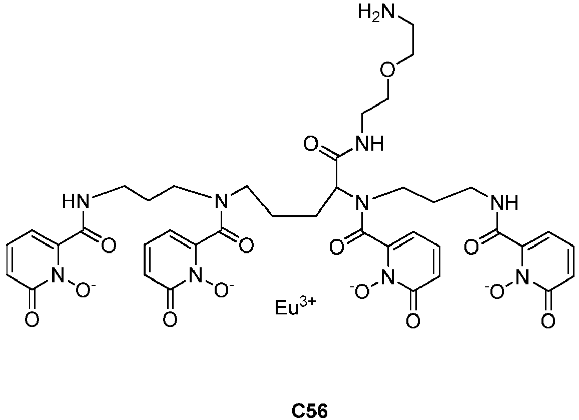

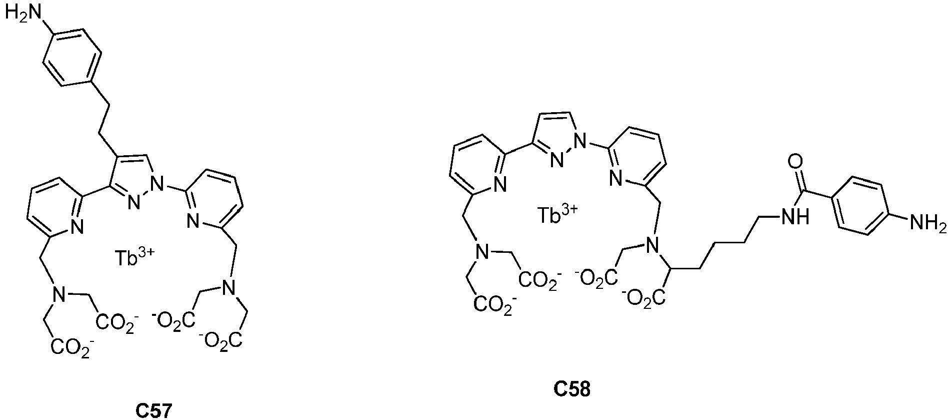

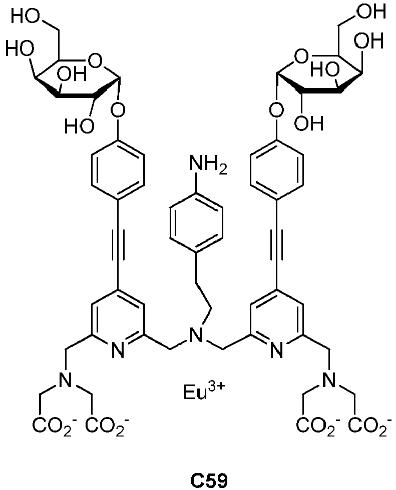

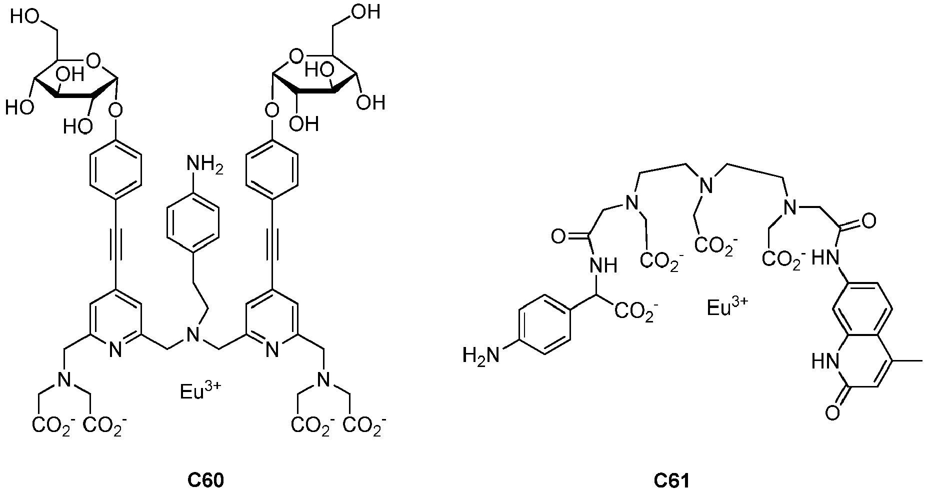

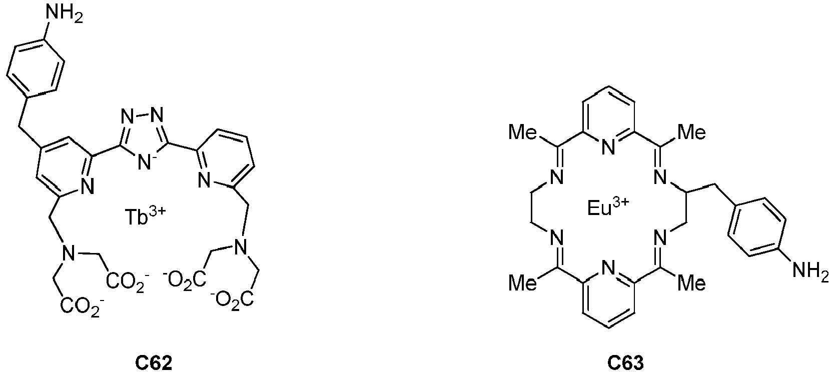

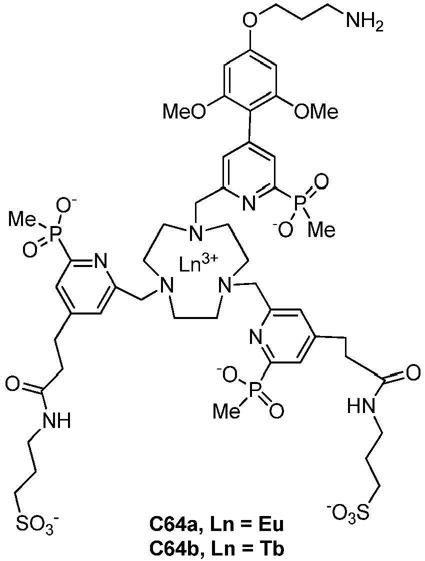

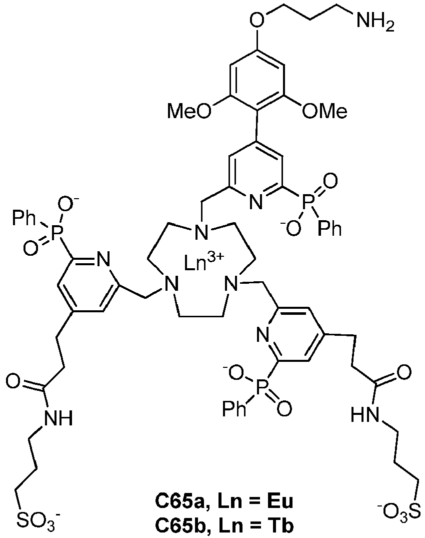

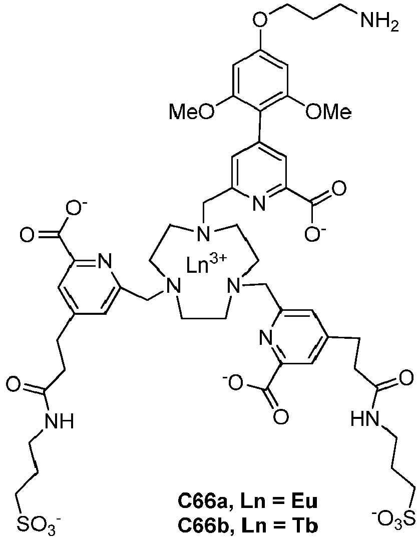

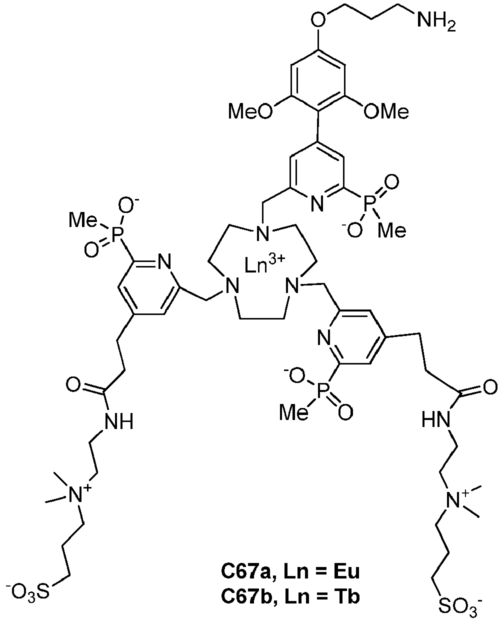

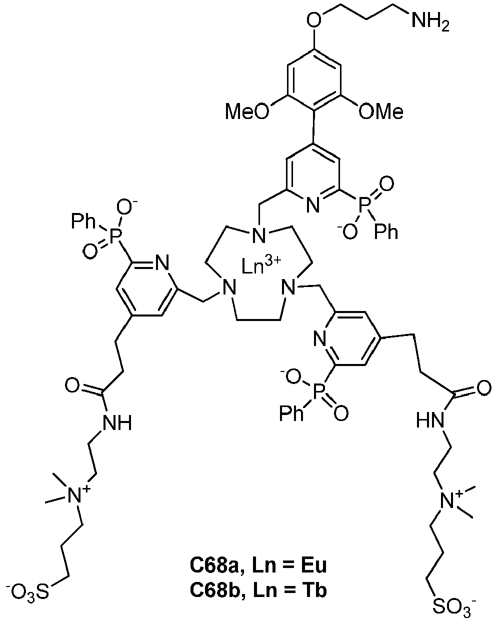

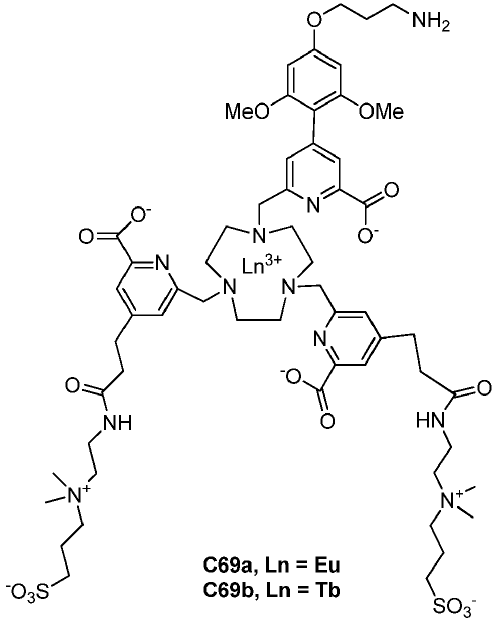

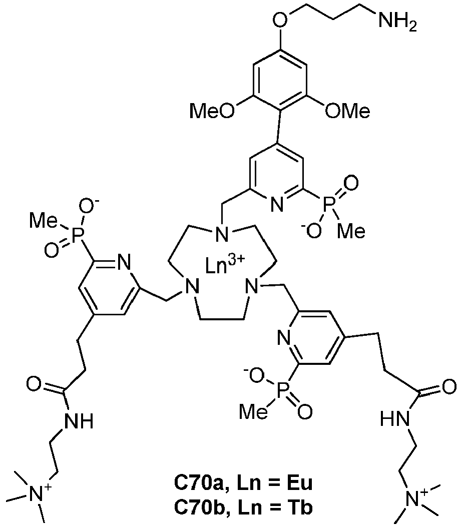

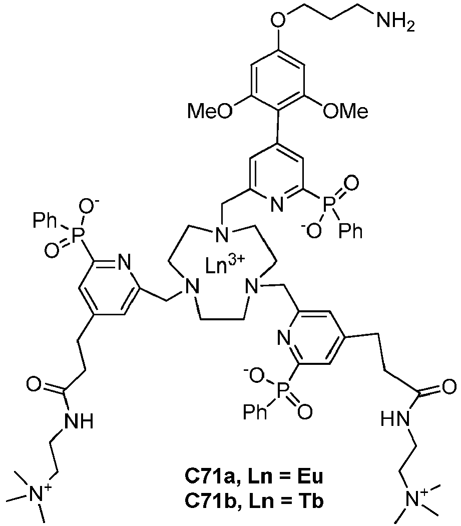

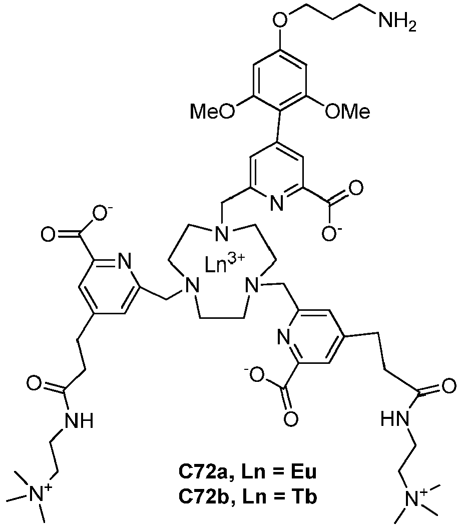

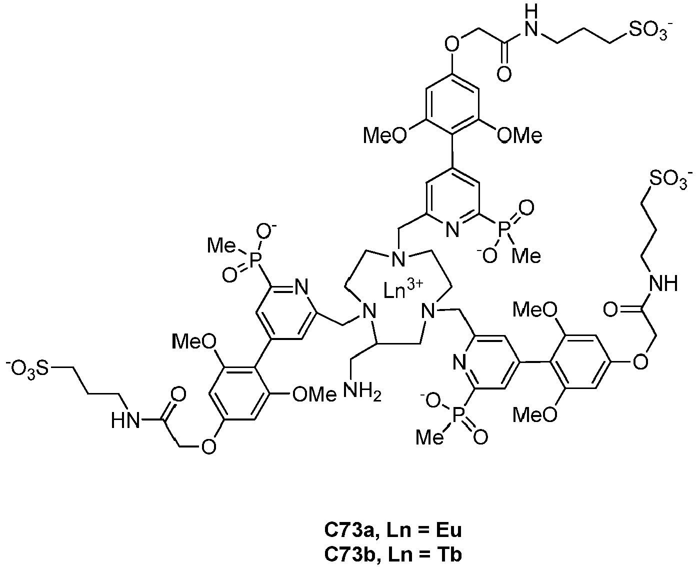

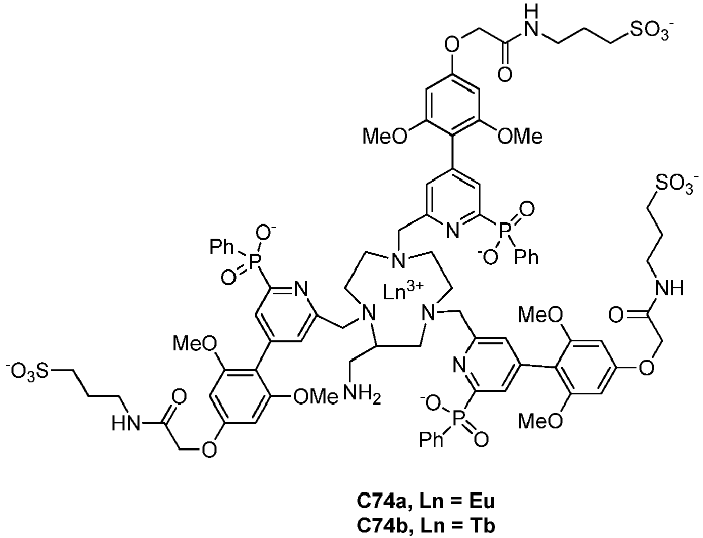

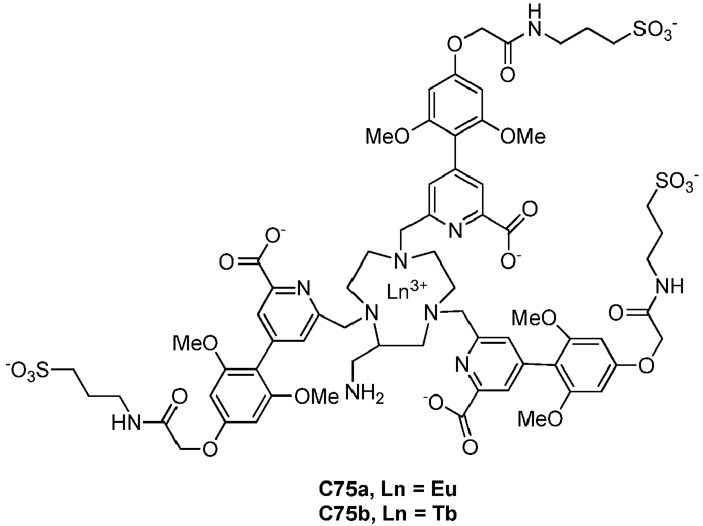

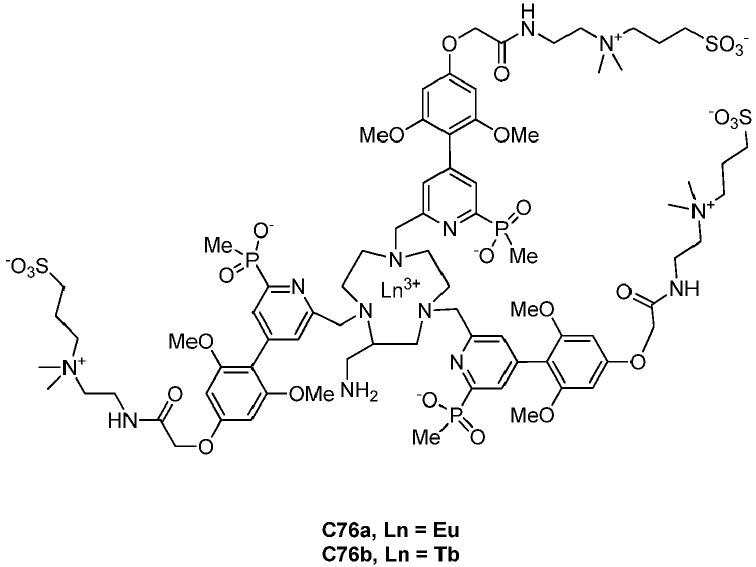

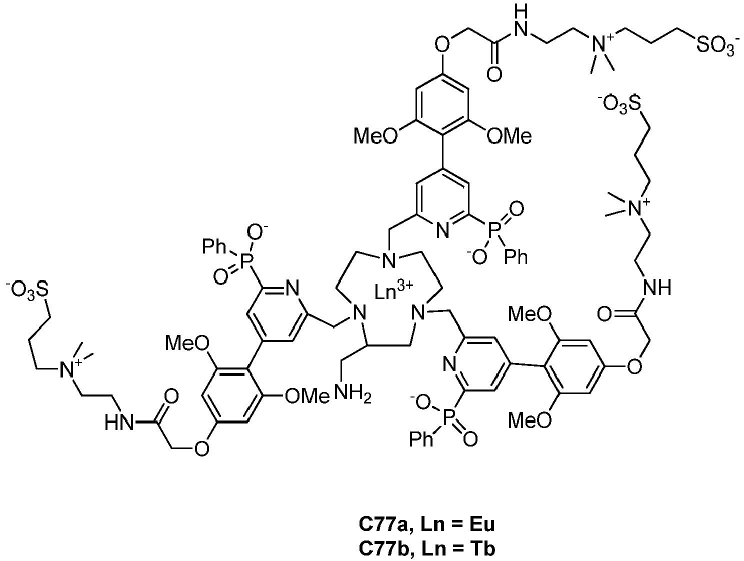

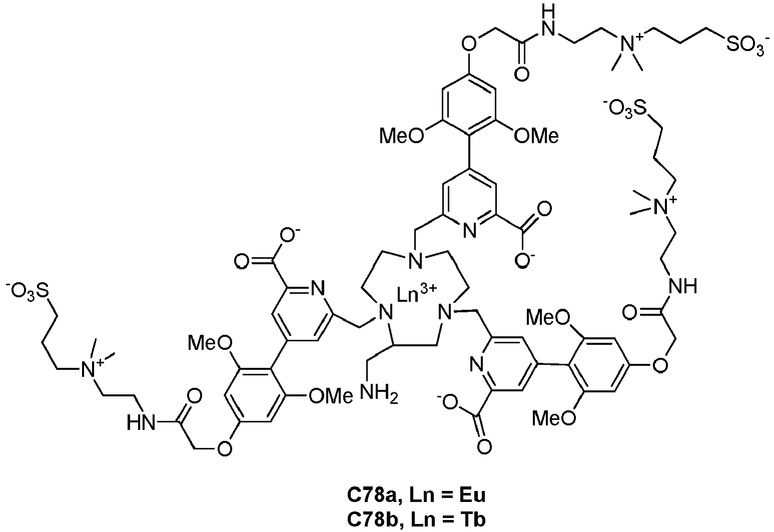

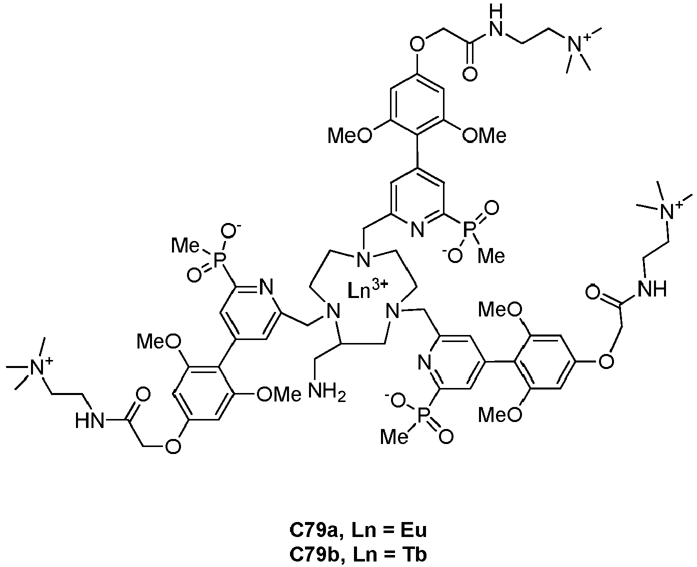

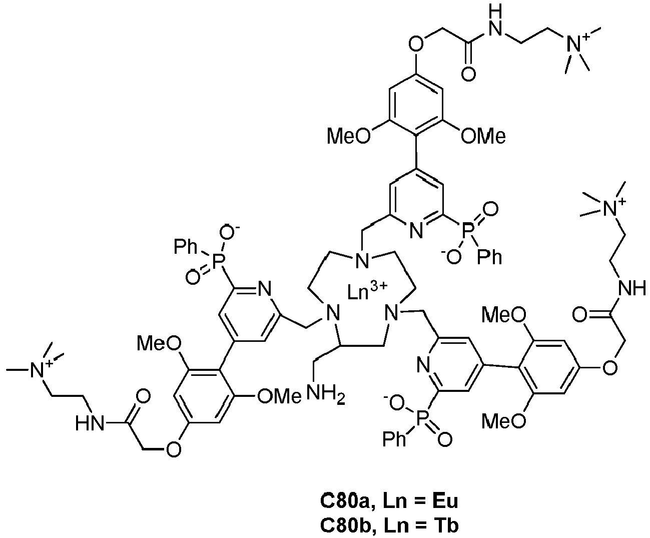

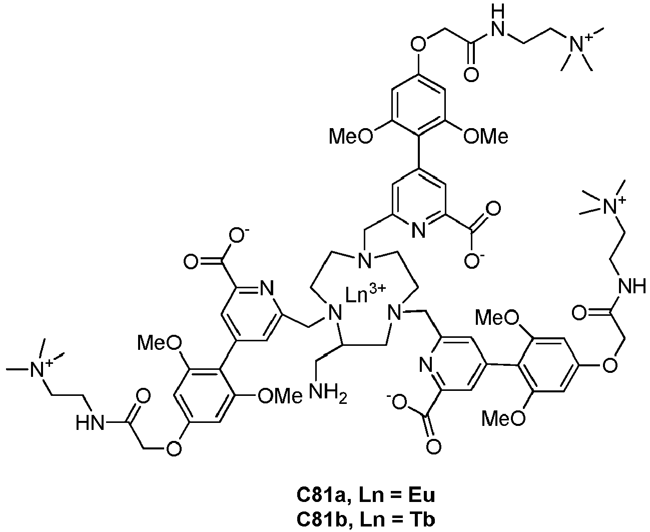

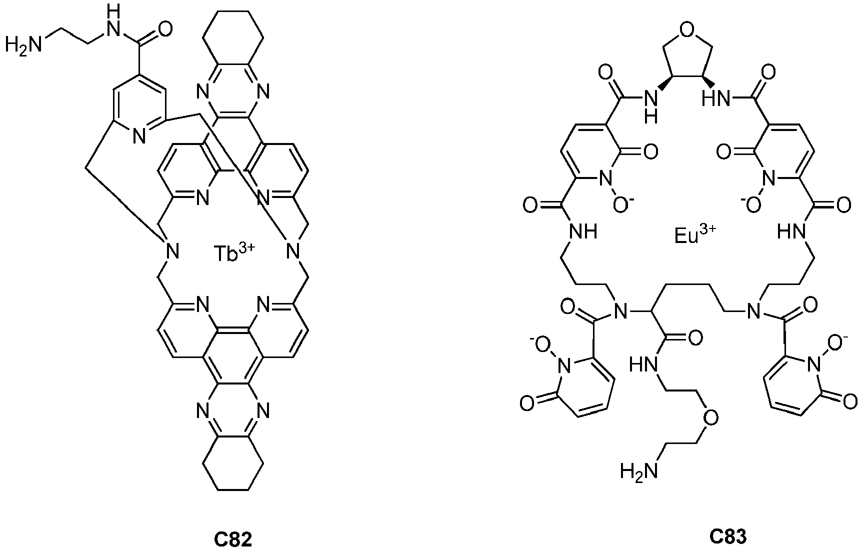

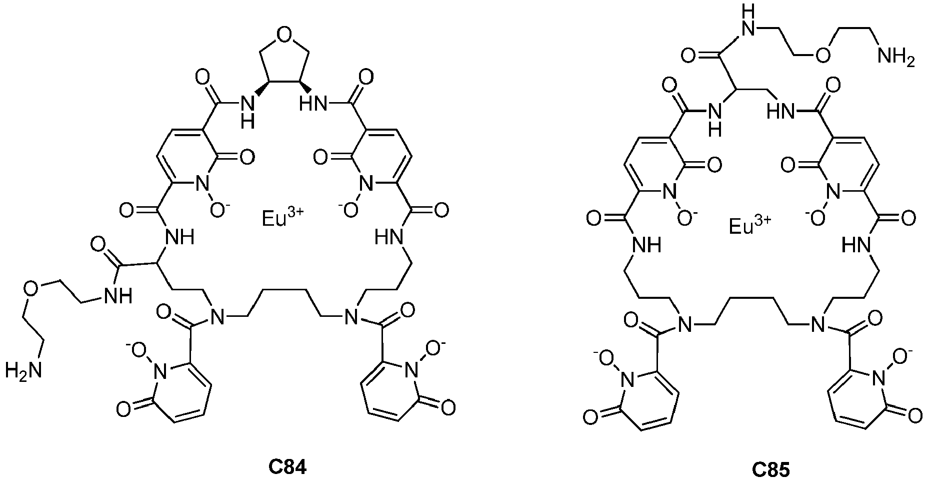

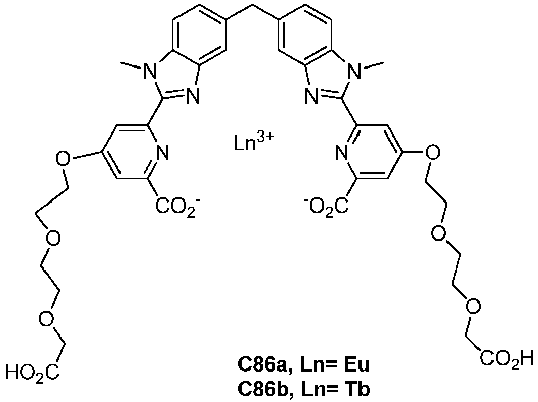

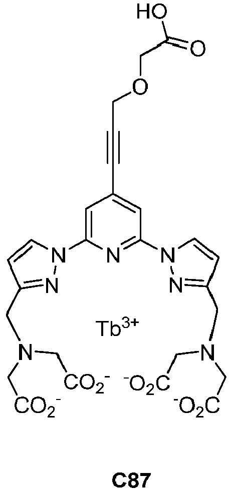

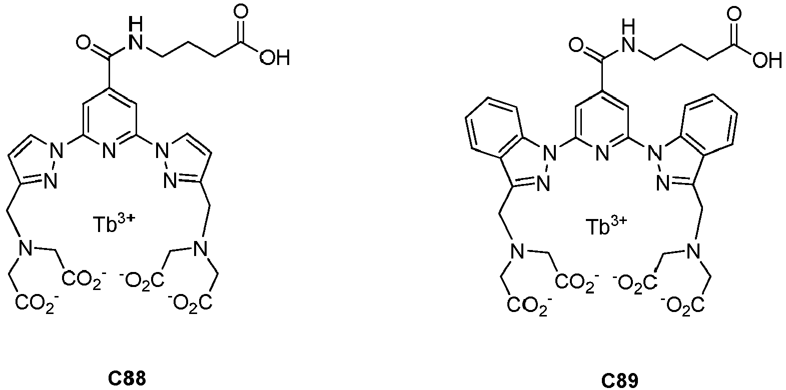

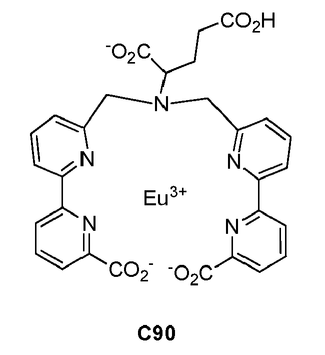

Depending on the pH, -SO₃H, -CO₂H et -PO(OH)₂ groups are in deprotonated form or not. Therefore these groups designate also the groups -SO₃⁻, -CO₂⁻ et -PO(OH)O⁻. The lanthanide complexes C1 to C90 are described in the following publications or patents. These complexes are either commercially available or can be obtained by synthetic routes, as described in the prior art, such as in WO 2020/157439 A1. Advantageously, the fluorescent donor compound is a FRET partner selected from: europium cryptate, europium chelate, terbium chelate, terbium cryptate, ruthenium chelate, quantum dot, allophycocyanins, rhodamines, cyanins, squarains, coumarins, proflavins, acridines, fluoresceins, boron-dipyrromethene derivatives and nitrobenzoxadiazole.

Particularly advantageously, the fluorescent donor compound is a FRET partner selected from: europium cryptate; europium chelate; terbium chelate; terbium cryptate; ruthenium chelate; and quantum dot; europium and terbium chelates and cryptates being particularly preferred.

### Fluorescent acceptor compounds

Fluorescent acceptor compounds can be selected from the following group: allophycocyanins, in particular those known under the trade name XL665; luminescent organic molecules, such as rhodamines, cyanins (such as Cy5), squarains, coumarins, proflavins, acridins, fluoresceins, boron-dipyrromethene derivatives (marketed as "Bodipy"), fluorophores known as "Atto", fluorophores known as "DY", compounds known as "Alexa", nitrobenzoxadiazole. Advantageously, fluorescent acceptor compounds are selected from allophycocyanins, rhodamines, cyanins, squarains, coumarins, proflavins, acridins, fluoresceins, boron-dipyrromethene derivatives, nitrobenzoxadiazole.

The terms "cyanins" and "rhodamines" should be understood as "cyanine derivatives" and "rhodamine derivatives" respectively. The skilled person is familiar with these different fluorophores, which are available on the market.

"Alexa" compounds are marketed by Invitrogen; "Atto" compounds are marketed by Attotec; "DY" compounds are marketed by Dyomics; "Cy" compounds are marketed by Amersham Biosciences; other compounds are marketed by various chemical reagent suppliers, such as Sigma, Aldrich or Acros.

The following fluorescent proteins can also be used as fluorescent acceptor compounds: cya fluorescent proteins (AmCyan1, Midori-Ishi Cyan, mTFP1), green fluorescent proteins (EGFP, AcGFP, TurboGFP, Emerald, Azami Green, ZsGreen), yellow fluorescent proteins (EYFP, Topaz, Venus, mCitrine, YPet, PhiYFP, ZsYellow1, mBanana), orange and red fluorescent proteins (Orange kusibari, mOrange, tdtomato, DsRed, DsRed2, DsRed-Express, DsRed-Monomer, mTangerine, AsRed2, mRFP1, JRed, mCherry, mStrawberry, HcRed1, mRaspberry, HcRed-Tandem, mPlim, AQ143), fluorescent proteins in far red (mKate, mKate2, tdKatushka2).

Advantageously, the fluorescent acceptor compound is a FRET partner selected from: allophycocyanins, rhodamines, cyanins, squarains, coumarins, proflavins, acridins, fluoresceins, boron-dipyrromethene derivatives, nitrobenzoxadiazole and a quantum dot, GFP, GFP variants selected from GFP10, GFP2 and eGFP, YFP, YFP variants selected from eYFP, YFP topaz, YFP citrine, YFP venus and YPet, mOrange, DsRed.

### • Labeling for the implementation of BRET

In a particular embodiment, the streptavidin and the C1q are each labeled with a member of a BRET partners pair, i.e. a luminescent donor compound or a fluorescent energy-accepting compound.

The direct labeling of the streptavidin and the C1q by a luminescent donor compound or a protein-type fluorescent acceptor compound, member of a BRET partners pair, can be carried out by the classical methods known to the skilled person and in particular described in the article by Tarik Issad and Ralf Jockers (Bioluminescence Resonance Energy Transfer to Monitor Protein-Protein Interactions, Transmembrane Signaling Protocolspp 195-209, Part of the Methods in Molecular Biology™ book series MIMB, volume 332) to which the skilled person may refer.

The direct labeling of the streptavidin and the C1q by an organic molecule-type fluorescent acceptor compound, member of a BRET partners pair, can be carried out by the classical methods known to the skilled person, based on the presence of reactive groups on the ligand as mentioned above. For example, the following reactive groups may be used: the terminal amino group, carboxylate groups of aspartic and glutamic acids, amino groups of lysines, guanidine groups of arginines, thiol groups of cysteines, phenol groups of tyrosines, indole rings of tryptophans, thioether groups of methionines, imidazole groups of histidines.

Reactive groups can form a covalent bond with a reactive group carried by a member of a BRET partner pair. The appropriate reactive groups, carried by the member of a BRET partner pair, are well known to the skilled person, for example an acceptor compound functionalized with a maleimide group will be able to bind covalently with thiol groups carried by cysteines carried by a protein or peptide, for example the C1q. Similarly, an acceptor compound carrying an N-hydroxysuccinimide ester will be capable of covalently binding to an amine containing a protein or peptide.

The selection of the BRET partner pair to obtain a BRET signal is within the reach of the skilled person. For example, donor-acceptor pairs that can be used to study BRET phenomena are described in particular in the article by Dasiel O. Borroto-Escuela (BIOLUMINESCENCE RESONANCE ENERGY TRANSFER (BRET) METHODS TO STUDY G PROTEIN-COUPLED RECEPTOR-RECEPTOR TYROSINE KINASE HETERORECEPTOR COMPLEXES, Cell Biol. 2013; 117: 141-164), which the skilled person may refer.

### Luminescent donor compounds

In a particular embodiment, the luminescent donor compound is a BRET partner selected from: Luciferase (luc), Renilla Luciferase (Rluc), variants of Renilla Luciferase (Rluc8) and Firefly Luciferase.

### Fluorescent acceptor compounds

In a particular embodiment, the fluorescent acceptor compound is a BRET partner selected from: allophycocyanins, rhodamines, cyanins, squarains, coumarins, proflavins, acridins, fluoresceins, boron-dipyrromethene derivatives, nitrobenzoxadiazole, a quantum dot, GFP, GFP variants (GFP10, GFP2, eGFP), YFP, YFP variants (eYFP, YFP topaz, YFP citrine, YFP venus, YPet), mOrange, DsRed.

As explained above, the streptavidin and/or the C1q may also be labeled indirectly with a member of a pair of RET partners, for example by introducing into the measurement medium an antibody or antibody fragment, which is itself covalently bound to an acceptor/donor compound, this antibody or antibody fragment specifically recognizing the streptavidin or the C1q.

### Method LOCI

If the HPA is a LOCI, the invention therefore relates to an *in vitro* method for determining the binding of an antibody (tested antibody) to a complement component 1q (C1q), comprising the following steps:
a) Contacting into a measurement medium:
   - a tested antibody,
   - a biotinylated anti-Fab ligand, said biotinylated anti-Fab ligand being able to bind the Fab region of the tested antibody,
   - a streptavidin directly or indirectly labelled with the first member of a pair of LOCI (Luminescent oxygen channeling immunoassay) partners,
   - a C1q directly or indirectly labelled with the second member of a pair of LOCI partners;
b) Measuring the LOCI signal in the measurement medium, the existence of LOCI signal being representative of the binding of the tested antibody to the C1q.

There are a number of LOCI technologies used to study biomolecular interactions in a microplate format, such as Amplified Luminescence Proximity Homogeneous Assay (ALPHA). For example, ALPHA kits are commercially available under the trademark AlphaScreen^{®} and AlphaLISA^{®}, manufactured by PerkinElmer of Waltham, Mass. These technologies are non-radioactive, homogeneous proximity assays. Binding of molecules captured on the beads leads to singlet oxygen diffusion from one bead to the other, ultimately producing a detectable luminescent/fluorescent signal, which provides qualitative and quantitative information about one or more analytes in a sample.

The pair of LOCI partners comprises two bead types: donor beads and acceptor beads. Donor beads comprise a photosensitizer, for example, phthalocyanine, which converts ambient oxygen to an excited and reactive form of oxygen, singlet oxygen, upon illumination at 680 nm. Singlet oxygen is not a radical; it is molecular oxygen with a single excited electron. Like other excited molecules, singlet oxygen has a limited lifetime prior to falling back to ground state. Within its 4 µsec half-life, singlet oxygen can diffuse approximately 200 nm in solution, as compared to TR-FRET which has a maximum transfer distance of about 10 nm. If an acceptor bead is within that proximity, energy is transferred from the singlet oxygen to thioxene derivatives within the acceptor bead, subsequently culminating in light production within a range of wavelengths, e.g., 520-620 nm (AlphaScreen^{®}) or at a particular wavelength, e.g., 615 nm (AlphaLISA^{®}). In the absence of an acceptor bead, singlet oxygen falls to ground state and no signal is produced. This proximity-dependent chemical energy transfer is the basis for LOCI's homogeneous nature, such that no washing steps are required, unlike ELISA assays, electrochemiluminescence, and flow cytometry assays, thereby offering a significant advantage.

Acceptor beads are embedded with three dyes: thioxene, anthracene, and rubrene. Rubrene, the final fluor, emits light detectable between 520-620 nm (e.g. AlphaScreen^{®}). Anthracene, and rubrene may be substituted with an Europium chelate (e.g. AlphaLISA^{®}). The Europium (Eu) chelate is directly excited by the 340 nm light resulting from the conversion of thioxene to a di-ketone derivative following its reaction with singlet oxygen. The excited Europium chelate generates an intense light detectable within a much narrower wavelength bandwidth centered around 615 nm. In contrast to the acceptor beads not substituted with an Europium chelate, the acceptor beads substituted with an Europium chelate emission is therefore less susceptible to interference by either artificial or natural compounds (such as hemoglobin) that absorb light between 500-600 nm.

### • Labeling of the streptavidin and the C1q with a member of a pair of LOCI partners

Direct labeling of the streptavidin and the C1q with a member of a pair of LOCI partners can be carried out by conventional methods known to the skilled person, based on the presence of reactive groups on the streptavidin and the C1q. For example, the following reactive groups may be used: the N-terminal amino group, carboxylate groups of aspartic and glutamic acids, amino groups of lysines, thiol groups of cysteines.

Reactive groups can form a covalent bond with a reactive group carried by a member of a pair of LOCI partners. The appropriate reactive groups, carried by the member of a pair of LOCI partners, are well known to the skilled person, e.g. a donor compound or an acceptor compound functionalized with a maleimide group will for example be capable of covalently binding with thiol groups carried by cysteines carried by a protein or peptide, e.g. the streptavidin or the C1q. Similarly, a donor/acceptor compound carrying an N-hydroxysuccinimide ester will be able to covalently bind to an amine containing a protein or peptide.

ALPHA beads (donor or acceptor) can be directly conjugated to a peptide, a protein (e.g. Streptavidin or C1q), or an antibody (e.g. an anti-Clq antibody). The conjugation is based on a reductive amination reaction between the reactive aldehyde groups present at the surface of ALPHA beads and the free amine groups (of lysines and N-terminus) of the molecule of interest. The reaction is performed in presence of sodium cyanoborohydride (NaBH₃CN) which stabilizes the bond formed, and is then stopped by the addition of carboxymethylamine (CMO) that blocks the remaining free aldehyde groups on ALPHA beads.

As explained above, the streptavidin and/or the C1q may also be labeled indirectly with a member of a pair of LOCI partners, for example by introducing into the measurement medium an antibody or antibody fragment, which is itself covalently bound to an acceptor/donor compound, this antibody or antibody fragment specifically recognizing the streptavidin or the C1q.

### Kit of reagents for carrying out the method of the invention

According to a second aspect, the invention relates to a kit of reagents for carrying out the method of the invention, comprising:
(i) a biotinylated anti-Fab ligand,
(ii) a streptavidin or a streptavidin directly labelled with the first member of a pair of HPA partners, and
(iii) a C1q or a C1q directly labelled with the second member of a pair of HPA partners, and
(iv) if the streptavidin is not directly labelled with the first member of a pair of HPA partners, an anti-streptavidin ligand directly labelled with the first member of a pair of HPA partners, and
(v) if the C1q is not directly labelled with the second member of a pair of HPA partners, an anti-Clq ligand directly labelled with the second member of a pair of HPA partners.

The specific embodiments of the different components of the kit of the invention, as detailed above in the description, also apply to the kit of the invention.

In a particularly preferred embodiment, the kit of reagents for carrying out the method of the invention, comprise:
(i) a biotinylated anti-Fab antibody, and
(ii) a streptavidin directly labelled with the first member of a pair of HPA partners, preferably the first member of a pair of TR-FRET partners, such as d2, and
(iii) a C1q, and
(iv) an anti-Clq antibody directly labelled with the second member of a pair of HPA partners, preferably the second member of a pair of TR-FRET partners, such as Europium cryptate.

In a preferred embodiment, the kit also comprises a buffer, the antigen of the tested antibody (assuming that the antigen should not be biotinylated) and/or any compound that is suitable for implementing the method of the invention, such as albumin, a surfactant and/or a preservative, as disclosed above in the description.

The reagents of the kit are contained in one or several container(s), preferably several containers.

### EXAMPLES

### Example 1: method for determining the binding of a tested antibody to C1q (TR-FRET) using a biotinylated anti-human Fab antibody and a C1q indirectly labelled with the donor

To determine the binding of a tested antibody to C1q, a TR-FRET sandwich assay was carried out as illustrated in Figure 1.

The TR-FRET detection was based on HTRF^{®} technology (PerkinElmer/Cisbio Bioassays). HTRF^{®} technology corresponds to a fluorescence resonance energy transfer between a fluorescent donor dye (donor), an Eu³⁺ cryptate or a Tb³⁺ cryptate, and a fluorescent acceptor dye (acceptor), the d2. Each dye is covalently labelled to a molecule (antibody or protein).

The method described here was based on the use of a complex between d2-labelled streptavidin (PerkinElmer/Cisbio Bioassays, #610SADLF) and a biotinylated mouse monoclonal anti-human IgG Fab antibody (ThermoFisher Scientific #SA1-19255) that was used to capture and aggregate the tested antibody in solution (Figure 1). The human C1q protein (Sigma-Aldrich #C1740) bound to the tested antibody was detected with a mouse monoclonal anti-Clq antibody (HycultBiotech #HM2382) conjugated to Eu³⁺ cryptate.

### Biotinylation of the anti-human IgG Fab antibody

A 5 mM stock solution of biotin N-hydroxysuccinimide ester (Sigma-Aldrich #B2643) was prepared in anhydrous DMSO. The antibody was placed at a concentration of 1 mg/mL in a carbonate buffer 0.1M pH9 and biotin was added using a molar ratio of 6 biotin/antibody. After a 30-minute incubation at room temperature (RT), the antibody was purified to remove the excess of unconjugated biotin in a phosphate buffer 0.1M pH7 using a pre-packed gel filtration gravity-flow column (Amersham NAP-10 column, Cytiva, #17085401) according to manufacturer's instructions. The stock solution of biotinylated antibody was supplemented with 0.1% BSA and stored at -20°C until use.

### Conjugation of the anti-C1q antibody to Eu³⁺ cryptate

A 5 mM stock solution of Eu³⁺ cryptate N-hydroxysuccinimide ester (Cisbio Bioassays) was prepared in anhydrous DMSO. The antibody was placed at a concentration of 1 mg/mL in a phosphate buffer 50 mM pH8 and Eu³⁺ cryptate was added using a molar ratio of 15 Eu³⁺ cryptate/antibody. After a 30-minute incubation at room temperature (RT), the antibody was purified to remove the excess of unconjugated Eu³⁺ cryptate in a phosphate buffer 0.1M pH7 using a pre-packed gel filtration gravity-flow column (Amersham NAP-5 column, Cytiva, #17085301) according to manufacturer's instructions. The stock solution of Eu³⁺ cryptate-antibody was supplemented with 0.1% BSA and stored at -20°C until use.

### Description of tested antibodies

The characteristics of the antibodies tested in the assay are detailed in Table 1. They are all purified antibodies of known concentrations.

**[Table 1]**

| **Denomination** | **Description** | **Supplier, #Catalog number** |
|---|---|---|
| IgG1 isotype control | Human IgG1 Isotype Control Recombinant Antibody | BioLegend, #403502 |
| IgG2 isotype control | Human IgG2 Isotype Control Recombinant Antibody | BioLegend, #403602 |
| IgG4 isotype control | Human IgG4 Isotype Control Recombinant Antibody | BioLegend, #403702 |
| Rituximab | Therapeutic anti-CD20 antibody (Type I), Chimeric IgG1 | - |
| Rituximab IgG2 isotype | Antibody consisting of the variable region of Rituximab and the constant region of the human IgG2 isotype | InvivoGen, #hcd20-mab2 |
| Rituximab IgG4 isotype | Antibody consisting of the variable region of Rituximab and the constant region of the human IgG4 isotype | InvivoGen, #hcd20-mab4 |
| Non-fucosylated Rituximab | Antibody consisting of the variable region of Rituximab and a non-fucosylated constant region of the human IgG1 isotype | InvivoGen, #hcd20-mab13 |
| Non-glycosylated Rituximab | Antibody consisting of the variable region of Rituximab and a mutated non-glycosylated constant region of the human IgG1 isotype | InvivoGen, #hcd20-mab12 |
| Obinutuzumab (also known as GA101) | Therapeutic anti-CD20 antibody (Type II), Humanized IgG1 | |
| Cetuximab | Therapeutic anti-EGFR antibody, Chimeric IgG1 | - |
| Cetuximab IgG2 isotype | Antibody consisting of the variable region of Cetuximab and the constant region of the human IgG2 isotype | InvivoGen, #hegfr-mab2 |
| Non-glycosylated Cetuximab | Antibody consisting of the variable region of Cetuximab and a mutated non-glycosylated constant region of the human IgG1 isotype | InvivoGen, #hegfr-mab12 |
| Ipilimumab | Therapeutic anti-CTLA-4 antibody, | - |

| Ipilimumab IgG2 isotype | Fully human IgG1 Antibody consisting of the variable region of Ipilimumab and the constant region of the human IgG2 isotype | InvivoGen, #hctla4-mab2 |
|---|---|---|
| Atezolizumab | Therapeutic anti-PD-L1 antibody, Humanized IgG1 | - |
| Spartalizumab | Therapeutic anti-PD-1 antibody, Humanized IgG4 | - |
| Adalimumab | Therapeutic anti-TNF-α antibody, Fully human IgG1 | - |

### Preparation of TR-FRET assay components

Just before use, all assay components were diluted either with buffer 1 (Na₂HPO₄ 3 mM, KH₂PO₄ 1 mM, NaCl 135 mM, BSA 0.1%, Tween-20 0.05%, ProClin-300 0.01%, pH 7.4) (Figures 2, 3, 4A and 5), or with buffer 2 (Na₂HPO₄ 42.13 mM, KH₂PO₄ 7.86 mM, BSA 0.1%, Tween-20 0.05%, pH 7.4) (Figure 6), depending on the experiments.

Serial dilutions of tested antibodies were prepared 4X to target final concentrations in the assay comprised between 1 and 200 nM. Adalimumab was also tested in presence of its antigen human TNF-α (R&D Systems, #10291-TA). In that case, the serial dilutions were prepared in the buffer supplemented with 150 nM TNF-α.

The premix [biotinylated anti-human IgG Fab antibody/streptavidin-d2] was prepared 4X using a ratio 1/1 to obtain final concentrations of 50 nM/50 nM. The human C1q protein was prepared 4X to reach a final concentration of 5 nM. The Eu³⁺cryptate-anti-C1q antibody was prepared 4X to target a final concentration of 0.6 nM (Figure 6) or 2.4 nM (Figures 2, 3, 4A and 5), depending on the experiments.

### TR-FRET assay protocol

The 4X solutions of assay components were sequentially dispensed in a 384-well low volume white microplate (Proxiplate Plus, PerkinElmer #6008280) as followed:
- 5 µL of serial dilutions of the tested antibody (or 5 µL of buffer for the negative control)
- 5 µL of the premix [biotinylated anti-human IgG Fab antibody/streptavidin-d2]
- 5 µL of human C1q protein
- 5 µL of Eu³⁺cryptate-anti-C1q antibody

The plate was covered with a sealer and incubated at RT for 3h (Figures 2, 3, 4A and 5) or overnight (Figure 6), depending on the experiments. The HTRF signal was recorded on the PHERAstar FS microplate reader (BMG Labtech) using an HTRF detection module (excitation with a flash lamp at 337 nm, signal emissions recorded at 665 nm & 620 nm).

### TR-FRET signal analysis and data handling

The "HTRF Ratio" was calculated using the following formula: Signal₆₆₅ₙₘ/Signal₆₂₀ₙₘ x 10,000. The specific signal "Delta Ratio" was then calculated: Delta Ratio = HTRF Ratio_{antibody} - HTRF Ratio_{negative} control. The binding curves were represented by plotting the log [antibody] (M) versus the Delta Ratio, and fitted on GraphPad Prism 9 using the model "Sigmoidal dose response curve - Variable slope (four parameters)", as illustrated in Figures 2, 3, 4A, 5 and 6. For each binding curve, the signal intensity was determined by calculating the Signal to Background (S/B): S/B = HTRF Ratio max_{antibody}/HTRF Ratio_{negative} control. The EC₅₀ value was also determined. The results obtained with each tested antibody are presented in Table 2.

**[Table 2]**

| **Tested antibody** | **S/B** | **EC₅₀ (nM)** |
|---|---|---|
| IgG1 isotype control | 6.0 | 17.3 |
| IgG2 isotype control | 1.7 | 34.0 |
| IgG4 isotype control | no binding | no binding |
| Rituximab | 8.2 | 15.5 |
| Rituximab IgG2 isotype | 1.7 | 38.7 |
| Rituximab IgG4 isotype | no binding | no binding |
| Non-fucosylated Rituximab | 8.3 | 16.2 |
| Non-glycosylated Rituximab | 4.0 | 25.0 |
| Obinutuzumab (GA101) | 3.5 | 18.6 |
| Cetuximab | 4.2 | 16.9 |
| Cetuximab IgG2 isotype | 1.5 | 48.0 |
| Non-glycosylated Cetuximab | 1.4 | 10.2 |
| Ipilimumab | 9.8 | 13.2 |
| Ipilimumab IgG2 isotype | 3.3 | 53.3 |
| Atezolizumab | 12.3 | 13.2 |
| Spartalizumab | no binding | no binding |
| Adalimumab | 6.6 | 7.2 |
| Adalimumab (+ 150 nM TNF-α) | 6.9 | 4.6 |

The TR-FRET assay was able to discriminate the C1q binding capacity of IgG antibodies of the different isotypes 1, 2 and 4 (Figure 2).
As presented in Figure 2A, the best S/B and EC₅₀ were obtained with the human IgG1 isotype control, indicating that this isotype binds the most efficiently to human C1q. The lower S/B and increased EC₅₀ obtained with the human IgG2 isotype control shows that human IgG2 interacts weakly with human C1q. No significant signal was measured with the human IgG4 isotype control, indicating that this isotype does not bind to human C1q. These results are consistent with literature [Ref. 6]. Similar results were obtained when comparing the therapeutic IgG1 antibody Rituximab with its IgG2 and IgG4 isotype variants (Figure 2B), as well as when comparing the therapeutic IgG1 antibodies Cetuximab and Ipilimumab with their corresponding IgG2 isotype variants (Figure 2C and 2D).

The TR-FRET assay was able to differentiate the capacity of interaction of glycosylated versus non-glycosylated antibodies to human C1q (Figure 3).
The therapeutic IgG1 antibody Rituximab was tested in parallel with a non-fucosylated variant (used here as an irrelevant control) and a non-glycosylated variant (Figure 3A). The absence of the fucose residue on the Fc domain is known to modulate ADCC activity only, and to not modulate C1q binding. As expected, no detectable change in C1q binding was obtained in the assay with the non-fucosylated antibody compared to Rituximab.
Conversely, the capacity of interaction of the non-glycosylated variant was altered (decreased S/B and increased EC₅₀), which is consistent with the fact that antibody Fc glycosylation is important for C1q binding. In a similar way, the assay showed an important decrease in the capacity of the non-glycosylated version of Cetuximab to bind to human C1q compared to the therapeutic IgG1 antibody Cetuximab (Figure 3B).

The TR-FRET assay allowed to discriminate the C1q binding capacity of therapeutic type I and type II anti-CD20 antibodies (Figure 4).
The reduced signal intensity obtained with Obinutuzumab (Type II anti-CD20) compared to Rituximab (Type I anti-CD20) indicates that Obinutuzumab is less efficient to bind to human C1q (Figure 4A). These results are in accordance with data from the literature that were obtained using an ELISA assay (Figure 4B adapted from [Ref. 5]).

The therapeutic antibodies Atezolizumab and Spartalizumab were also tested in the TR-FRET assay (Figure 5).
As expected, the anti-PD-L1 IgG1 Atezolizumab interacted efficiently with human C1q, while the anti-PD-1 IgG4 Spartalizumab showed no capacity to bind to the protein.

The TR-FRET assay was capable to detect the interaction between human C1q and the therapeutic anti-TNF-α IgG1 antibody Adalimumab pre-complexed or not with its antigen human TNF-α (Figure 6).
Besides, the assay was able to discriminate the C1q binding capacity of the antibody in absence or in presence of its antigen, based on the improvement of the EC₅₀ value in presence of the antigen. These results are consistent with literature [Ref. 7].

### Example 2: method for determining the binding of an antibody to a C1q (TR-FRET) using a biotinylated anti-human Fab antibody and a C1q directly labelled with the donor

An alternative method consists to carry out a TR-FRET assay with the human C1q protein directly conjugated to the donor, as presented in Figure 7.

The TR-FRET detection is based on HTRF^{®} technology (PerkinElmer/Cisbio Bioassays), as described in Example 1.

The method described here is based on the use of a complex between d2-labelled streptavidin (PerkinElmer/Cisbio Bioassays, #610SADLF) and a biotinylated mouse monoclonal anti-human IgG Fab antibody (ThermoFisher Scientific #SA1-19255) that was used to capture and aggregate the tested antibody in solution (Figure 7). The human C1q protein (Sigma-Aldrich #C1740) bound to the tested antibody was directly conjugated to Tb³⁺ cryptate.

The anti-human IgG Fab antibody was biotinylated as described in Example 1. The human C1q protein was conjugated to Lumi4^{®}-Terbium cryptate using the Terbium Cryptate labeling kit (PerkinElmer/Cisbio Bioassays, #62TBSPEA) following manufacturer's instructions.

### Description of tested antibodies

The human isotype controls IgG1, IgG2 and IgG4, as well as the therapeutic IgG1 antibody Rituximab were tested in the assay. Their characteristics are detailed in Table 1. They are all purified antibodies of known concentrations.

### Preparation of TR-FRET assay components

Just before use, all assay components were diluted with buffer 2 whose formulation is detailed in Example 1. Serial dilutions of tested antibodies were prepared 4X to target final concentrations in the assay comprised between 1 and 200 nM. The premix [biotinylated anti-human IgG Fab antibody/streptavidin-d2] was prepared 4X using a ratio 1/1 to obtain final concentrations of 50 nM/50 nM. The Tb³⁺ cryptate-human C1q protein was prepared 2X to reach a final concentration of 5 nM.

### TR-FRET assay protocol

The working solutions of assay components were sequentially dispensed in a 384-well low volume white microplate (Proxiplate Plus, PerkinElmer #6008280) as followed:
- 5 µL of serial dilutions of the tested antibody (or 5 µL of buffer for the negative control)
- 5 µL of the premix [biotinylated anti-human IgG Fab antibody/streptavidin-d2]
- 10 µL of Tb³⁺cryptate-human C1q protein

The plate was covered with a sealer and incubated overnight at RT. The HTRF signal was recorded on the PHERAstar FS microplate reader (BMG Labtech) using an HTRF detection module (excitation with a flash lamp at 337 nm, signal emissions recorded at 665 nm & 620 nm).

### TR-FRET signal analysis and data handling

HTRF data were analysed and represented as described in Example 1. The binding curves obtained for each tested antibody are presented in Figure 8. For each binding curve, the S/B was calculated as detailed in Example 1, and the EC₅₀ value was also determined. The results obtained with each tested antibody are presented in Table 3.

**[Table 3]**

| **Tested antibody** | **S/B** | **EC₅₀ (nM)** |
|---|---|---|
| Rituximab | 7.6 | 14.4 |
| IgG1 isotype control | 7.9 | 13.7 |
| IgG2 isotype control | 2.9 | 73.6 |
| IgG4 isotype control | no binding | no binding |

In the same way as the method presented in Example 1, the TR-FRET assay was able to discriminate the C1q binding capacity of human IgG antibodies of the different isotypes 1, 2 and 4. The best S/B and EC₅₀ were obtained with the human IgG1 isotype control, indicating that this isotype binds the most efficiently to human C1q. The lower S/B and increased EC₅₀ obtained with the human IgG2 isotype control shows that human IgG2 interacts weakly with human C1q. No significant signal was measured with the human IgG4 isotype control, indicating that this isotype does not bind to human C1q. These results are consistent with the literature [Ref. 6].

The binding profile of the therapeutic IgG1 chimeric anti-CD20 antibody Rituximab was close to the one obtained with the human IgG1 isotype control, with similar S/B and EC₅₀ values.

### Example 3: method for determining the binding of a tested antibody to C1q (ALPHA) using a biotinylated anti-human Fab antibody

To determine the binding of a tested antibody to C1q, an ALPHA sandwich assay was carried out as illustrated in Figure 9.

The ALPHA detection was based on AlphaLISA^{®} technology (PerkinElmer). AlphaLISA^{®} technology corresponds to the diffusion of singlet oxygen between an Alpha donor bead (donor) and an AlphaLISA acceptor bead (acceptor). Each bead is covalently conjugated to a molecule (antibody or protein).

The method described here is based on the use of a complex between Alpha streptavidin-coated donor beads (PerkinElmer, #6760002) and a biotinylated mouse monoclonal anti-human IgG Fab antibody (ThermoFisher Scientific #SA1-19255) that was used to capture and aggregate the tested antibody in solution (Figure 9). The human C1q protein (Sigma-Aldrich #C1740) bound to the tested antibody was detected with a mouse monoclonal anti-Clq antibody (HycultBiotech #HM2382) conjugated to AlphaLISA acceptor beads (PerkinElmer #6772002).

The anti-human IgG Fab antibody was biotinylated as described in Example 1.

### Conjugation of the anti-C1q antibody to AlphaLISA acceptor beads

The conjugation was performed in PBS buffer pH 7.4 (Thermo Fisher Scientific #10010023) using a ratio of 5 mg AlphaLISA acceptor beads/100 µg anti-Clq antibody, in presence of 20 mM sodium cyanoborohydride (NaBH3CN) (SIGMA #156159) and 0.06% Tween-20 (Thermo Fisher Scientific #85113). After an overnight incubation at 37°C, the reaction was stopped by the addition of 3.1 mg/mL of carboxy-methoxylamine (CMO) (Sigma #C13408). The beads were purified to remove the excess of unconjugated antibody by several washing/centrifugation steps in PBS buffer pH 7.4.

### Description of tested antibodies

The human isotype controls IgG1, IgG2 and IgG4, as well as the therapeutic IgG1 antibody Rituximab were tested in the assay. Their characteristics are detailed in Table 1. They are all purified antibodies of known concentrations.

### Preparation of ALPHA assay components

Alpha streptavidin-coated donor beads are light-sensitive. All steps using this reagent (preparation, dispensing, plate reading) were performed under subdued laboratory lighting. Just before use, all assay components were diluted with buffer 1 whose formulation is detailed in Example 1.

Serial dilutions of tested antibodies were prepared 8X to target final concentrations in the assay comprised between 1 and 200 nM. The premix [biotinylated anti-human IgG Fab antibody/Alpha streptavidin-coated donor beads] was prepared 2.67X to obtain final concentrations of 50 nM biotin-antibody/67 µg/mL beads. The human C1q protein was prepared 4X to reach a final concentration of 5 nM. The AlphaLISA acceptor beads conjugated to the anti-Clq antibody were prepared 4X to target a final concentration of 20 µg/mL.

### ALPHA assay protocol

The working solutions of assay components were sequentially dispensed in a 384-well light gray microplate (AlphaPlate-384, PerkinElmer #6005350) as followed:
- 5 µL of serial dilutions of the tested antibody (or 5 µL of buffer for the negative control)
- 15 µL of the premix [biotinylated anti-human IgG Fab antibody/Alpha streptavidin-coated donor beads]
- 10 µL of human C1q protein. The plate was covered with a sealer and preincubated in the dark for 30 minutes at 23°C.
- 10 µL of AlphaLISA acceptor beads conjugated to the anti-Clq antibody. The plate was covered with a sealer and incubated in the dark for another 30 minutes at 23°C.

The ALPHA signal was recorded on the VICTOR^{®} Nivo^{™} reader (PerkinElmer) using standard Alpha settings (excitation at 680 nm, reading emission at 520-620 nm).

### ALPHA signal analysis and data handling

The binding curves were represented by plotting the log [antibody] (M) versus the ALPHA signal and fitted on GraphPad Prism 9 using the model "Sigmoidal dose response curve - Variable slope (four parameters)", as illustrated in Figure 10. For each binding curve, the signal intensity was determined by calculating the S/B: S/B = ALPHA signal max_{antibody}/ALPHA signal_{negative control}. The EC₅₀ value was also determined. The results obtained with each tested antibody are presented in Table 4.

**[Table 4]**

| **Tested antibody** | **S/B** | **EC₅₀ (nM)** |
|---|---|---|
| Rituximab | 659 | 26.8 |
| IgG1 isotype control | 752 | 23.7 |
| IgG2 isotype control | 43 | 115.4 |
| IgG4 isotype control | no binding | no binding |

In the same way as the method based on TR-FRET presented in Example 1, the ALPHA assay was able to discriminate the C1q binding capacity of human IgG antibodies of the different isotypes 1, 2 and 4. The best S/B and EC₅₀ were obtained with the human IgG1 isotype control, indicating that this isotype binds the most efficiently to human C1q. The lower S/B and increased EC₅₀ obtained with the human IgG2 isotype control shows that human IgG2 interacts weakly with human C1q. No significant signal was measured with the human IgG4 isotype control, indicating that this isotype does not bind to human C1q. These results are consistent with literature [Ref. 6].

The binding profile of the therapeutic IgG1 chimeric anti-CD20 antibody Rituximab was close to the one obtained with the human IgG1 isotype control, with similar S/B and EC₅₀ values.

### Example 4: method for determining the binding of an antibody to a C1q (TR-FRET) using a biotinylated antigen

Another method consists to carry out a TR-FRET assay with a biotinylated antigen instead of a biotinylated anti-human Fab antibody, as illustrated in Figure 11.

The TR-FRET detection is based on HTRF^{®} technology (PerkinElmer/Cisbio Bioassays), as described in Example 1.

The method described here is based on the use of a complex between d2-labelled streptavidin (PerkinElmer/Cisbio Bioassays, #610SADLF) and a biotinylated antigen (biotinylated recombinant human TNF-α protein, Abcam #ab167747) that was used to capture and aggregate the tested antibody in solution (Figure 11). The human C1q protein (Sigma-Aldrich #C1740) bound to the tested antibody was detected with a mouse monoclonal anti-Clq antibody (HycultBiotech #HM2382) conjugated to Eu³⁺ cryptate as described in Example 1. The antibody tested in the assay is the therapeutic anti-TNF-α antibody Adalimumab whose characteristics are detailed in Table 1. It is a purified antibody of known concentration.

### Preparation of TR-FRET assay components

Just before use, all assay components were diluted with buffer 2 whose formulation is detailed in Example 1. Serial dilutions of Adalimumab were prepared 4X to target final concentrations in the assay comprised between 1 and 100 nM. The premix [biotinylated human TNF-α/streptavidin-d2] was prepared 4X using a ratio 1/1 to obtain final concentrations of 100 nM/100 nM. The human C1q protein was prepared 4X to reach a final concentration of 5 nM. The Eu³⁺cryptate-anti-C1q antibody was prepared 4X to target a final concentration of 0.6 nM.

### TR-FRET assay protocol

The 4X solutions of assay components were sequentially dispensed in a 384-well low volume white microplate (Proxiplate Plus, PerkinElmer #6008280) as followed:
- 5 µL of serial dilutions of Adalimumab (or 5 µL of buffer for the negative control)
- 5 µL of the premix [biotinylated human TNF-α/streptavidin-d2]
- 5 µL of human C1q protein
- 5 µL of Eu³⁺cryptate-anti-C1q antibody

The plate was covered with a sealer and incubated overnight at RT. The HTRF signal was recorded on the PHERAstar FS microplate reader (BMG Labtech) using an HTRF detection module (excitation with a flash lamp at 337 nm, signal emissions recorded at 665 nm & 620 nm).

### TR-FRET signal analysis and data handling

HTRF data were analysed and represented as described in Example 1. The binding curve obtained with Adalimumab is presented in Figure 12. The S/B was calculated as detailed in Example 1, and the EC₅₀ value was also determined. The results obtained are presented in Table 5.

**[Table 5]**

| **Tested antibody** | **S/B** | **EC₅₀ (nM)** |
|---|---|---|
| Adalimumab | 7.2 | 7.6 |

The TR-FRET assay using a biotinylated human TNF-α allowed to detect the interaction between human C1q and the therapeutic anti-TNF-α IgG1 antibody Adalimumab (Figure 12). The S/B and EC₅₀ value obtained with this method (Table 5) are similar to the one obtained with the TR-FRET assay using a biotinylated anti-human Fab antibody (Table 2).

### Example 5: Test format [anti-human Fab-biotin/Streptavidin-d2] versus anti-human Fab-d2 (TR-FRET)

The TR-FRET assay format described in Example 1 and illustrated in Figure 13A was compared to another assay format using an anti-human IgG Fab directly labelled with d2 (Figure 13B).

The TR-FRET detection is based on HTRF^{®} technology (PerkinElmer/Cisbio Bioassays), as described in Example 1.

The assay format illustrated in Figure 13A is based on the use of a complex between d2-labelled streptavidin (PerkinElmer/Cisbio Bioassays, #610SADLF) and a biotinylated mouse monoclonal anti-human IgG Fab antibody (GeneTex, #GTX27497) that was used to capture and aggregate the tested antibody in solution. The anti-human IgG Fab antibody was biotinylated as described in Example 1.

The assay format illustrated in Figure 13B is based on the use of the same mouse monoclonal anti-human IgG Fab antibody (GeneTex, #GTX27497) that was directly labelled with d2 using the d2 labeling kit (PerkinElmer/Cisbio Bioassays #62D2DPEA) according to manufacturer's instructions.

In both assay formats, the human C1q protein (Sigma-Aldrich #C1740) bound to the tested antibody was detected with a mouse monoclonal anti-Clq antibody (HycultBiotech #HM2382) conjugated to Eu³⁺ cryptate as described in Example 1.

The antibody tested in the assay was the therapeutic antibody Rituximab whose characteristics are detailed in Table 1. Rituximab was a purified antibody of known concentration.

### Preparation of TR-FRET assay components

Just before use, all assay components were diluted with buffer 2 whose formulation is detailed in Example 1. Serial dilutions of Rituximab were prepared 4X to target final concentrations in the assay comprised between 1 and 100 nM. The premix [biotinylated anti-human IgG Fab/streptavidin-d2] was prepared 4X using a ratio 1/1 to obtain final concentrations of 50 nM/50 nM. The anti-human IgG Fab-d2 was prepared 4X to target a final concentration of 50 nM. The human C1q protein was prepared 4X to reach a final concentration of 5 nM. The Eu³⁺cryptate-anti-C1q antibody was prepared 4X to target a final concentration of 0.6 nM.

### TR-FRET assay protocol

The 4X solutions of assay components were sequentially dispensed in a 384-well low volume white microplate (Proxiplate Plus, PerkinElmer #6008280) as followed:
- 5 µL of serial dilutions of Rituximab (or 5 µL of buffer for the negative control)
- 5 µL of the premix [biotinylated anti-human IgG Fab/streptavidin-d2] or 5 µL of anti-human IgG Fab-d2
- 5 µL of human C1q protein
- 5 µL of Eu³⁺cryptate-anti-C1q antibody

The plate was covered with a sealer and incubated overnight at RT. The HTRF signal was recorded on the PHERAstar FS microplate reader (BMG Labtech) using an HTRF detection module (excitation with a flash lamp at 337 nm, signal emissions recorded at 665 nm & 620 nm).

### TR-FRET signal analysis and data handling

The "HTRF Ratio" and "Delta Ratio" values were calculated as detailed in Example 1. The normalized signal "Delta F%" was then calculated using the following formula: Delta F% = Delta Ratio_{antibody}/ HTRF Ratio_{negative control}. The binding curve was represented by plotting the log [antibody] (M) versus the Delta F%, and fitted on GraphPad Prism 9 using the model "Sigmoidal dose response curve - Variable slope (four parameters)", as illustrated in Figure 14. The S/B was calculated as detailed in Example 1, and the EC₅₀ value was also determined. The results obtained are presented in Table 6.

**[Table 6]**

| | **[Anti-human Fab-biotin + Streptavidin-d2]** | **Anti-human Fab-d2** |
|---|---|---|
| **S/B** | 5.5 | 1.8 |
| **EC₅₀ (nM)** | 11.7 | 24.0 |

The S/B obtained on the Rituximab binding curve using the anti-human IgG Fab directly labelled with d2 was 3-times lower than the one obtained using the complex [anti-human IgG Fab-biotin/streptavidin-d2]. Besides, the EC₅₀ value of Rituximab was increased by a factor of 2.1 when the complex [biotin-antibody/streptavidin-d2] was substituted by the d2-antibody. These data suggest that the anti-human IgG Fab-d2 alone (not complexed to streptavidin) is not able to properly induce the aggregation of Rituximab and therefore its proper binding to human C1q.

### Example 6: sandwich method to determine the affinity of anti-human Fab antibodies for human IgG of different isotypes (IgG1, IgG2 and IgG4) (TR-FRET)

A TR-FRET sandwich assay was set up to perform saturation binding experiments and determine the affinity of anti-human Fab antibodies for human IgG of different isotypes (IgG1, IgG2 and IgG4), as illustrated in Figure 15.

The TR-FRET detection is based on HTRF^{®} technology (PerkinElmer/Cisbio Bioassays), as described in Example 1.

Two different binding assay formats have been tested (Figure 15A & B). Both assay formats are based on the use of a human IgG (IgG1, IgG2 or IgG4) antibody labelled with Eu³⁺ cryptate using the Europium cryptate labeling kit (PerkinElmer/Cisbio Bioassays #62EUSUEA) according to manufacturer's instructions. The human IgG antibodies used in the assay are described in Table 7.

**[Table 7]**

| **Denomination** | **Description** | **Supplier, #Catalog number** |
|---|---|---|
| Human IgG1 | Human myeloma IgG1 purified from human plasma | Sigma-Aldrich, #15154 |
| Human IgG2 | Human myeloma IgG2 purified from human plasma | Sigma-Aldrich, #15404 |
| Human IgG4 | Human myeloma IgG4 purified from human plasma | Sigma-Aldrich, #14764 |

The binding assay format illustrated in Figure 15A uses increasing concentrations of anti-human IgG Fab antibody directly labelled with d2 using the d2 labeling kit (PerkinElmer/Cisbio Bioassays #62D2DPEA) according to manufacturer's instructions. The binding assay format illustrated in Figure 15B uses increasing concentrations of biotinylated anti-human IgG Fab antibody complexed to streptavidin-d2 (PerkinElmer/Cisbio Bioassays, #610SADLF). The anti-human IgG Fab antibody was biotinylated as described in Example 1.

The characteristics of the three different anti-human IgG Fab antibodies tested in the assay are presented in Table 8.

**[Table 8]**

| **Denomination** | **Description** | **Supplier, #Catalog number** |
|---|---|---|
| Anti-human Fab 1 | Anti-human IgG Fab Mouse IgG2b, monoclonal antibody, clone 4A11 | ThermoFisher Scientific, #SA1-19255 |
| Anti-human Fab 2 | Anti-human IgG Fab Mouse IgG2b, monoclonal antibody, clone 2A11 | GeneTex, #GTX27497 |
| Anti-human Fab 3 | Anti-human IgG Fab Goat IgG, polyclonal antibody | Sigma-Aldrich, #15260 |

### Preparation of TR-FRET assay components

Just before use, all assay components were diluted with buffer 3 (Tris-HCI 50 mM, 0.1% BSA, 100 mM KF, pH 7.4). The human IgG (IgG1, IgG2 or IgG4) antibody labelled with Eu³⁺ cryptate was prepared 4X to reach a final concentration of 0.3 nM. A 2X solution of the corresponding unlabelled human IgG was prepared to obtain a final concentration of 300 nM (used in large excess to compete with the labeled human IgG). Serial dilutions of anti-human IgG Fab-d2 were prepared 4X to target final concentrations in the assay comprised between 0.02 and 50 nM. The premix [biotinylated anti-human IgG Fab/streptavidin-d2] was prepared 4X and serially diluted keeping a ratio of 1/1 to obtain final concentrations in the assay comprised between 0.02 nM/0.02 nM and 50 nM/50 nM.

### TR-FRET assay protocol

The working solutions of assay components were sequentially dispensed in a 384-well low volume white microplate (Proxiplate Plus, PerkinElmer #6008280) as followed:
- 10 µL of buffer 3 (or 10 µL of unlabelled human IgG to determine the non-specific signal)
- 5 µL of Eu³⁺cryptate-human IgG
- 5 µL of serial dilutions of anti-human IgG Fab-d2 or 5 µL of serial dilutions of the premix [biotinylated anti-human IgG Fab/streptavidin-d2]

The plate was covered with a sealer and incubated overnight at RT. The HTRF signal was recorded on the PHERAstar FS microplate reader (BMG Labtech) using an HTRF detection module (excitation with a flash lamp at 337 nm, signal emissions recorded at 665 nm & 620 nm).

### TR-FRET signal analysis and data handling

The "HTRF Ratio" was calculated as detailed in Example 1. The total signal corresponds to the HTRF Ratio obtained in absence of unlabeled human IgG. The non-specific signal corresponds to the HTRF Ratio obtained in the presence of unlabelled IgG. The specific signal was calculated as followed: Specific signal = HTRF Ratio_{Total signal} - HTRF Ratio_{Non-specific signal}. The saturation binding curves (of the total and specific signals) were represented by plotting the [anti-human IgG Fab antibody] (nM) versus the HTRF Ratio, and fitted on GraphPad Prism 9 using the model "Binding - Saturation (One site)". The non-specific signal was fitted using the model "Simple linear regression". The results obtained with Eu³⁺cryptate-human IgG1, Eu³⁺cryptate-human IgG2, and Eu³⁺cryptate-human IgG4 are presented in Figures 16, 17, and 18 respectively. The saturation binding experiments with Eu³⁺cryptate-human IgG2 were performed only with the assay format based on the biotinylated anti-human Fab complexed to streptavidin-d2 (Figure 17). The Kd values determined from the specific binding saturation curves are presented in Table 9 (nd = not determined).

**[Table 9]**

| **Kd (nM)** | **Anti-human Fab 1** | | **Anti-human Fab 2** | | **Anti-human Fab 3** | |
|---|---|---|---|---|---|---|
| | d2 | Biotin + Streptavid in-d2 | d2 | Biotin + Streptavidin-d2 | d2 | Biotin + Streptavidin-d2 |
| **Eu³⁺ cryptate-Human IgG1** | 0.16 | 0.20 | 0.20 | 0.26 | 1.05 | 0.59 |
| **Eu³⁺ cryptate-human IgG2** | nd | 0.37 | nd | 0.47 | nd | 3.28 |
| **Eu³⁺ cryptate-human IgG4** | 0.44 | 0.33 | 0.45 | 0.40 | 9.6 | 4.20 |

The Kd values determined with each assay format (anti-human IgG Fab-d2 or biotinylated anti-human IgG Fab complexed to streptavidin-d2) were relatively close and varied by a factor of 2.3 at most. Anti-human Fab 1 and 2 gave similar Kd values for Eu³⁺ cryptate-human IgG1, IgG2 and IgG4 antibodies, ranging from 0.16 to 0.47 nM. This indicates that these mouse monoclonal antibodies recognize similarly the different human IgG isotypes (IgG1, IgG2 and IgG4), with a good affinity in the sub nanomolar range. Conversely, the anti-human Fab 3 showed lower affinities for Eu³⁺ cryptate-human IgG2 and IgG4 (Kd values comprised between 3.3 and 9.6 nM) compared to the one obtained with Eu³⁺ cryptate-human IgG1 (Kd value ∼ 0.6 to 1 nM). This indicates that this goat polyclonal antibody does not recognize similarly the different human IgG isotypes (IgG1, IgG2 and IgG4), and has an affinity approximately 6-times less good for IgG2 compared to IgG1, and about 7 to 9-times less good for IgG4 compared to IgG1.

### Example 7: competition method to determine the affinity of anti-human Fab antibodies for fully human, humanized and chimeric IgG of different isotypes (IgG1, IgG2 and IgG4) (TR-FRET)

Based on the TR-FRET sandwich assay presented in Example 6, a competitive binding assay was carried out to determine the affinity of anti-human Fab antibodies for fully human, humanized and chimeric IgG antibodies of different isotypes (IgG1, IgG2 and IgG4), as illustrated in Figure 19.

The TR-FRET detection is based on HTRF^{®} technology (PerkinElmer/Cisbio Bioassays), as described in Example 1.

Two different competitive binding assay formats have been tested (Figure 19) based on the two binding assay formats described in Example 6 (Figure 15A & B, Table 7, Table 8). The concentration of the anti-human-Fab (labelled with d2, or biotinylated and complexed with streptavidin-d2) was fixed based on the Kd values determined in Example 6. Competitive binding experiments were performed by adding increasing concentrations of unlabeled IgG antibodies of different isotypes (IgG1, IgG2 and IgG4) and different formats (fully human, humanized and chimeric). The characteristics of the IgG antibodies tested in the assay are described in Table 10. They are all purified antibodies of known concentrations.

**[Table 10]**

| **Isotype** | **Format** | **Denomination** | **Description** | **Supplier, #Catalog number** |
|---|---|---|---|---|
| **IgG1** | Fully human | IgG1 isotype control | Human IgG1 Isotype Control Recombinant Antibody | BioLegend, #403502 |
| | | Sarilumab | Therapeutic anti-IL-6R antibody | - |
| | | Ipilimumab | Therapeutic anti-CTLA-4 antibody | - |
| | | Adalimumab | Therapeutic anti-TNF-α antibody | - |
| | Humanized | Pertuzumab | Therapeutic anti-HER2 antibody | - |
| | | Atezolizumab | Therapeutic anti-PD-L1 antibody | - |
| | | Tocilizumab | Therapeutic anti-IL-6R antibody | - |
| | Chimeric | Rituximab | Therapeutic anti-CD20 antibody | - |
| | | Cetuximab | Therapeutic anti-EGFR antibody | - |
| **IgG2** | Fully human | IgG2 isotype control | Human IgG2 Isotype Control Recombinant Antibody | BioLegend, #403602 |
| | | Ipilimumab IgG2 isotype | Antibody consisting of the variable region of Ipilimumab and the constant region of the human IgG2 isotype | InvivoGen, #hctla4-mab2 |
| | | Nivolumab IgG2 isotype | Antibody consisting of the variable region of Nivolumab and the constant region of the human IgG2 isotype | InvivoGen, #hpd1nimab2 |
| | Chimeric | Rituximab IgG2 isotype | Antibody consisting of the variable region of Rituximab and the constant region of the human IgG2 isotype | InvivoGen, #hcd20-mab2 |
| | | Cetuximab IgG2 isotype | Antibody consisting of the variable region of Cetuximab and the constant region of the human IgG2 isotype | InvivoGen, #hegfr-mab2 |
| **IgG4** | Fully human | IgG4 isotype control | Human IgG4 Isotype Control Recombinant Antibody | BioLegend, #403702 |
| | | Nivolumab | Therapeutic anti-PD-1 antibody | - |
| | Humanized | Spartalizumab | Therapeutic anti-PD-1 antibody | - |
| | | Camrelizumab | Therapeutic anti-PD-1 antibody | - |
| | | Pembrolizumab | Therapeutic anti-PD-1 antibody | - |
| | Chimeric | Rituximab IgG4 isotype | Antibody consisting of the variable region of Rituximab and the constant region of the human IgG4 isotype | InvivoGen, #hcd20-mab4 |

### Preparation of TR-FRET assay components

Just before use, all assay components were diluted with buffer 3 whose formulation is described in Example 6. The human IgG (IgG1, IgG2 or IgG4) antibody labelled with Eu³⁺ cryptate was prepared 4X to reach a final concentration of 0.3 nM. The anti-human IgG Fab (labelled with d2 or biotinylated and complexed with Streptavidin-d2 with a ratio 1/1) was prepared 4X to target a final concentration corresponding to 2 x the Kd value determined in Example 6 (Table 9). Serial dilutions of unlabeled IgG antibodies were prepared 2X to target final concentrations in the assay comprised between 0.03 and 150 nM.

### TR-FRET assay protocol

The working solutions of assay components were sequentially dispensed in a 384-well low volume white microplate (Proxiplate Plus, PerkinElmer #6008280) as followed:
- 10 µL of serial dilutions of unlabeled IgG antibody (or 10 µL of buffer 3 to determine the signal max)
- 5 µL of Eu³⁺cryptate-human IgG
- 5 µL of anti-human IgG Fab-d2 or 5 µL of the premix [biotinylated anti-human IgG Fab/streptavidin-d2]

The plate was covered with a sealer and incubated overnight at RT. The HTRF signal was recorded on the PHERAstar FS microplate reader (BMG Labtech) using an HTRF detection module (excitation with a flash lamp at 337 nm, signal emissions recorded at 665 nm & 620 nm).

### TR-FRET signal analysis and data handling

The "Delta F%" values were calculated as detailed in Example 5. The Delta F%ₘₐₓ corresponds to the Delta F% obtained in absence of unlabeled IgG antibody. The % of signal max was calculated for each concentration of unlabeled IgG antibody as followed: % signal max = Delta F%_{unlabeled IgG antibody/}Delta F%ₘₐₓ x 100. The competitive binding curves were represented by plotting the log [unlabeled IgG antibody] (M) versus the % signal max and fitted on GraphPad Prism 9 using the model "Dose-response - Inhibition (Variable slope - four parameters)". The competitive binding curves obtained with unlabeled IgG1 antibodies, unlabeled IgG2 antibodies, and unlabeled IgG4 antibodies are represented in Figures 20, 21 and 22 respectively. The IC₅₀ values determined from the competitive binding curves were used to calculate the Ki values using the Cheng-Prusoff equation: Ki = IC₅₀ / (1+([labelled human IgG]/Kd)). The IC₅₀ and Ki values obtained with unlabeled IgG1, IgG2, and IgG4 are presented in Tables 11, 12 and 13 respectively (nd = not determined).

The competitive binding experiments with fully human, humanized and chimeric IgG1 antibodies were carried out with the biotinylated anti-human Fab 1 complexed to streptavidin-d2 (Figure 20A), and with the anti-human Fab 2 and 3 directly labelled with d2 (Figure 20B & C). The anti-human Fab 3-d2 was not tested with all IgG1 antibodies.

**[Table 11]**

| **Unlabelled IgG1 antibody** | **Competition on anti-human-Fab 1-biotin** + **Streptavidin-d2** | | **Competition on anti-human-Fab 2-d2** | | **Competition on anti-human-Fab 3-d2** | |
|---|---|---|---|---|---|---|
| | **IC₅₀ (nM)** | **Ki (nM)** | **IC₅₀ (nM)** | **Ki (nM)** | **IC₅₀ (nM)** | **Ki (nM)** |
| IgG1 isotype control | 0.37 | 0.148 | 0.51 | 0.204 | 0.20 | 0.16 |
| Rituximab | 0.46 | 0.184 | 0.54 | 0.216 | 0.22 | 0.17 |
| Cetuximab | 0.44 | 0.176 | 0.53 | 0.212 | nd | nd |
| Pertuzumab | 0.37 | 0.148 | 0.45 | 0.18 | nd | nd |
| Atezolizumab | 0.42 | 0.168 | 0.55 | 0.22 | 0.17 | 0.13 |
| Tocilizumab | 0.40 | 0.16 | 0.63 | 0.252 | nd | nd |
| Ipilimumab | 0.55 | 0.22 | 0.72 | 0.288 | nd | nd |
| Adalimumab | 0.35 | 0.14 | 0.63 | 0.252 | nd | nd |
| Sarilumab | 0.45 | 0.18 | 0.55 | 0.22 | nd | nd |
| | | Mean = 0.17 ± 0.05 | | Mean = 0.23 ± 0.06 | | Mean = 0.15 ± 0.04 |

The competition curves obtained with all tested IgG1 antibodies were superposed, whatever the anti-human Fab used. The Ki values, corresponding to the affinity of each anti-human Fab for each tested IgG1 antibody, were very close whatever the anti-human Fab used, with an average around 0.2 nM (Table 11). This indicates that the anti-human Fab 1, 2 and 3 recognize similarly fully human, humanized, and chimeric IgG1 antibodies, with an affinity in the sub nanomolar range.

**[Table 12]**

| **Unlabelled IgG2 antibody** | **Competition on anti-human-Fab 1-biotin** + **Streptavidin-d2** | |
|---|---|---|
| | **IC₅₀ (nM)** | **Ki (nM)** |
| IgG2 isotype control | 0.90 | 0.50 |
| Rituximab IgG2 isotype | 0.96 | 0.53 |
| Cetuximab IgG2 isotype | 1.30 | 0.72 |
| Ipilimumab IgG2 isotype | 0.71 | 0.39 |
| Nivolumab IgG2 isotype | 0.49 | 0.27 |
| | | Mean = 0.48 ± 0.33 |

The competitive binding experiments with fully human and chimeric IgG2 antibodies were carried out only with the biotinylated anti-human Fab 1 complexed to streptavidin-d2 (Figure 21). The profiles of the competition curves obtained with all tested IgG2 antibodies were similar, with Ki values ranging from 0.27 to 0.72 nM (Table 12). This indicates that the anti-human Fab 1 recognize similarly fully human and chimeric IgG2 antibodies, with an average affinity of 0.5 nM.

**[Table 13]**

| **Unlabelled IgG4 antibody** | **Competition on anti-human-Fab 1-biotin + Streptavidin-d2** | | **Competition on anti-human-Fab 2-d2** | | **Competition on anti-human-Fab 3-d2** | |
|---|---|---|---|---|---|---|
| | **IC₅₀ (nM)** | **Ki (nM)** | **IC₅₀ (nM)** | **Ki (nM)** | **IC₅₀ (nM)** | **Ki (nM)** |
| IgG4 isotype control | 0.14 | 0.07 | 0.60 | 0.36 | 0.13 | 0.13 |
| Spartalizumab | 0.15 | 0.08 | 0.68 | 0.41 | nd | nd |
| Camrelizumab | 0.16 | 0.08 | 0.66 | 0.40 | nd | nd |
| Pembrolizumab | 0.14 | 0.07 | 0.49 | 0.29 | Partial inhibition (31%) | |
| Nivolumab | 0.14 | 0.07 | 0.65 | 0.39 | Partial inhibition (32%) | |
| Rituximab IgG4 isotype | 0.25 | 0.13 | 0.69 | 0.41 | nd | nd |
| | | Mean = 0.08 ± 0.05 | | Mean = 0.38 ± 0.09 | | |

The competitive binding experiments with fully human, humanized and chimeric IgG4 antibodies were carried out with the biotinylated anti-human Fab 1 complexed to streptavidin-d2 (Figure 22A), and with the anti-human Fab 2 and 3 directly labelled with d2 (Figure 22B & C). The anti-human Fab 3-d2 was not tested with all IgG4 antibodies.

The profiles of the competition curves obtained with all tested IgG4 antibodies were similar using either the anti-human Fab 1 or the anti-human Fab 2 (Figure 22A & B). The Ki values determined with each of the two anti-human Fab were very close, with an average of 0.1 nM for the anti-human Fab 1, and an average of 0.4 nM for the anti-human Fab 2 (Table 13). This indicates that these mouse monoclonal anti-human Fab antibodies bind in a similar way to fully human, humanized, and chimeric IgG4 antibodies, with an affinity in the sub nanomolar range. The competitive binding curves obtained with the anti-human Fab 3 showed different profiles depending on the nature of the antibodies tested (Figure 22C). Indeed, the human IgG4 isotype control antibody induced a total inhibition of the signal, with a Ki value of 0.13 nM. Conversely, the competition with the therapeutic antibodies Pembrolizumab (humanized IgG4) and Nivolumab (fully human IgG4) was partial, with only 31 to 32% of signal inhibition. These results suggest that the goat polyclonal anti-human Fab 3 antibody does not properly recognize therapeutic IgG4 antibodies.

### Example 8: optimisation of the concentration of biotinylated anti-Fab ligand

To optimize the concentration of biotinylated anti-Fab ligand, the TR-FRET assay described in Example 1 was carried out using different concentrations of anti-Fab ligand.

The TR-FRET detection is based on HTRF^{®} technology (PerkinElmer/Cisbio Bioassays), as described in Example 1.

The assay format uses a complex between d2-labelled streptavidin (PerkinElmer/Cisbio Bioassays, #610SADLF) and a mouse monoclonal anti-human IgG Fab antibody (ThermoFisher Scientific #SA1-19255) conjugated to biotin as described in Example 1. The human C1q protein (Sigma-Aldrich #C1740) bound to the tested antibody was detected with a mouse monoclonal anti-Clq antibody (HycultBiotech #HM2382) conjugated to Eu³⁺ cryptate as described in Example 1.

The antibodies tested in the assay were the therapeutic antibody Atezolizumab and the human IgG2 isotype control whose characteristics are detailed in Table 1. Both are purified antibodies of known concentration.

### Preparation of TR-FRET assay components

Just before use, all assay components were diluted with buffer 2 whose formulation is detailed in Example 1. Serial dilutions of tested antibodies were prepared 4X to target final concentrations in the assay comprised between 0.5 and 100 nM. Three different solutions of premix [biotinylated anti-human IgG Fab/streptavidin-d2] were prepared 4X using a ratio 1/1 to obtain final concentrations of 30 nM/30 nM, 40 nM/40 nM or 50 nM/50 nM. The human C1q protein was prepared 4X to reach a final concentration of 5 nM. The Eu³⁺cryptate-anti-C1q antibody was prepared 4X to target a final concentration of 0.6 nM.

### TR-FRET assay protocol

The 4X solutions of assay components were sequentially dispensed in a 384-well low volume white microplate (Proxiplate Plus, PerkinElmer #6008280) as followed:
- 5 µL of serial dilutions of tested antibody (or 5 µL of buffer for the negative control)
- 5 µL of the premix [biotinylated anti-human IgG Fab/streptavidin-d2] tested at three different concentrations
- 5 µL of human C1q protein
- 5 µL of Eu³⁺cryptate-anti-C1q antibody

The plate was covered with a sealer and incubated overnight at RT. The HTRF signal was recorded on the PHERAstar FS microplate reader (BMG Labtech) using an HTRF detection module (excitation with a flash lamp at 337 nm, signal emissions recorded at 665 nm & 620 nm).

### TR-FRET signal analysis and data handling

The "Delta F%" values were calculated, and the dose-response binding curves were fitted as described in Example 5. The binding profiles using different concentrations of biotinylated anti-human IgG Fab with Atezolizumab (IgG1) and the IgG2 isotype control are represented in Figures 23A and 23B respectively. The S/B were calculated as detailed in Example 1, and the EC₅₀ values were also determined. The results are presented in Table 14.

**[Table 14]**

| **[Biotinylated anti-human Fab antibody]** | **IgG1** | | **IgG2** | |
|---|---|---|---|---|
| | **EC₅₀ (nM)** | **S/B** | **EC₅₀ (nM)** | **S/B** |
| 30 nM | 7.2 | 7.4 | 19.2 | 2.7 |
| 40 nM | 9.5 | 7.4 | 25.7 | 2.7 |
| 50 nM | 10.9 | 7.7 | 30.6 | 2.8 |

With both IgG1 and IgG2 antibodies, the binding curve obtained with 30 nM of biotinylated anti-human IgG Fab showed an EC₅₀ value shifted to the left compared to the binding curve obtained with 50 nM of biotinylated anti-Fab ligand. The binding curves obtained with 40 or 50 nM of biotinylated anti-human IgG Fab were almost superposed, with a signal intensity (S/B) slightly better with 50 nM of biotinylated anti-Fab ligand.

These results indicate that the optimal concentration of biotinylated anti-human IgG Fab to be used to ensure working with a saturating dose is 50 nM. This concentration is approximately 100-times higher than the affinities previously determined in Example 7 (∼ 0.2 nM for IgG1 and ∼ 0.5 nM for IgG2) and confirm that it is in large excess.

### Example 9: optimisation of the salt concentration

To optimize the concentration of salt in the assay, the TR-FRET assay described in Example 1 was carried out in a 4 mM PO₄ buffer supplemented with different concentrations of NaCl.

The TR-FRET detection is based on HTRF^{®} technology (PerkinElmer/Cisbio Bioassays), as described in Example 1.

The assay format uses a complex between d2-labelled streptavidin (PerkinElmer/Cisbio Bioassays, #610SADLF) and a mouse monoclonal anti-human IgG Fab antibody (ThermoFisher Scientific #SA1-19255) conjugated to biotin as described in Example 1. The human C1q protein (Sigma-Aldrich #C1740) bound to the tested antibody was detected with a mouse monoclonal anti-Clq antibody (HycultBiotech #HM2382) conjugated to Eu³⁺ cryptate as described in Example 1.

The antibodies tested in the assay were the therapeutic antibodies Rituximab and Obinutuzumab, as well as the Rituximab IgG2 isotype variant. Their characteristics are detailed in Table 1. All are purified antibodies of known concentration.

### Preparation of TR-FRET assay components

A 4 mM PO₄ buffer (Na₂HPO₄ 3 mM, KH₂PO₄ 1 mM, BSA 0.1%, Tween-20 0.05%, ProClin-300 0.01%, pH 7.4) was prepared and supplemented with different concentrations of NaCl ranging from 125 to 155 mM. Just before use, all assay components were diluted with each buffer containing a different concentration of NaCl. Serial dilutions of tested antibodies were prepared 4X to target final concentrations in the assay comprised between 1 and 100 nM. The premix [biotinylated anti-human IgG Fab/streptavidin-d2] was prepared 4X using a ratio 1/1 to obtain a final concentration of 50 nM/50 nM. The human C1q protein was prepared 4X to reach a final concentration of 5 nM. The Eu³⁺cryptate-anti-C1q antibody was prepared 4X to target a final concentration of 1.2 nM.

### TR-FRET assay protocol

The 4X solutions of assay components prepared in each buffer were sequentially dispensed in a 384-well low volume white microplate (Proxiplate Plus, PerkinElmer #6008280) as followed:
- 5 µL of serial dilutions of tested antibody (or 5 µL of buffer for the negative control)
- 5 µL of the premix [biotinylated anti-human IgG Fab/streptavidin-d2]
- 5 µL of human C1q protein
- 5 µL of Eu³⁺cryptate-anti-C1q antibody

The plate was covered with a sealer and incubated overnight at RT. The HTRF signal was recorded on the PHERAstar FS microplate reader (BMG Labtech) using an HTRF detection module (excitation with a flash lamp at 337 nm, signal emissions recorded at 665 nm & 620 nm).

### TR-FRET signal analysis and data handling

The "Delta Ratio" and "Delta F%" values were calculated as described in Example 1 and Example 5 respectively. The binding curves were fitted as described in Example 5.

The max Delta Ratio values obtained for 50 nM of Rituximab and 50 nM of Obinutuzumab in the buffer supplemented with different concentrations of NaCl are represented in Figure 24. For each concentration of NaCl, the corresponding osmolarity of the buffer was calculated, as well as the ratio Signal maX_{Rituximab}/Signal max_{Obinutuzumab} used to evaluate the capacity of the assay to discriminate the C1q binding capacity of the two anti-CD20 antibodies (Table 15).

**[Table 15]**

| | | | | | | |
|---|---|---|---|---|---|---|
| **[NaCl] (mM) added in the 4 mM PO4 buffer** | 125 | 130 | 135 | 140 | 145 | 155 |
| **Osmolarity of the buffer (mOsm/L)** | 262 | 272 | 282 | 292 | 302 | 322 |
| **Delta Ratio Rituximab at 50 nM/ Delta Ratio Obinutuzumab at 50 nM** | 2.2 | 2.8 | 3.6 | 4.8 | 6.0 | 5.8 |

In presence of a concentration of NaCl ranging from 135 to 155 mM (corresponding to an osmolarity of 282 to 322 mOsm/L), the ratio is > 3, indicating that the assay discriminates properly the capacity of the Type I anti-CD20 antibody Rituximab and the Type II anti-CD20 antibody Obinutuzumab to interact with human C1q. Below this concentration of NaCl (osmolarity < 282 mOsm/L), the ratio is < 3, indicating that the discrimination is less good.

The binding curves (represented in Delta F%) of Rituximab, Obinutuzumab, and the Rituximab IgG2 isotype variant obtained in presence of 155 and 135 mM of NaCl are presented in Figure 25A & B respectively. For each curve, the S/B was calculated as detailed in example 1, and the EC₅₀ value was also determined. These values are presented in Table 16.

**[Table 16]**

| **[NaCl]** | **Rituximab** | | **Obinutuzumab** | | **Rituximab IgG2 isotype** | |
|---|---|---|---|---|---|---|
| | **EC₅₀ (nM)** | **S/B** | **EC₅₀ (nM)** | **S/B** | **EC₅₀ (nM)** | **S/B** |
| **155 mM** | 28.9 | 4.6 | 19.7 | 1.6 | 37.6 | 1.2 |
| **135 mM** | 15.5 | 8.2 | 18.6 | 3.5 | 38.3 | 1.7 |

The results showed that the concentration of NaCl also influenced assay sensitivity. With 155 mM of NaCl, the S/B obtained with Obinutuzumab and the Rituximab IgG2 isotype variants were very low (comprised between 1.2 and 1.6). Using 135 mM of NaCl, the discrimination between the different antibodies was still good and the S/B were more robust (≥ 1.7). This concentration of NaCl was therefore optimal to study the binding of antibodies to human C1q.

### REFERENCES

[Ref. 1] Kaul et al., Dissection of C1q Capability of Interacting with IgG, THE JOURNAL OF BIOLOGICAL CHEMISTRY, 1997
[Ref. 2] Patel et al., IgG subclass specificity to C1q determinated by surface plasmon resonance using Protein L capture technique, Analytical biochemistry, 2015
[Ref. 3] Lilienthal et al., Potential of Murine IgG1 and Human IgG4 to Inhibit the Classical Complement and Fcγ Receptor Activation Pathways, Frontiers in Immunology, 2018
[Ref. 4] Diebolder et al., Complement Is Activated by IgG Hexamers Assembled at the Cell Surface, Science, 2014
[Ref. 5] Herter et al., Preclinical Activity of the Type II CD20 Antibody GA101 (Obinutuzumab) Compared with Rituximab and Ofatumumab In Vitro and in Xenograft Models, Molecular Cancer Therapeutics, 2013
[Ref. 6] Almagro et al., Progress and Challenges in the Design and Clinical Development of Antibodies for Cancer Therapy, Frontier in Immunology, 2018
[Ref. 7] Zhou et al., Characterization of antibody-C1q interactions by Biolayer Interferometry, Analytical Biochemistry, 2018

## Claims

1. An *in vitro* method for determining the binding of an antibody (tested antibody) to a complement component 1q (C1q), comprising the following steps:
a) Contacting into a measurement medium:
- a tested antibody,
- a biotinylated anti-Fab ligand, said biotinylated anti-Fab ligand being able to bind the Fab region of the tested antibody,
- a streptavidin directly or indirectly labelled with the first member of a pair of HPA (Homogeneous Proximity Assay) partners, and
- a C1q directly or indirectly labelled with the second member of a pair of HPA partners;
b) Measuring the HPA signal in the measurement medium, the existence of a HPA signal being representative of the binding of the tested antibody to the C1q.

2. The method according to claim 1, wherein the HPA is selected from (i) Amplified Luminescent Oxygen Channeling Immunoassay (LOCI), such as Amplified Luminescence Proximity Homogeneous Assay (ALPHA), (ii) Resonance Energy Transfer (RET) and (iii) Spatial Proximity Analyte Reagent Capture Luminescence (SPARCL).

3. The method according to any of claims 1 and 2, wherein the tested antibody is an IgG, such as an IgG1, an IgG2 or an IgG4, preferably an IgG1 or an IgG2.

4. The method according to any of claims 1 to 3, wherein the biotinylated anti-Fab ligand is a biotinylated anti-Fab antibody or antibody fragment, such as a biotinylated anti-Fab murine antibody or antibody fragment, such as a biotinylated anti-Fab mouse antibody or antibody fragment.

5. The method according to any of claims 1 to 3, wherein the biotinylated anti-Fab ligand is a biotinylated antigen or antigen fragment.

6. The method according to any of claims 1 to 5, wherein the streptavidin is directly labelled with the first member of a pair of HPA partners, such as the first member of a pair of RET partners or the first member of a pair of LOCI partners.

7. The method according to any of claims 1 to 6, wherein the C1q is indirectly labelled with an anti-Clq ligand labelled with the second member of a pair of HPA partners, such as the second member of a pair of RET partners or the second member of a pair of LOCI partners.

8. The method according to any of claims 1 to 7, wherein:
(i) the first member of a pair of HPA partners is an acceptor and the second member of a pair of HPA partners is a donor; or
(ii) the first member of a pair of HPA partners is a donor and the second member of a pair of HPA partners is an acceptor.

9. The method according to claim 8, wherein the HPA is a RET and the donor is selected from a luminescent donor compound or a fluorescent donor compound, such as:
- a FRET (Forster Resonance Energy Transfer) donor selected from europium cryptate, europium chelate, terbium chelate, terbium cryptate, ruthenium chelate, quantum dot, allophycocyanins, rhodamines, cyanins, squarains, coumarins, proflavins, acridines, fluoresceins, boron-dipyrromethene derivatives and nitrobenzoxadiazole; or
- a BRET (Bioluminescence Resonance Energy Transfer) donor selected from Luciferase (luc), Renilla Luciferase (Rluc), variants of Renilla Luciferase (Rluc8) and Firefly Luciferase.

10. The method according to any of claims 8 and 9, wherein the HPA is a RET and the acceptor is selected from a fluorescent acceptor compound or a non-fluorescent acceptor compound (quencher), such as:
- a FRET acceptor selected from allophycocyanins, rhodamines, cyanins, squarains, coumarins, proflavins, acridins, fluoresceins, boron-dipyrromethene derivatives, nitrobenzoxadiazole, a quantum dot, GFP, GFP variants selected from GFP10, GFP2 and eGFP, YFP, YFP variants selected from eYFP, YFP topaz, YFP citrine, YFP venus and YPet, mOrange, DsRed; or
- a BRET acceptor selected from allophycocyanins, rhodamines, cyanins, squarains, coumarins, proflavins, acridins, fluoresceins, boron-dipyrromethene derivatives, nitrobenzoxadiazole, a quantum dot, GFP, GFP variants selected from GFP10, GFP2 and eGFP, YFP, YFP variants selected from eYFP, YFP topaz, YFP citrine, YFP venus and YPet, mOrange, DsRed.

11. The method according to claim 8, wherein the HPA is a LOCI and the donor is phthalocyanine.

12. The method according to claim 11, wherein the HPA is a LOCI and the acceptor comprises (i) thioxene, anthracene, and rubrene, or (ii) thioxene and Europium chelate.

13. The method according to any of claims 1 to 12, wherein steps (a) and (b) are repeated with different concentrations of the tested antibody and, preferably, said method comprises an additional step (c) of determining the dissociation constant (Kd) and/or the EC₅₀ of the binding of the tested antibody to the C1q.

14. The method according to any of claims 1 to 13, wherein the measurement medium has an osmolarity from 250 mOsm/L to 500 mOsm/L.

15. Kit of reagents for carrying out the method as claimed in any one of claims 1 to 14, comprising :
(i) a biotinylated anti-Fab ligand, and
(ii) a streptavidin or a streptavidin directly labelled with the first member of a pair of HPA partners, and
(iii) a C1q or a C1q directly labelled with the second member of a pair of HPA partners, and
(iv) if the streptavidin is not directly labelled with the first member of a pair of HPA partners, an anti-streptavidin ligand directly labelled with the first member of a pair of HPA partners, and
(v) if the C1q is not directly labelled with the second member of a pair of HPA partners, an anti-Clq ligand directly labelled with the second member of a pair of HPA partners.
